(19) Europäisches Patentamt
European Patent Office
Office européen des brevets

(11) Numéro de publication : 0 391 799 B1

(12) # FASCICULE DE BREVET EUROPEEN

(45) Date de publication du fascicule du brevet : **07.10.92 Bulletin 92/41**

(21) Numéro de dépôt : **90400923.0**

(22) Date de dépôt : **04.04.90**

(51) Int. Cl.[5] : **C07D 307/85,** C07D 317/46, C07D 333/70, C07D 311/66, C07C 311/19, A61K 31/19, A61K 31/215, A61K 31/335, A61K 31/38

(54) **Nouveaux sulfonamides dérivés d'acides benzocycliques ou benzohétérocycliques, leur préparation et leur application en thérapeutique.**

(30) Priorité : **05.04.89 FR 8904470**

(43) Date de publication de la demande : **10.10.90 Bulletin 90/41**

(45) Mention de la délivrance du brevet : **07.10.92 Bulletin 92/41**

(84) Etats contractants désignés : **AT BE CH DE DK ES FR GB GR IT LI LU NL SE**

(56) Documents cités :
**EP-A- 154 508**
**EP-A- 0 253 321**
**EP-A- 0 255 728**
**EP-A- 0 322 692**
**FR-A- 1 461 111**
**GB-A- 2 118 552**
**Drugs of the Future, vol.11, no.8, 1986, pages 689-699, M.Kuchar et al.**

(73) Titulaire : **PIERRE FABRE MEDICAMENT**
**45, Place Abel Gance**
**F-92100 Boulogne (FR)**

(72) Inventeur : **Bigg, Dennis**
**122 avenue de Lavaur**
**F-81100 Castres (FR)**
Inventeur : **Duflos, Alain**
**12, rue Mathieu Estadieu**
**F-81100 Castres (FR)**
Inventeur : **Rieu, Jean-Pierre**
**La Vixiére Haute**
**Avenue du Sidobre, F-81100 Castres (FR)**

(74) Mandataire : **Ahner, Francis et al**
**CABINET REGIMBEAU, 26, avenue Kléber**
**F-75116 Paris (FR)**

EP 0 391 799 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention réalisée au Centre de Recherche PIERRE FABRE a pour objet de nouveaux sulfonamides, leur préparation et leur utilisation en tant que médicament.

Les antiagrégants plaquettaires - dont l'activité essentielle est de lutter contre la thrombose - sont de plus en plus utilisés soit à titre préventif, soit comme adjuvants thérapeutiques dans cette indication ainsi que dans l'angor, l'infarctus du myocarde, l'athérome et l'ischémie cardiaque. Le thromboxane $A_2$ ($TxA_2$) est le médiateur le plus souvent impliqué dans l'agrégation plaquettaire, c'est l'agent proagrégant le plus puissant qui se forme au cours d'une voie de métabolisation de l'acide arachidonique au niveau des plaquettes (F. Numano, Atherosclerose and antiplatelet therapy, Drugs of Today, 21, 41, 1985). Lors des désordres physiologiques, l'action du $TxA_2$ peut être stoppée au cours des divers stades de sa formation et, en particulier, en inhibant soit sa synthèse, soit son action, en bloquant les récepteurs du $TxA_2$ provoquant l'agrégation.
La recherche de molécules antagonisant les récepteurs du $TxA_2$ est une approche récente et prometteuse pour lutter contre ses méfaits. (Antithrombotic Agents, M. KUCHAR et V. REJMOLEC, Drug of the Future 11, 689, 1986) ; Comparison of the action of $TxA_2$ receptor antagonists, A.M. LEFER, Drugs of Today 21, 283, 1985).

Des dérivés d'acides sulfonamidoalcoyl phenoxy-acétiques et phényl-butyriques possédant une activité antagoniste du TxA2 ont par ailleurs été décrits dans EP-A-0 255 728 (TANABE SEIYAKU) et EP-A-0 253 321 (K. THUMAE).

Les composés de la présente invention répondent à la formule générale I

$$R - SO_2 - \underset{\displaystyle |}{\overset{\displaystyle R_1}{\overset{|}{N}}} - (\overset{\displaystyle R_2}{\overset{|}{C}}H)_n - X - A - COOR_3 \qquad \underline{I}$$

dans laquelle :

R - représente un radical alcoyle léger, ramifié ou non, renfermant de 1 à 9 C et, à titre d'exemple non limitatif Me, Et, Pr, Bu, iBu... ;

un radical phényle substitué ou non par un ou plusieurs groupes : alcoyle inférieur (1 à 4 C) ou halogène ou alcoyloxy, trifluorométhyle, nitro, amino, dialkylamino léger (1 à 4 C), et, à titre d'exemple non limitatif : Me, Et, i-Pr, F, Cl, Br, MeO, EtO, $NO_2$, $NH_2$, $NMe_2$, $CF_3$ ;

un radical naphtyle substitué ou non comme ci-dessus ; ou

un radical thiophényle.

$R_1$ - représente un hydrogène ou un alcoyle inférieur en $C_{1-4}$ linéaire ou ramifié, un benzyle, et, à titre d'exemple non limitatif : Me, Et, i-Pr.

$R_2$ - représente un hydrogène, un groupe alcoyle inférieur de 1 à 6 C ramifié ou non et, à titre d'exemple non limitatif: Me, Et, i-Pr, i-Bu ;

un groupement phényle substitué ou non par un chlore, ou un méthoxyle ;

un groupement arylalcoyle renfermant de 7 à 9 atomes de carbone.

$R_3$ - représente un hydrogène ou un alcoyle inférieur, ramifié ou non (1 à 6 C) et, à titre d'exemple non limitatif : Me, Et, i-Pr, i-Bu.

X - représente un radical fonctionnel divalent choisi parmi les suivants : $-CH_2-$ ; $-CH-OR_4$ ; -C=O ; $-C=N-OR_4$ ; et

$$-C=N-\underset{\displaystyle R_4}{\underset{|}{N}}-R_4$$

avec $R_4$=H ou Me.

A - représente un radical bivalent benzocarbocyclique ou benzohétérocyclique choisi parmi les suivants de (a) à (j)

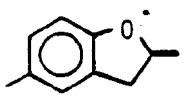 ; 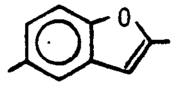 ; 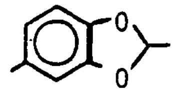 ; 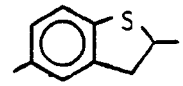 ;

(a) (b) (c) (d)

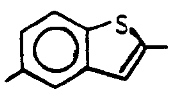 ; 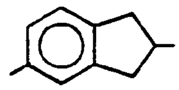 ; 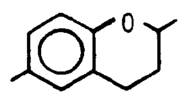 ; 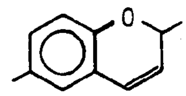 ;

(e) (f) (g) (h)

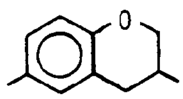 ; 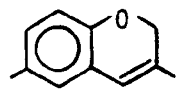

(i) (j)

n = 1 à 4 inclus.

La présente invention inclue aussi les sels minéraux ou organiques thérapeutiquement acceptables des composés de formule générale I avec $R_3$=H et, à titre d'exemple non limitatif, les sels de sodium, de calcium, de zinc, d'hydroxy-2-éthylammonium, de bis(hydroxy-2 éthyl) ammonium, de tris(hydroxy-2 éthyl)ammonium, ainsi que leurs hydrates éventuels et les hydrates des acides précurseurs. Quand les composés de formule générale I renferment au moins un carbone asymétrique, la présente invention concerne aussi bien les mélanges racémiques que les différents énantiomères ou diastéroisomères ou leurs mélanges.

La présente invention concerne également l'utilisation des composés de formule générale I à titre de médicament et les compositions pharmaceutiques renfermant ce médicament. Les compositions pharmaceutiques selon la présente invention peuvent utiliser un ou plusieurs composés de formule I éventuellement en association avec un ou plusieurs autres principes actifs.

Enfin les procédés de synthèse des composés de formule générale I font aussi partie de la présente invention.

## SYNTHESE DES COMPOSES DE STRUCTURE GENERALE I

La matière première de base pour toutes les synthèses est l'ester de l'acide en position 2 ou 3 du dérivé de l'indane ou du benzo hétérocycle envisagé (**composé II**) où **A** a la même signification que dans I, à l'exclusion des radicaux b, e, h, j, et où $R_5$ a la même valeur que $R_3$ défini dans I, à l'exclusion de l'hydrogène.

Dans un premier temps, le **composé II** est soumis à une réaction de Friedel-Crafts à l'aide d'un chlorure d'acide convenablement substitué de formule générale **III** où n et $R_2$ ont la même signification que dans la formule I et où Y représente un halogène (brome ou chlore) ou un alcoxy carbonylamino de formule $R_8OCONR_1$- (où $R_8$ représente un alcoyl linéaire léger ou un benzyle) pour donner le dérivé acylé **IV** correspondant (méthode **A**).

$$\underset{\textbf{II}}{H\text{-}A\text{-}COOR_5} + \underset{\textbf{III}}{Y\text{-}(\overset{R_2}{\overset{|}{C}}H)_n\text{-}COCl} \longrightarrow \underset{\textbf{IV}}{Y\text{-}(\overset{R_2}{\overset{|}{C}}H)_n\text{-}CO\text{-}A\text{-}COOR_5} \quad (\underline{A})$$

Dans un deuxième temps, le **composé IV** est mis en réaction selon le schéma de synthèse I dans le cas où Y est un halogène (chlore ou brome) ou selon le schéma de synthèse II dans le cas où Y représente un alcoxy carbonylamino pour donner les composés de formule générale I, objets de la présente invention.

Dans ces schémas, les radicaux ont la même signification que dans I et les nouveaux groupements sont définis dans le schéma de synthèse.

**1) Cas où Y représente dans IV un halogène (brome, chlore)** (schéma **1**)

La condensation de **IVa** avec l'azoture de sodium dans un mélange hydroalcoolique fournit le **composé V** (méthode **B**), celui-ci est réduit par l'hydrogène en présence de catalyseur en milieu acido alcoolique pour donner l'aminocétone estérifiée et salifiée **VI** (chlorhydrate par exemple) (méthode **C**). La condensation du dérivé précédent **VI** avec un halogénure de sulfonyle convenablement substitué de formule générale **VII**

$$R\text{-}SO_2\text{-}Z \qquad \underline{VII}$$

où R a la même signification que dans I et Z représente un chlore ou un brome ou un fluor est réalisée en utilisant une base organique (la pyridine par exemple) pour donner l'ester **VIII** de **I** avec $R_1$=H (méthode **D**). Cet ester peut être saponifié dans un mélange dioxanne ou alcool- soude pour donner le sulfamido céto acide **IX** de formule générale I avec $R_1$ et $R_3$=H (méthode **G**).

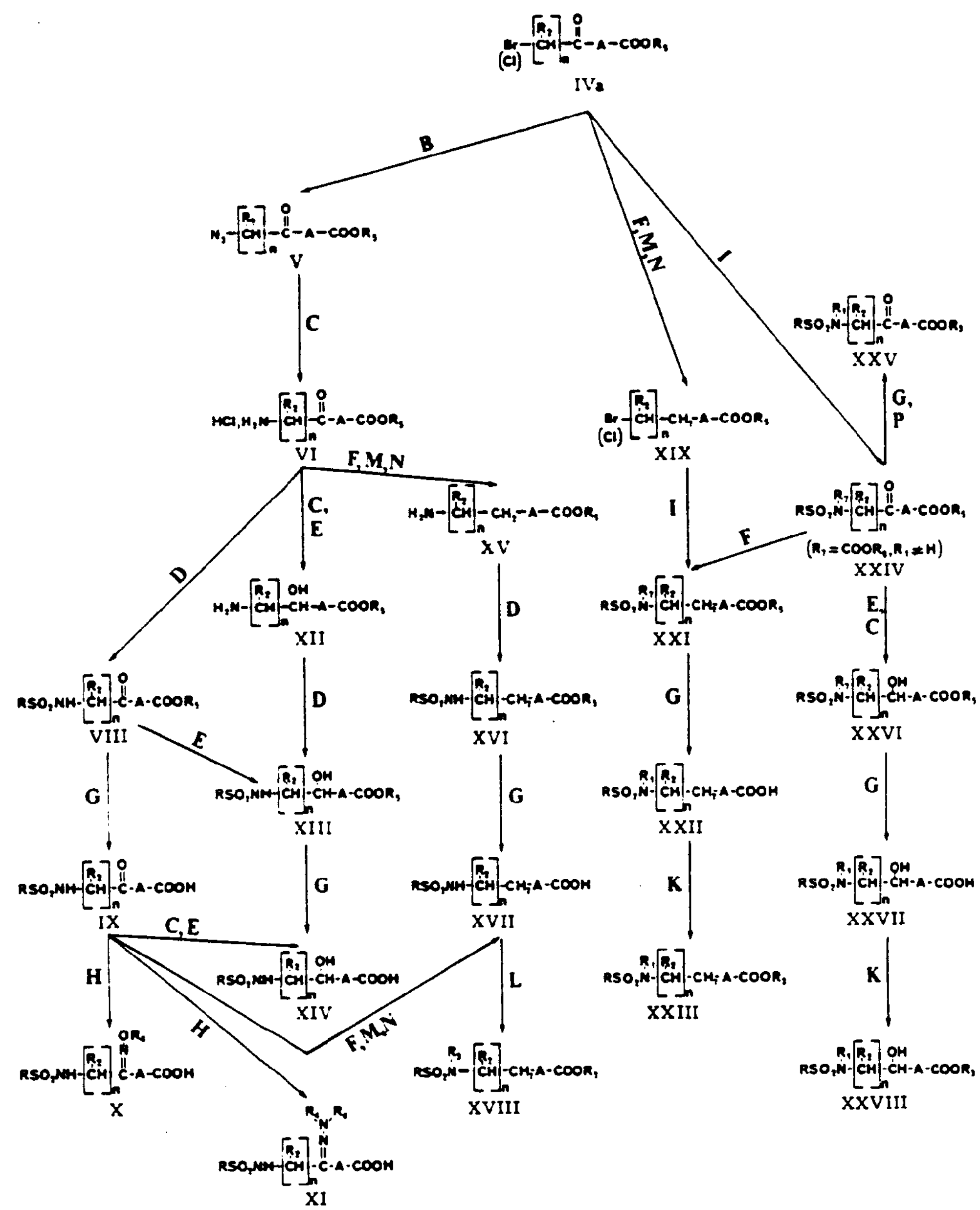

SCHEMA DE SYNTHESE : I

Ce céto acide **IX** réagit avec les hydroxylamines substituées ou non de formule :

$R_4ONH_2$

pour donner à chaud dans la pyridine les oximes correspondants **X** (méthode **$H_1$**). La condensation avec une hydrazine substituée ou non de formule :

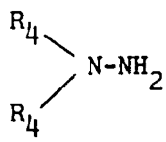

dans un acide organique à chaud conduit à l'hydrazone correspondante **XI** (méthode **$H_2$**). Si l'hydrogénation du composé **V** est plus poussée, elle peut conduire à l'amino alcool **XII** via l'intermédiaire **VI** (méthode **C**). Cet amino alcool peut aussi être préparé à partir du composé cétonique par réduction au borohydrure (Na ou K) en milieu alcoolique (méthode **E**). Il est préférable d'utiliser le même alcool que celui de la fonction ester car s'il est différent, on obtient le **composé XII** transestérifié.

L'hydroxy sulfamido ester **XIII** peut être obtenu soit à partir du dérivé cétonique **IX** selon la méthode **E**, soit par condensation de l'halogénure de sulfonyle de formule **VII** sur le **composé aminé XII** précédent (méthode **D**). La saponification de ce composé réalisée selon la méthode **G**donne l'acide hydroxy sulfamide **XIV**. L'action du triéthylsilane en milieu acide trifluoroacétique sur l'aminocétoester **VI** permet de réduire sélectivement le carbonyle en méthylène pour donner l'aminoester **XV** (méthode **F**). Ce composé se forme aussi selon la méthode **C** en milieu acide fort (méthode **M**). La condensation de **XV** avec le chlorure de sulfonyle correspondant (méthode **D**) donne le sulfamidoester **XVI** qui est saponifié selon la même méthode **G** pour donner le composé acide saturé correspondant **XVII**. Celui-ci peut aussi être obtenu à partir du dérivé cétonique correspondant **IX**, soit par réduction au triéthylsilane (méthode **F**), soit par hydrogénation catalytique en milieu acide fort (méthode **M**), soit par réduction selon la méthode de Clemmensen en milieu hydrogène naissant (zinc-acide chlorhydrique concentré) dans le toluène (méthode **N**). La dialcoylation de **XVII** en présence d'halogénures (bromure, iodure) $R_3I$ ou du sulfate correspondant $(R_3)_2SO_4$ conduit, après disodation, au composé estérifié et N-alcoylé avec $R_1 = R_3$ de formule **XVIII** (méthode **L**).

Selon le schéma **I**, le carbonyle de l'intermédiaire **IVa** peut être réduit en méthylène pour donner l'halogénoester **XIX** soit à l'aide du couple $Et_3SiH/CF_3COOH$ (méthode **F**), soit par hydrogénation catalytique en milieu acide fort (méthode **M**), soit par réduction selon Clemmensen en milieu Zn-HCl concentré en présence de toluène à chaud (méthode **N**).

Ce bromoester **XIX** se condense facilement à chaud dans le DMF avec le sel de sodium des sulfonamides secondaires de formule générale **XX**

$R\text{-}SO_2NH\text{-}R_7$ **XX**

dans laquelle $R_7=R_1$ (sauf H), ou $COOR_6$ avec $R_6$=alcoyle ramifié ou non ou benzyle pour donner le sulfamide totalement substitué sur l'azote **XXI** (méthode **I**). La saponification selon la méthode **G** conduit à l'acide correspondant **XXII**. Celui-ci peut être estérifié à chaud en milieu alcoolique ($R_3OH$) en présence d'acide fort ($H_2SO_4$ concentré) (méthode **K**) pour donner l'ester correspondant **XXIII**.

L'intermédiaire de départ **IV** peut aussi être directement condensé sur le sel de sodium du composé de formule générale **XX** pour donner le sulfonamide correspondant (méthode **I**), de formule **XXIV**. Celui-ci peut être saponifié dans les conditions opératoires **G** pour donner le céto, sulfonamido acide correspondant **XXV**. Dans le cas où $R_7$ représente un radical tertiobutoxycarbonyle, la réaction est précédée par le clivage du groupement en milieu chlorhydrique dans l'acétate d'éthyle (méthode **P**) pour donner l'ester **XXV** avec $R_1$=H qui est ensuite saponifié en acide $R_3$=H selon la méthode **G**. La fonction cétone du **composé XXIV** peut aussi être réduite totalement ou partiellement comme pour le **composé IX**. La réduction partielle de **XXIV** fournit l'hydroxy sulfamido ester **XXVI** (méthodes **C** et **E**) ; ce dérivé est ensuite saponifié pour donner l'acide dérivé **XXVII** (méthode **G**) qui peut être estérifié en **composé XXVIII** dans l'alcool à chaud en milieu sulfurique (méthode **K**). La réduction totale du carbonyle de **XXIV** en $CH_2$ (**composé XXI**) est réalisée selon une des trois méthodes **F**, **M** et **N** précédemment décrites. Le sulfamido ester est aisément saponifié selon le procédé **G** en acide correspondant **XXII**.

**2) Cas où Y représente dans IV un alcoxy ou benzyloxycarbonyle** (schéma de synthèse **II**)

Le carbamate cétoester de formule générale **IVb** où $R_6$ représente un alcoyle linéaire léger ($C_1$ à $C_8$) peut aussi être réduit partiellement en dérivé hydroxylé ou totalement en $CH_2$. La réduction partielle de **IVb** en

**composé XXIX** est réalisée dans un solvant alcoolique en présence de borohydrure (méthode E). La déprotection en aminohydroxy acide XXX selon la méthode G doit être réalisée en milieu basique plus concentré et à chaud. L'aminohydroxy acide XXX, ou son sel de sodium brut, est ensuite condensé avec un halogénure de sulfonyle de formule générale VII en milieu aqueux basique par exemple dans la soude à un pH compris entre 9 et 11 pour donner, après acidification, l'acide XXVII.

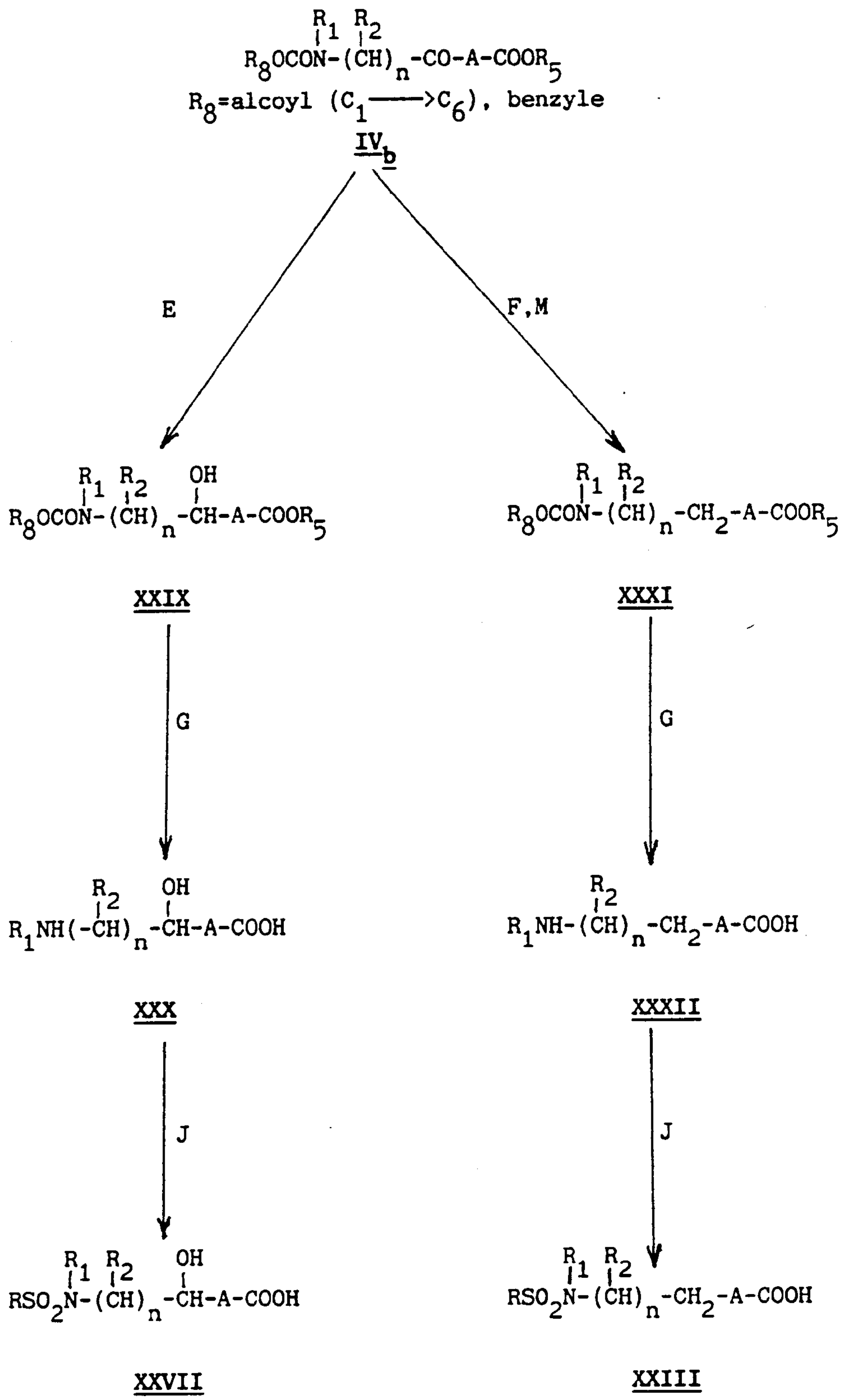


IVb XXIX XXXI XXX XXXII XXVII XXIII

**Schéma de synthèse II**

Parallèlement, la réduction du carbonyle de <u>$IV_b$</u> en $CH_2$ peut être réalisée selon les méthodes <u>F</u>, <u>M</u> pour donner le **composé** <u>XXXI</u> déprotégé dans les mêmes conditions que <u>XXX</u> en amino acide correspondant <u>XXXII</u>. Cet amino acide, ou son sel de sodium à l'état brut est condensé avec l'halogénure de sulfonyle <u>VII</u>

selon la méthode K pour fournir le **composé XXII**.

**3) Les dérivés renfermant un hétérocycle -A- insaturé** sont préparés à partir des esters saturés de formule générale **XXI** ou **XXIV** par bromation substituante à l'aide de N-bromosuccinimide et débromhydratation simultanée à chaud dans un solvant chloré comme le $CCl_4$ par exemple pour donner l'ester correspondant **XXXIII** (méthode **R**) puis saponification selon la méthode **G** pour conduire à l'acide **XXXIV** en série furannique par exemple

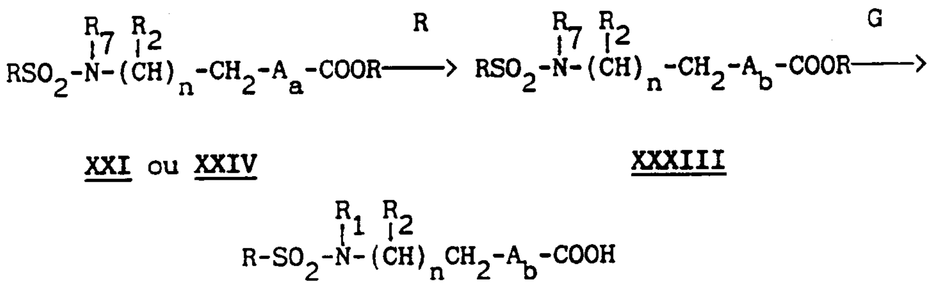


XXI ou XXIV

XXXIII

XXXIV

**Schéma de synthèse III**

**4) Les sels organiques ou minéraux** des composés de formule générale **I** où $R_3$=H sont préparés par réaction stoëchiométrique entre le **composé I** ($R_3$=H) et une base organique ou minérale (sous forme hydroxylée ou d'alcoolate) dans un solvant alcoolique ou un mélange alcool-eau ou dans l'acétone. Le sel est récupéré par filtration (si insoluble) ou par évaporation du solvant à siccité et recristallisation éventuelle.

**Exemple 1**

**Préparation de l'acide (parachlorobenzènesulfonamidoacétyl)-5 dihydro-2,3 benzofuranecarboxylique-2 (composé 1)**

**a**) Bromacétyl-5, dihydro-2,3 benzofuranecarboxylate-2 d'éthyle **IV** (Y=Br, $R_2$=H, A=a, $R_5$=Et, n=1) (**1a**) (méthode **A**)

Une solution de 42,5 g (0,22 mole) de dihydro-2,3 benzofuranecarboxylate-2 d'éthyle et 58,1 g (0,288 mole) de bromure de bromacétyle dans 500 ml de chlorure de méthylène est refroidie à 0°C puis traitée en 15 minutes avec 73,3 g (0,55 mole) de chlorure d'aluminium anhydre.

Le mélange est agité une heure de plus à 0°C puis on laisse revenir lentement à 20°C en 3 heures. Le mélange est hydrolysé à 0°C avec de l'acide chlorhydrique concentré de la manière habituelle puis extrait au chlorure de méthylène, lavé à l'eau, au bicarbonate de sodium dilué, séché et évaporé à siccité (80 g). Le résidu est recristallisé de 250 ml d'alcool isopropylique bouillant en amorçant sous agitation. A froid, on récupère 56 g (Rdt = 81 %) d'intermédiaire de formule **1a**

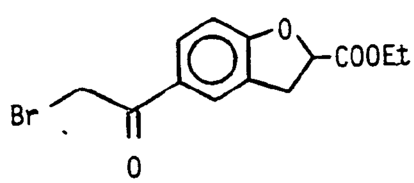

**Composé 1a**

- -formule brute : $C_{13}H_{13}BrO_4$
- -masse moléculaire : 313,152

- cristaux blanc cassé
- point de fusion : 70°C
- IR (KBr) : √CO 1675, √COOEt 1750 $cm^{-1}$.
- MN ($CDCl_3$) δ : 1.32, t, 3H, $CH_3$ ; 3.40, q, 1H, $ArCH_2C(O)COOEt$ ; 3.62, q, 1H, $ArCH_2C(O)COOEt$ ; 4.28, q, 2H, $COOCH_2Me$ ; 4.38, s, 2H, $BrCH_2CO$ ; 5.29, q, 1H, OCHCOOEt ; 6.94, d, 1H, Ar ortho $OCH_2$ ; 7.84 à 7.88, m, 2H, Ar ortho CO.

**b**) Azidoacétyl-5, dihydro-2,3 benzofuranecarboxylate-2 d'éthyle **V** ($R_2$=H, n=1, A=a, $R_5$=Et) (**1b**) (méthode **B**)

Un mélange de 10 g (32 mmoles) de bromoacétyl-5 dihydro-2,3 benzofuranecarboxylate-2 d'éthyle (**1a**) dans 130 ml d'éthanol est refroidie sur bain de glace puis traité par une solution de 2,3 g (35 mmoles) d'azoture de sodium dans 6 ml d'eau ajoutée goutte à goutte en 10 mn. Le bain de glace est retiré puis la solution est agitée pendant 5 h à 25°C.

Le dérivé attendu précipite. Après abandon pendant 16 h au réfrigérateur, on récupère de la manière habituelle les cristaux de composé de formule **1b**

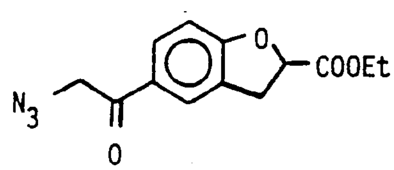

**Composé 1b**

- formule brute : $C_{13}H_{13}N_3O_4$
- masse moléculaire : 275,264
- cristaux blancs
- point de fusion : 85°C
- IR (KBr) : √CO 1683, √COOEt 1738, $√CN_3$ 2125 $cm^{-1}$.
- RMN ($CDCl_3$) δ : 4.49, s, 2H, $N_3CH_2CO$.

**c**) Chlorhydrate d'aminoacétyl-5, dihydro-2,3 benzofurannecarboxylate-2 d'éthyle **VI** ($R_2$=H, n=1, A=a, $R_5$=Et) (**1c**) (méthode **C**).

Une solution de 9,2 g (33,4 mmoles) d'azidoacétyl-5, dihydro-2,3 benzofuranecarboxylate-2 d'éthyle dans 500 ml de méthanol et 30 ml d'acide chlorhydrique N est hydrogénée de la manière habituelle en présence de 1,5 g de palladium sur charbon à 10 % sous courant d'hydrogène pendant 2 h 30. Après purge à l'azote, le catalyseur est éliminé par filtration, le filtrat est évaporé à siccité, le résidu est repris dans 100 ml d'alcool isopropylique et agité une nuit à 25°C.

Les cristaux sont constitués par le composé de formule **1c** et sont récupérés de la manière habituelle (m = 6 g - Rdt = 63 %).

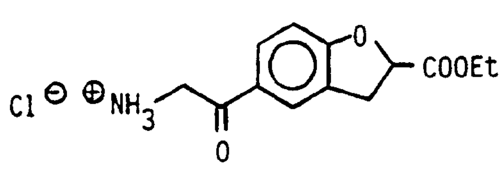

**Composé 1c**

- formule brute : $C_{13}H_{16}ClNO_4$
- masse moléculaire : 285,727
- cristaux blancs
- point de fusion : 152°C
- IR (KBr) : √NH 3000, √COOEt 1740, √CO 1680 $cm^{-1}$.

- RMN ($CDCl_3$) δ : 4.5 à 4.7, m, 2H, N<u>CH</u>$_2$CO.

**d**) (parachlorobenzènesulfonamidoacétyl)-5, dihydro-2,3 benzofuranecarboxylate-2 d'éthyle **VIII** ((R=p-$ClC_6H_4$-, $R_2$=H, n=1, A=a, $R_5$=Et ; (et pour I $R_1$=H et X=CO) (**1d**) (méthode D).

Une suspension de 5,8 g (20,2 mmoles) de chlorhydrate d'aminoacétyl-5 dihydro-2,3 benzofuranecarboxylate-2 d'éthyle (**1c**) dans 65 ml de pyridine refroidie à -5°C est traitée avec 5,5 g (26,1 mmoles) de chlorure de parachlorobenzènesulfonyle. Le mélange est maintenu 1/4 d'heure à cette température puis l'agitation est poursuivie pendant 2 heures après retour à 20°. Le mélange réactionnel est hydrolysé avec 100 ml d'eau puis extrait à l'acétate d'éthyle. La phase organique est lavée 2 fois avec 100 ml d'eau dont le pH est amené à 5,5 par addition de HCl concentré puis lavée avec de l'eau salée, séchée sur sulfate de sodium et évaporée. Le résidu fournit deux jets de cristaux de formule **1d** par solubilisation dans l'acétate d'éthyle et précipitation à l'éther ou à l'éther isopropylique (m = 6,3 g - Rdt = 73 %)

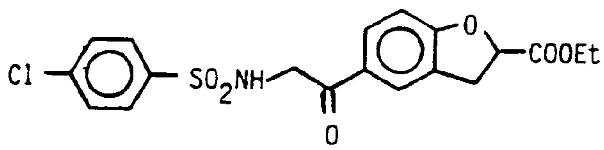

**Composé 1d**

- formule brute : $C_{19}H_{18}ClNO_6S$
- masse moléculaire : 423,871
- cristaux blancs cassé
- point de fusion : 153°C
- IR (KBr) √$SO_2$N 1160-1350, √CO 1688, √COOEt 1738, √NH 3300 $cm^{-1}$.
- RMN ($CDCl_3$) δ : 4.39, d, 2H, N<u>CH</u>$_2$CO ; 5.8, t, 1H, <u>NH</u> ; 7.42 à 7.48, dd, 2H, Ar ortho de Cl 7.78 à 7.84, dd, 2H, Ar ortho de $SO_2$

**e**) Saponification (méthode **G**), synthèse de l'acide parachlorobenzènesulfonamidoacétyl-5 dihydro-2,3 benzofuranecarboxylique-2 **IX** (R=p$ClC_6H_4$, $R_2$=H, A=a, (I $R_1$=H, $R_3$=H, X=CO) (1)

A 25°C, une solution de 3,8 g (9 mmoles) de parachlorobenzènesulfonamidoacétyl-5 dihydro-2,3 benzofuranecarboxylate-2 d'éthyle (**1d**) dans 40 ml de méthanol est amenée à pH 11,8 par addition de 10 ml de solution de soude N. Le pH est maintenu à 11,5 par addition goutte à goutte de 6 ml de soude N. L'agitation est poursuivie pendant 1 h 30 puis le mélange est refroidi avec de la glace et on acidifie à pH 3,3 par addition d'acide chlorhydrique N. Après 20 mn d'agitation à 0°C, les cristaux du dérivé attendu sont collectés, rincés à l'eau glacée et séchés (2,7 g). Par recristallisation dans l'alcool isopropylique, on récupère 2,05 g (Rdt = 58 %) de cristaux de **composé** 1 de formule :

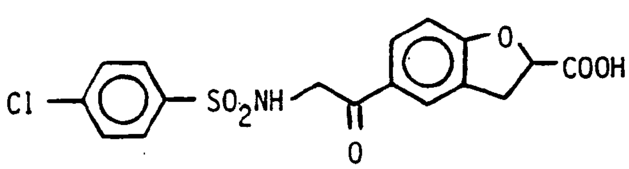

**Composé 1**

- formule brute : $C_{17}H_{14}ClNO_6S$
- masse moléculaire : 395,817
- cristaux blancs cassé
- point de fusion : 124°C
- IR (KBr) : √$SO_2$N 1170-1360, √CO 1695, √COOH 1730, √NH 3300 $cm^{-1}$.
- RMN (($CDCl_3$) δ : 5.31, q, 1H, OC<u>H</u>COO
- CCM : gel de silice 60 Merck F 254

éluant : chloroforme-méthanol-acide acétique : 80-18-02
Rf = 0,48

- Soluble à 10 % dans le DMSO.

L'exemple 2 suivant illustre une seconde voie de synthèse des sulfonamides objets de la présente invention. Toutefois, le composé 2, obtenu par cette synthèse n'entre pas dans la définition de la formule générale I.

**Exemple 2**

**Préparation de l'acide (parachlorobenzènesulfonamido-2 éthyl)-5 dihydro-2,3 benzofuranecarboxylique-2 (composé 2)**

**a**) sel de sodium du N-éthoxy carbonyl parachlorobenzènesulfonamide **XX** ($R_7$=EtOCO, R=p-$ClC_6H_4$ ; sel de Na) (**2a**)

Un mélange formé de 85 g (0,443 mole) de parachlorobenzènesulfonamide et de 157,2 g (1,13 mole) de $K_2CO_3$/KI 98/02 dans 500 ml d'acétone est traitée à 25°C goutte à goutte en 30 mn avec 62,6 g (0,576 mole) de chloroformiate d'éthyle en agitant mécaniquement. La réaction est légèrement exothermique et, après stabilisation de la température à 40°C, on porte progressivement au reflux pendant 2 h. Après refroidissement, le mélange est versé dans 500 ml de glace pilée et le sulfonamide est extrait à l'éther. Le résidu obtenu après lavage, séchage et évaporation à siccité est dissous dans 80 ml d'éther isopropylique chaud puis on précipite par addition de cyclohexane pour obtenir 100 g (Rdt = 86 %) de dérivé blanc de formule :

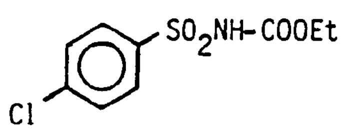

- formule brute : $C_9H_{10}ClNO_4S$
- masse moléculaire : 263,70
- cristaux blancs cassé
- point de fusion : 80°C.

Vingt grammes (75,8 mmoles) de sulfonamide précédent sont ajoutés à 25°C à une solution méthanolique de méthylate de sodium (préparé à partir de 1,75 g (75,8 mmoles) de sodium) en refroidissant sur bain d'eau froide et ensuite on agite pendant 2 h 30 à 25°C. Le mélange est évaporé à siccité puis le résidu est trituré et agité une nuit dans 250 ml d'éther. L'insoluble formé par le **composé 2a** est récupéré par filtration (m = 14 g - Rdt = 65 %).

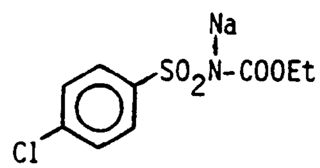

**Composé 2a**

- formule brute : $C_9H_9ClNNaO_4S$
- masse moléculaire : 285,68
- cristaux blancs
- point de fusion : 220°C.

**b**) (N-éthoxycarbonyl parachlorobenzènesulfonamidoacétyl)-5 dihydro-2,3 benzofuranecarboxylate-2 d'éthyle **XXIV** (R=p-$ClC_6H_4^-$, $R_7$=EtOCO, $R_2$=H, n=1, A=a, $R_5$=Et) (**2b**) (méthode **I**)

Un mélange de 12,8 g (44,8 mmoles) de sel de sodium du N-éthoxycarbonyl parachlorobenzènesulfonamide (**2a**) précédent dans 200 ml de DMF refroidi à 4°C sur bain de glace est traité rapidement avec 12,79 g (40,7 mmoles) de bromoacétyl-5 dihydro-2,3 benzofuranecarboxylate-2 d'éthyle préparé comme dans l'exemple **1a**. L'agitation est ensuite poursuivie une nuit à 25°C puis pendant 30 mn sur bain d'huile à 50°C. Après retour à 25°C, le mélange est versé sur 400 ml de glace pilée et le dérivé attendu est extrait à l'éther et récupéré de la manière habituelle. On obtient 22,4 g (Rdt = 100 %) de produit de formule **2b**. Le produit peut être utilisé brut ou purifié dans l'alcool isopropylique.

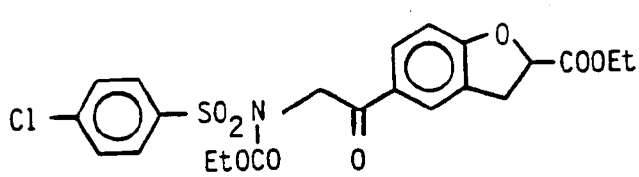

**Composé 2b**

- formule brute : $C_{22}H_{22}ClNO_8S$
- masse moléculaire : 495,934
- poudre blanchâtre amorphe
- point de fusion : 98°C
- IR (KBr) : √$SO_2$N 1170-1350, √CO 1700, √COOEt et NCOOEt 1755 $cm^{-1}$.
- RMN ($CDCl_3$) δ : 1.12, t, 2H, $NCOOCH_2C\underline{H_3}$ ; 4.12, q, 2H, $NCCOC\underline{H_2}CH_3$ ; 5.27, s, 3H, $COC\underline{H_2}N$ ; 7.51, d, 2H, Ar ortho de Cl ; 8.03, d, 2H, Ar ortho de $SO_2$.

**c)** ((N-éthoxycarbonyl parachlorobenzènesulfonamido)-2 éthyl)-5 dihydro-2,3 benzofuranecarboxylate-2 d'éthyle **XXI** (R=p-$ClC_6H_4$-, $R_7$=EtOCO-, $R_2$=H, A=a, $R_5$=Et) (méthode **F**)

Une solution de 22,4 g (40,7 mmoles) de (N-éthoxycarbonyl parachlorobenzènesulfonamidoacétyl)-5 dihydro-2,3 benzofuranecarboxylate-2 d'éthyle brut précédent (**2b**) dans 80 ml de $CF_3COOH$ est traitée à 25°C avec 19,5 ml (14,19 g ou 122 mmoles) de triéthylsilane et agité pendant 24 h. La solution formée est évaporée à siccité sous vide puis le résidu est repris dans du toluène et évaporé à siccité pour donner le **composé 2c** qui peut être utilisé brut (Rdt = 100 %) ou purifié sur colonne de silice normale en éluant avec un mélange de 60 % de cyclohexane, 30 % de chlorure de méthylène et 10 % d'acétate d'éthyle pour donner avec un rendement de 70 % le **composé 2c** de formule :

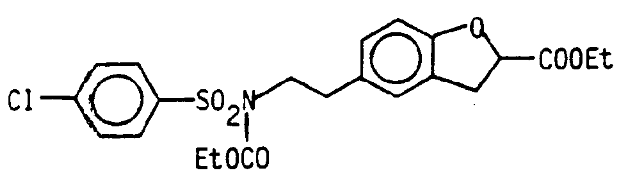

**Composé 2c**

- formule brute : $C_{22}H_{24}ClNO_7S$
- masse moléculaire : 481,951
- huile incolore
- IR (film) : √$SO_2$N 1170-1350, √COOEt et NCOOEt 1755 $cm^{-1}$.
- RMN ($CDCl_3$) δ : 2.96, t, 2H, $ArC\underline{H_2}CH_2N$ ; 4, t, 2H, $ArCH_2C\underline{H_2}N$ ; 7 à 7.06, m, 2H, Ar en ortho du $CH_2$.

**d)** Acide (parachlorobenzènesulfonamido-2 éthyl)-5 dihydro-2,3 benzofuranecarboxylique-2 **XXII** (R=p-$ClC_6H_4$, $R_1$=$R_2$=H, n=1, A=a et pour **I**, X=$CH_2$, $R_5$=H) (**composé 2**) (méthode **G**)

Le ((N-éthoxycarbonyl parachlorobenzènesulfonamido)-2 éthyl)-5 dihydro-2,3 benzofuranecarboxylate-2 d'éthyl (**2c**) brut précédent (40,7 mmoles) est repris dans 80 ml de dioxanne puis traité avec 80 ml de soude 5N et chauffé sous agitation à 50°C pendant 15 mn. Le mélange est versé dans 500 ml de glace et extrait à l'éther. La phase aqueuse est acidifiée à pH 5. L'acide attendu est extrait à l'acétate d'éthyle de la manière habituelle et le résidu est trituré dans l'éther puis agité pendant 1 h à 25°C. On récupère 11,45 g (Rdt = 74 %)

de cristaux blancs pulvérulents de **composé 2** ayant pour formule :

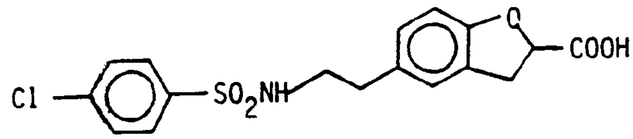

**Composé 2**

- formule brute : $C_{17}H_{16}ClNO_5S$
- masse moléculaire : 381,834
- cristaux blancs pulvérulents
- point de fusion : 153°C
- IR (KBr) : √$SO_2N$ 1160-1320, √COOH 1700, √NH 3240 $cm^{-1}$.
- RMN ($CDCl_3$) δ : identique à celle du **composé 4**.
- CCM : gel de silice 60 Merck F 254
  éluant : chloroforme-méthanol-acide acétique :
  80-18-02
  Rf = 0,45
- Soluble dans le DMSO à 10 %.

**Exemple 3**

**Préparation de l'acide (parachlorobenzènesulfonamido-2 hydroxy-1 éthyl)-5 dihydro-2,3 benzofuranecarboxylique-2 (composé 3)** (schéma de synthèse II)

**a**) Ethoxycarbonylaminoacétyl-5 dihydro-2,3 benzofuranecarboxylate-2 d'éthyle **IV** (Y=EtOCONH-, $R_2$=H, n=1, A=a, $R_5$=Et) (**3a**)

En utilisant le mode opératoire A décrit dans l'exemple 1 pour le **composé 1a** mais en substituant le bromure de bromacétyle par le chlorure d'éthoxycarbonylaminoacétyle préparé in situ à partir de 36,1 g (0.245 mole) d'acide correspondant et en le condensant sur 24.8 g (0,129 mole) de dihydro-2,3 benzofurane carboxylate-2 d'éthyle en présence de 86 g (0,645 mole) de chlorure d'aluminium, on obtient après purification sur colonne de silice ($C_6H_{12}$-AcOEt-$CH_2Cl_2$ : 60-20-20) et recristallisation dans l'éther isopropylique 11,4 g (Rdt = 28 %) de **composé 3a** de formule :

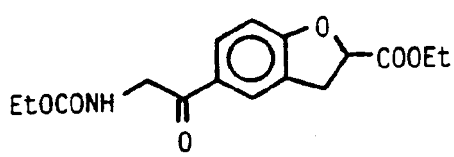

**Composé 3a**

- formule brute : $C_{16}H_{19}NO_6$
- masse moléculaire : 321,329
- cristaux blancs cassé
- point de fusion : 82°C
- IR (KBr) : √NCOOEt 1670, √CO 1700, √COOEt 1730 $cm^{-1}$.
- RMN ($CDCl_3$) δ : 1.24 à 1.35, m, 6H, 2 $CH_3$ ; 3.34 à 3.68, m, 2H, $OCHCH_2$ ; 4.10 à 4.33, m, 4H, 2 $CH_2$ ; 4.62, d, 2H, $NCH_2CO$ ; 5.29, q, 1H, $OCHCH_2$ ; 5.7, t, 1H, NH ; 6.95. d, 1H, Ar ortho 0 ; 7.84, m, 2H, Ar ortho CO.

**b**) (Ethoxycarbonylamino-2, hydroxy-1 éthyl)-5 benzofuranecarboxylate-2 d'éthyle **XXIX** ($R_8$=Et, $R_1$=$R_2$=H, n=1, A=a, $R_5$=Et) (**3b**) (méthode **E**)

Un mélange de 3,2 g (10 mmoles) d'(éthoxycarbonylamino-2 acétyl)-5 dihydrobenzofurannecarboxylate-2 d'éthyle précédent **3a** dans 30 ml d'éthanol est traité à 25°C avec 0,27 g (5 mmoles) de borohydrure de potassium. Après 2 h 30 d'agitation, on rajoute 0,11 g (2 mmoles) de $KBH_4$ supplémentaire et on maintient une heure de plus à 25°C. Le mélange réactionnel est versé sur de l'eau salée saturée, extrait à l'éther, puis lavé à l'eau et à l'eau salée et enfin séché sur sulfate et évaporé à siccité. Le résidu est purifié sur colonne de silice en éluant successivement avec un mélange hexane-acétate d'éthyle 90-10 puis cyclohexane-chlorure de méthylène-acétate d'éthyle 60-20-20. Les fractions renfermant le dérivé attendu sont évaporées à siccité puis le solide obtenu est repris dans l'éther isopropylique. Les cristaux pulvérulents du composé de formule **3b** sont récupérés de la manière habituelle (m = 1,7 g - Rdt = 53 %)

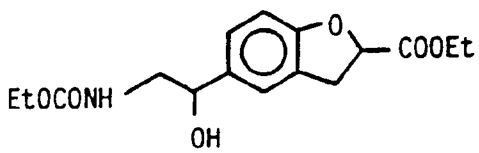

**Composé 3b**

- formule brute : $C_{16}H_{21}NO_6$
- masse moléculaire : 323,345
- cristaux pulvérulents blancs cassé
- point de fusion : 95°C
- IR (KBr):$\sqrt{}$NCOOEt 1680, $\sqrt{}$COOEt 1730-1750, $\sqrt{}$NH 3300, $\sqrt{}$OH 3370 $cm^{-1}$.
- RMN ($CDCl_3$) δ : 3.15 à 3.60, m, 5H, $ArC\underline{H}_2CHOAr$ et $NC\underline{H}_2CHO\underline{H}$ ; 4.67 à 4.75, m, 1H, $C\underline{H}OH$ ; 6.83, d, 1H, Ar ortho de $OCH_2$ ; 7.10, d et 7.18 s, 2H, Ar ortho CHOH.

**c**) Acide (amino-2 hydroxy-1 éthyl)-5 dihydro-2,3 benzofuranecarboxylique-2 **XXX** ($R_1$=$R_2$=H, n=1, A=a (**3c**) (méthode **G**)

En utilisant la méthode **G** décrite dans l'exemple **2d**, on obtient à partir de 3 g (9,3 mmoles) d'(éthoxycarbonyl-2 hydroxy-1 éthyl)-5 dihydro-2,3 benzofuranecarboxylate-2 d'éthyle et après 1 h 30 de chauffage au reflux une solution de sel de sodium de l'acide **XXX** de formule **3c** qui n'est pas isolée

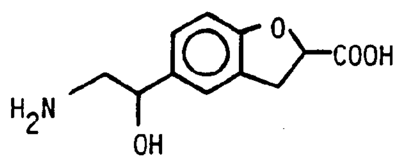

**Composé 3c**

**d**) Acide (parachlorobenzènesulfonamido-2 hydroxy-1 éthyl)-5 dihydro-2,3 benzofuranecarboxylique-2 **XXVII** (R=p-$ClC_6H_4$-, $R_1$=$R_2$=H, A=a et pour **I** $R_5$=H et X=CHOH) (**composé 3**) (méthode **J**)

La solution basique brute obtenue dans l'exemple précédent **3c** est diluée avec de la glace pilée et amenée à pH 11 par addition d'HCl concentré puis on ajoute 3 g (14,2 mmoles) de chlorure de parachlorobenzènesulfonyle. Le mélange est ensuite agité pendant 4 h à 25°C, retraité avec 0,5 g (≈ 2,4 mmoles) de chlorure précédent et agité 2 h de plus.

Le pH est amené à 12 par addition de soude puis extrait à l'éther. La phase aqueuse basique est acidifiée (HCl) jusqu'à pH 7 et extraite à l'acétate d'éthyle puis acidifiée jusqu'à pH 2 et extraite à l'acétate d'éthyle de la manière habituelle. Le résidu (2 g) obtenu après évaporation du solvant est purifié sur colonne de silice (20 g) en éluant avec un mélange AcOEt-AcOH : 99-01. Les fractions renfermant le dérivé attendu sont réunies et évaporées à siccité. Le résidu est trituré dans l'éther isopropylique et l'insoluble constitué du **composé 3**

est récupéré de la manière habituelle (m = 1,6 g - Rdt = 42 %)

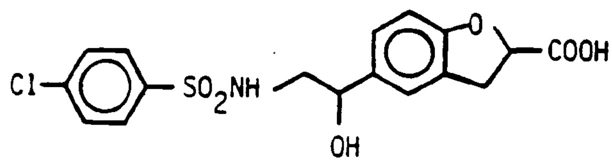

**Composé 3**

- formule brute : $C_{17}H_{16}ClNO_6S$
- masse moléculaire : 397,83
- cristaux blancs pulvérulents
- point de fusion : 77°C lent
- IR (KBr) : √$SO_2$NH 1150-1320, √COOH 1720, √NH 3400 $cm^{-1}$.
- RMN ($CDCl_3$) δ : 2.7 à 3, m, 2H, NC<u>H</u>$_2$CHOH ; 4.46 à 4.52, q, 1H, (C<u>H</u>OH ; 6.49 à 6.55, q, 1H, N<u>H</u> ; 7.30, d, 2H, Ar ortho Cl ; 7.60, d, 2H, Ar ortho $SO_2$.
- CCM : gel de silice 60 Merck F 254
  éluant : chloroforme-méthanol-acide acétique :
  80-18-02
  Rf = 0,35
- Soluble à 10 % dans le DMSO.

**Exemple 4**

**Préparation de l'acide (α-méthyl parachlorobenzènesulfonamido-acétyl)-5 dihydro-2,3 benzofuranecarboxylique-2 IX** (R=p-$ClC_6H_4$, $R_2$=Me, n=1, A=a et pour I X=CO et $R_5$=H) (**composé 4**) (Schéma de synthèse I)

**a**) α-bromopropionyl-5 dihydro-2,3 benzofurannecarboxylate-2 d'éthyle **IV** (Y=Br, $R_2$=Me, n=1, A=a, $R_5$=Et).

En adaptant le mode opératoire **A** décrit dans l'exemple **1a** au bromure d'α-bromopropionyle, on obtient, avec un rendement de 72 %, le **composé 4a** de formule :

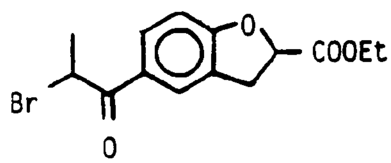

**Composé 4a**

- formule brute : $C_{14}H_{15}BrO_4$
- masse moléculaire : 327,179
- cristaux blancs
- point de fusion : 97°C
- IR (KBr) : √CO 1680, √COOEt 1760 $cm^{-1}$.
- RMN ($CDCl_3$) ; δ : 1.32, t, 3H, $CH_2CH_3$ ; 1.88, d, 3H, $CHCH_3$ 3.35 à 3.68, m, 2H, $OCHCH_2$ ; 4.28, q, 2H, $CH_2CH_3$ ; 5.19 à 5.34, m, 2H, $CHCH_3$ et $OCHCH_2$ ; 6.95, d, 1H, Ar ortho O ; 7.89 à 7.92, m, 2H, Ar ortho CO.

**b**) (α-azidopropionyl-5 dihydro-2,3 benzofuranecarboxylate-2 d'éthyle **V** ($R_2$=Me, n=1, A=a, $R_5$=Et).

En utilisant le mode opératoire utilisé pour préparer le **composé 1b**, on obtient, à partir de l'α-bromopropionyl-5 dihydro-2,3 benzofuranecarboxylate-2 d'éthyle avec un rendement de 100 %, le **composé 4b** de formule :

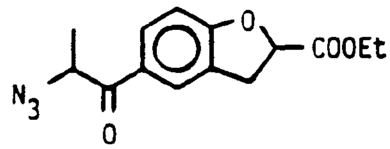

**Composé 4b**

- formule brute : $C_{14}H_{15}N_3O_4$
- masse moléculaire : 289,291
- huile incolore.

**c**) Chlorhydrate d'α-aminopropionyl-5 dihydro-2,3 benzofuranecarboxylate d'éthyle **VI** ($R_2$=Me, n=1, A=a, $R_5$=Et) (**4c**)

L'adaptation du mode opératoire **C** (Cf. exemple **1c**) à l'(α-azidopropionyl-5 dihydro-2,3 benzofuranecarboxylate-2 d'éthyle (**4b**) permet de préparer, avec un rendement de 61,4 %, le **composé 4c** de formule :

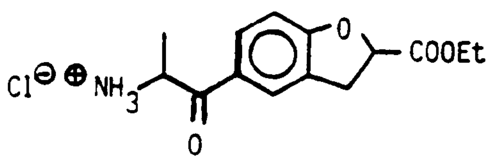

**Composé 4c**

- formule brute : $C_{14}H_{18}ClNO_4$
- masse moléculaire : 299,754
- cristaux blancs.

**d**) α-méthylparachlorobenzènesulfonamidoacétyl)-5 dihydro-2,3 benzofuranecarboxylate-2 d'éthyle **VIII** (R=p-$ClC_6H_4$-, $R_2$=Me, n=1, A=a, $R_5$=Et) (**4d**).

En adaptant le mode opératoire **D** (Cf. exemple **1d**) au chlorhydrate d'α-aminopropionyl-5 dihydro-2,3 benzofuranecarboxylate-2 d'éthyle, on obtient, avec un rendement de 45 %, le **composé 4d** de formule :

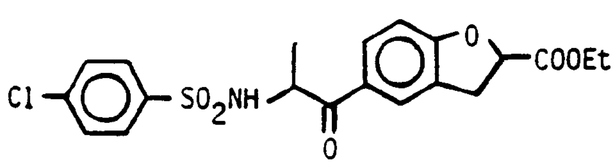

**Composé 4d**

- formule brute : $C_{20}H_{20}ClNO_6S$
- masse moléculaire : 437,898
- cristaux blancs cassé
- point de fusion : 137°C
- IR (KBr) : √$SO_2$N 1170-1355, √CO 1670, √COOEt 1755, √NH 3280 $cm^{-1}$.

**e**) Acide (α-méthyl parachlorobenzènesulfonamidoacétyl)-5 dihydro-2,3 benzofuranecarboxylique-2 (**composé 4**).

En appliquant le mode opératoire **G** (Cf. exemple **1e**) pour saponifier l'(α-méthyl parachlorobenzènesulfonamidoacétyl-5 dihydrobenzofurannecarboxylate-2 d'éthyle, on obtient avec un rendement de 63 % le

**composé 4** de formule :

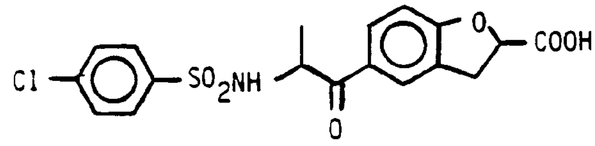

**Composé 4**

- formule brute : $C_{18}H_{16}ClNO_6S$
- masse moléculaire : 409,844
- cristaux blancs
- point de fusion : 134-136°C
- IR (KBr) : √$SO_2N$ 1175-1355, √CO 1670, √COOH 1730, √NH 3290 $cm^{-1}$.
- RMN ($CDCl_3$) δ : 1.37, d, 3H, $CH_3$ ; 3.33 à 3.70, m, 2H, $C\underline{H}_2CHO$; 4.84, m, 1H, $C\underline{H}CH_3$ ; 5.27, q, 1H, $CH_2C\underline{H}O$ ; 6.1, d, 1H, N$\underline{H}$ ; 6.9, d, 1H, Ar ortho O ; 7.33, d, 2H, Ar ortho Cl ; 7.62 à 7.8, m, 4H, Ar ortho $SO_2$ et Ar ortho CO.
- CCM : gel de silice 60 Merck F 254

  éluant : chloroforme-méthanol-acide acétique :

  80-18-02

  Rf = 0,49
- Soluble à 10 % dans le DMSO.

**Exemple 5**

**Acide (parachlorobenzènesulfonamido-2 hydroxy-1 propyl)-5 dihydro-2,3 benzofuranecarboxylique-2 XIV** (R=p-Cl-$C_6H_4$-, $R_2$=Me, n=1, A=a, et pour I $R_1$=$R_3$=H, et X=CHOH) (Schéma **I**) (**Composé 5**)

**a**) (amino-2 hydroxy-1 propyl)-5 dihydro-2,3 benzofuranecarboxylate-2 d'éthyle **XII** ($R_2$=Me, n=1, A=a, $R_5$=Et) (**5a**).

En partant de 21 mmoles d'α-azidopropionyl-5 dihydro-2,3 benzofuranecarboxylate-2 d'éthyle déjà décrit dans l'exemple **4b** et en utilisant la méthode **C** mais en hydrogénant pendant une durée beaucoup plus longue, on obtient après purification sur colonne de silice et élution avec un mélange chloroforme-méthanol-ammoniaque: 90-09-01, 2,5 g (Rdt = 45 %) de **composé 5a** de formule :

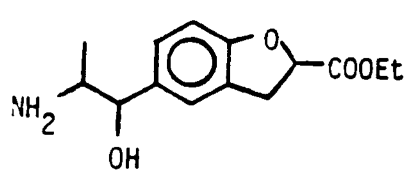

**Composé 5a**

- formule brute : $C_{14}H_{19}NO_4$
- masse moléculaire : 265,31
- cristaux blancs cassé
- point de fusion : 115°C
- IR (KBr) : √COOEt 1730, √NH et OH 3280-3340 $cm^{-1}$.
- RMN($CDCl_3$) δ : 0.86, d, 3H, $C\underline{H}_3CH$ ; 1.19, t, 3H, $C\underline{H}_3CH_2$ ; 2.95, m, 1H, $C\underline{H}CH_3$ ; 3.15 à 3.50, m, 2H, $ArC\underline{H}_2CHO$ ; 3.68, s, 1H, OH ; 4.13, q, 2H, $CH_3C\underline{H}_2$ ; 4.26, d, 1H, C$\underline{H}$OH ; 5.03 à 5.15, m, 1H, $ArCH_2C\underline{H}O$ ; 6.70, d, 1H, Ar ortho O ; 6.95, d, 1H et 7.07, s, 1H, Ar ortho CHOH.

**b**) (parachlorobenzènesulfonamido-2 hydroxy-1 propyl)-5 dihydro-2,3 benzofuranecarboxylate-2 d'éthyle **XIII** (R=p-Cl-$C_6H_4$, $R_2$=Me, n=1, $R_5$=Et) (**composé 5b**).

En utilisant l'(amino-2 hydroxy-1 propyl)-5 dihydro-2,3 benzofuranecarboxylate-2 d'éthyle précédent comme matière première et en adaptant la méthode D déjà décrite dans l'exemple 1c, on obtient avec un rendement de 73% le **composé 5b** de formule :

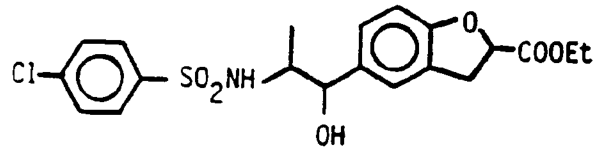

**Composé 5b**

- formule brute : $C_{20}H_{22}ClNO_6S$
- masse moléculaire : 439,91

**c**) Acide (parachlorobenzènesulfonamido-2 hydroxy-1 propyl)-5 dihydro-2,3 benzofuranecarboxylique-2 (**composé 5**).

A partir du **composé 5b** précédent (3,3 mmoles) et en utilisant la méthode G (Cf. exemple 1d), on obtient avec un rendement de 44 % le **composé 5** de formule :

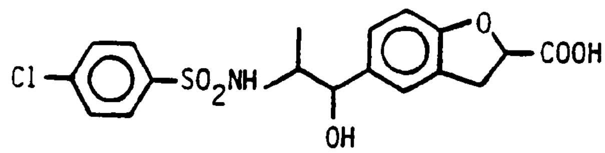

**Composé 5**

- formule brute : $C_{18}H_{18}ClNO_6S$
- masse moléculaire : 411,86
- cristaux blancs
- point de fusion : 185°C
- IR (KBr) : √$SO_2$N 1170-1335, √COOH 1710-1735, √NH 3350, √OH 3490 $cm^{-1}$.
- RMN ($CDCl_3$) δ : 0.79, d, 3H, $CH_3$ ; 4.5, d, 1H, CHOH ; 6.43, d, 1H, NH ; 7.35, d, 2H, Ar ortho Cl ; 7.65, d, 2H, ortho $SO_2$.
- CCM : gel de silice 60 Merck F 254
  éluant : chloroforme-méthanol-acide acétique :
  80-18-02
  Rf : 0,34
- Soluble dans le DMSO à 5 %.

**Exemple 6**

**Acide (parachlorobenzènesulfonamido-2 éthyl)-6 chromanecarboxylique-2 I** (R=p-Cl$C_6H_4$-, $R_1$=$R_2$=$R_3$=H, n=1, A=g, X=$CH_2$) (**composé 6**) (Schéma **I**)

**a**) Bromacétyl-6 chromanecarboxylate-2 d'éthyle **IV** (Y=Br, $R_2$=H, n=1, A=g, $R_5$=Et) (**composé 6a**).

En utilisant la méthode A selon l'exemple **1a** et en l'appliquant à 42 mmoles de chromanecarboxylate-2 d'éthyle, on obtient avec un rendement de 83 % le **composé 6a** de formule:

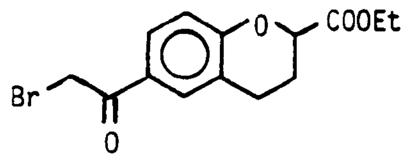

**Composé 6a**

- formule brute : $C_{14}H_{15}BrO_4$
- masse moléculaire : 327,18
- cristaux blancs pulvérulents
- point de fusion : 89°C
- IR (KBr) : √CO 1690, √COOEt 1755 cm$^{-1}$.
- RMN ($CDCl_3$) δ : 1.28, t, 3H, C<u>H</u>$_3$ ; 2.15 à 2.30, m, 2H, OCHC<u>H</u>$_2$ ; 2.7 à 2.9, m, 2H, OCHCH$_2$C<u>H</u>$_2$ ; 4.25, q, 2H, C<u>H</u>$_2$CH$_3$ ; 4.38, s, 2H, Br C<u>H</u>$_2$ ; 4.82, t, 1H, OC<u>H</u> ; 6.98, d, 1H, Ar ortho O ; 7.73 à 7.79, m, 2H, Ar ortho CO.

**b**) (N-éthoxycarbonyl parachlorobenzènesulfonamido) acétyl-6 chromanecarboxylate-2 d'éthyle <u>**XXIV**</u> (R=p-$ClC_6H_4$-, $R_7$=EtOCO-, $R_2$=H, n=1, A=g, $R_5$=Et) (**composé <u>6b</u>**)

En utilisant la méthode <u>**I**</u> décrite dans l'exemple <u>**2a**</u> et <u>**2b**</u> et en l'adaptant au **composé <u>6a</u>** précédent, on obtient avec un rendement de 91 % le **composé <u>6b</u>** de formule :

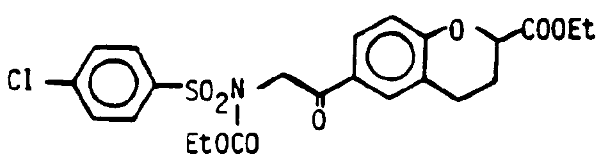

**Composé 6b**

- formule brute : $C_{23}H_{24}ClNO_8S$
- masse moléculaire : 509,96
- cristaux blancs pulvérulents
- point de fusion : 131°C
- IR (KBr) : √SO$_2$N 1170-1330, √CO 1690, √COOEt et NCOOEt 1745 cm$^{-1}$.
- RMN ($CDCl_3$) δ : 1.12, t, 3H, NCOOCH$_2$C<u>H</u>$_3$ ; 4.11, q, 2H, NCOOC<u>H</u>$_2$CH$_3$ , 5.28, s, 2H, NC<u>H</u>$_2$CO ; 7.51, d, 2H, Ar ortho Cl ; 8.02, d, 2H, Ar ortho SO$_2$.

**c**) Acide (parachlorobenzènesulfonamido-2 éthyl)-6 chromanecarboxylique-2 (**composé <u>6</u>**) <u>**XXII**</u> (R=p-$ClC_6H_4$-, $R_1$=$R_2$=H, n=1, A=g, et pour <u>**I**</u> $R_3$=H, X=$CH_2$).

L'adaptation du mode opératoire <u>**F**</u> décrit dans l'exemple <u>**2c**</u> à 9,8 mmoles de [(N-éthoxycarbonyl parachlorobenzènesulfonamido)acétyl]-6 chromanecarboxylate-2 d'éthyle permet de préparer avec un rendement quantitatif le composé de formule <u>**6c**</u> qui est saponifié brut sans purification

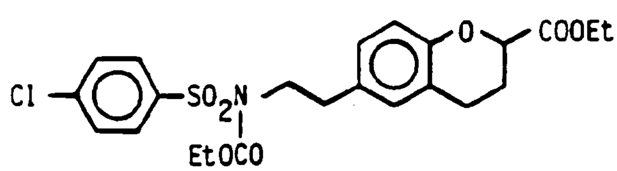

**Composé 6c**

Le **composé <u>6c</u>** est traité directement selon la méthode <u>**G**</u> décrite dans l'exemple <u>**2d**</u> pour donner après

recristallisation dans l'éthanol avec un rendement de 35 % le **composé 6** de formule :

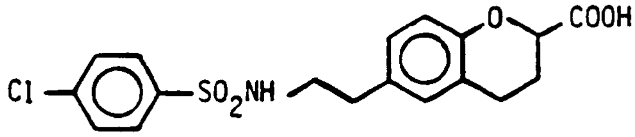

**Composé 6**

- formule brute : $C_{18}H_{18}ClNO_5S$
- masse moléculaire : 395,86
- cristaux blancs
- point de fusion : 142°C
- IR (KBr) : √$SO_2$NH 1165-1325, √COOH 1720, √NH 3300 $cm^{-1}$.
- RMN ($CDCl_3$) δ : 2.08 à 2.35, m, 2H, OCHC$H_2$C$H_2$ 2.63 à 2.78, m, 4H, OCHC$H_2$C$H_2$ et ArC$H_2$C$H_2$N ; 3.14, t, 2H, ArC$H_2$C$H_2$N ; 4.68, q, 1H, O CH ; 6.74 à 6.86, m, 3H, Ar ortho O et ortho C$H_2$ ; 7.45, d, 2H, Ar ortho Cl ; 7.72, d, 2H, Ar ortho $SO_2$.
- CCM : gel de silice 60 Merck F 254
  éluant : chloroforme-méthanol-acide acétique :
  80-18-02
  Rf : 0,67
- Soluble dans le DMSO à 20 %.

**Exemple 7**

**Acide (parachlorobenzènesulfonamido-2 éthyl)-5 benzodioxole-1,3 carboxylique-2 I** ($R_1$=$R_2$=$R_3$=H, n=1, A=c, X=$CH_2$) (Schéma I) (**composé 7**)

**a**) Bromacétyl-5 benzodioxole-1,3 carboxylate-2 d'éthyle **IV** ($R_2$=H, n=1, A=c, $R_5$=Et)

En utilisant le mode opératoire **A** décrit dans l'exemple **1a** mais en partant du benzodioxole-1,3 carboxylate-2 d'éthyle (20,6 mmoles) on obtient avec un rendement de 60 % le **composé 7a** de formule :

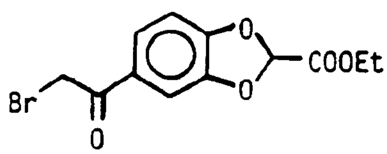

**Composé 7a**

- formule brute : $C_{12}H_{11}BrO_5$
- masse moléculaire : 315,124
- cristaux blancs
- point de fusion :50-51°C
- IR (KBr) : √CO 1685, √COOEt 1760 $cm^{-1}$.
- RMN ($CDCl_3$) δ : 1.34, t, 3H, $CH_3$ ; 4.32, q, 2H, O$CH_2$ ; 4.37, s, 2H, Br$CH_2$ ; 6.40, s, 1H, CH ; 6.94, d, 2H, Ar ortho O ; 7.51, d, 1H et 7.63, dd, 1H, Ar ortho CO.

**b**) [(N-éthoxycarbonyl parachlorobenzènesulfonamido)acétyl]-5 benzodioxole-1,3 carboxylate-2 d'éthyle **XXIV** (R=p-Cl$C_6H_4$-, $R_7$=COOEt, $R_2$=H, n=1, A=c, $R_5$=Et) (**composé 7b**).

L'adaptation de la méthode **I** décrite dans l'exemple **2b** à 11 mmoles de bromoacétyl-5 benzodioxole-1,3 carboxylate-2 d'éthyle permet de préparer avec un rendement quantitatif le **composé 7b** de formule :

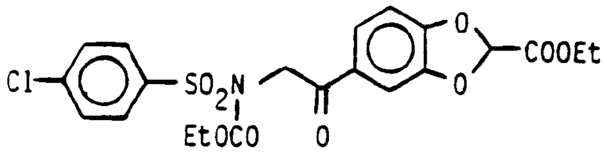

**Composé 7b**

- formule brute : $C_{21}H_{20}ClNO_9S$
- masse moléculaire : 497,906
- mousse incolore
- RMN ($CDCl_3$) δ : 1.13, t, 3H, $C\underline{H}_3CH_2OCON$ ; 4.12, q, 2H, $CH_3C\underline{H}_2OCON$ ; 5.26, s, 2H, $C\underline{H}_2N$ ; 7.52, d, 2H, Ar ortho Cl; 8.02, d, 2H, Ar ortho $SO_2$.

**c**) Acide (parachlorobenzènesulfonamido-éthyl)-5 benzodioxole-1,3 carboxylique-2 (**composé 7**).

En appliquant la méthode **F** décrite dans l'exemple **2c** à 11 mmoles de dérivé précédent **7b**, on obtient avec un rendement quantitatif l'intermédiaire **7c** de formule :

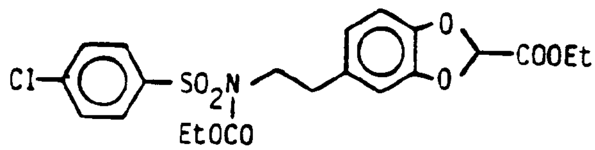

**Composé 7c**

Ce dérivé n'est pas purifié et est utilisé brut pour donner par saponification selon la méthode **G** (Cf. exemple **2d**) et purification dans l'éther isopropylique avec un rendement de 68 % le **composé 7** de formule :

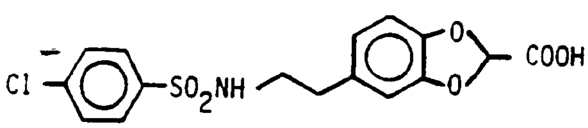

**Composé 7**

- formule brute : $C_{16}H_{14}ClNO_6S$
- masse moléculaire : 383,806
- cristaux blancs
- point de fusion : 138°C
- IR (KBr) : √$SO_2N$ 1165-1330, √COOH 1730, √NH 3295 $cm^{-1}$.
- RMN ($CDCl_3$) δ : 2.65, t, 2H, $ArC\underline{H}_2CH_2N$ ; 3.07, t, 2H, $ArCH_2C\underline{H}_2N$ ; 6.24, s, 1H, CH ; 6.52 à 6,59, m, 2H, Ar ortho O ; 6.71, d, 1H, Ar ortho $CH_2$ ; 7.42, d, 2H, Ar ortho Cl ; 7.70, d, 2H, Ar ortho $SO_2$.
- CCM : gel de silice 60 Merck F 254
  éluant : chloroforme-méthanol-acide acétique :
  80-18-02
  Rf : 0,34
- Soluble dans le DMSO à 20 %.

## Exemple 8

**Préparation de l'acide (parachlorobenzènesulfonamido-2 éthyl)-5 indanecarboxylique-2 I** ($R_1$=$R_2$=$R_3$=H, n=1, X=$CH_2$, A=f) (**composé 8**) (Schéma de synthèse I).

**a**) Bromoacétyl-5 indanecarboxylate-2 de méthyle.

En utilisant la méthode **A** décrite dans l'exemple **1a**, on obtient à partir de 17 mmoles d'indanecarboxylate-2 d'éthyle, et après purification dans l'éther isopropylique avec un rendement de 80 % le **composé 8a** de formule :

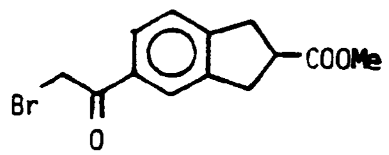

**Composé 8a**

- formule brute : $C_{13}H_{13}BrO_3$
- masse moléculaire : 297,153
- cristaux blancs
- point de fusion : 91°C
- IR (KBr) √CO 1690, √COOEt 1730 $cm^{-1}$.
- RMN ($CDCl_3$) δ : 3.27 à 3.41, m, 5H, $CH_2CHCH_2$ ; 3.74, s, 3H, $CH_3O$ ; 4.43, s, 2H, $BrCH_2CO$ ; 7.32, d, 1H, Ar meta CO ; 7.78 à 7.83, m, 2H, Ar ortho CO.

**b**) [(N-éthoxycarbonyl parachlorobenzènesulfonamido) acétyl]-5 indanecarboxylate-2 d'éthyle **XXIV** (R=p-$ClC_6H_4$, $R_7$=EtOCO, n=1, A=f, $R_5$=Et) (**composé 8b**).

En appliquant la méthode **I** décrite dans l'exemple **2b** à 13 mmoles de bromoacétyl-5 indanecarboxylate-2 d'éthyle, on obtient avec un rendement de 94 % le **composé 8b** de formule :

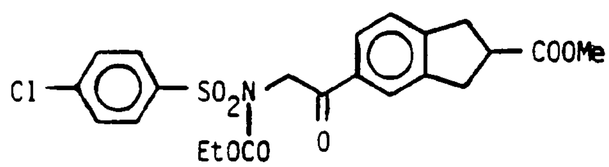

**Composé 8b**

- formule brute : $C_{22}H_{22}ClNO_7S$
- masse moléculaire : 479,935
- cristaux blancs
- point de fusion : 45°C
- IR (KBr) : √$SO_2N$ 1185-1330, √CO 1700, √COOEt et NCOOEt 1740 $cm^{-1}$.
- RMN ($CDCl_3$) δ : 1.12, t, 3H, $CH_3CH_2$ ; 4.13, q, 2H, $CH_3CH_2$ ; 5.32, s, 2H, $NCH_2CO$ ; 7.52, d, 2H, Ar ortho Cl 8.03, d, 2H, Ar ortho $SO_2$.

**c**) Acide (parachlorobenzènesulfonamido-2 éthyl)-5 indanecarboxylique-2 (**composé 8**)

En appliquant la méthode **F** décrite dans l'exemple **2c** à 12 mmoles de dérivé précédent **8b**, on obtient avec un rendement quantitatif l'intermédiaire **8c** de formule :

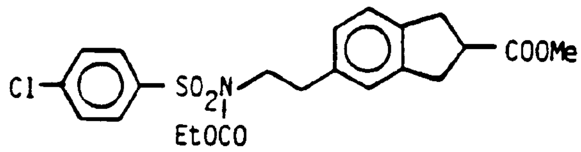

**Composé 8c**

Ce dérivé n'est pas purifié et est utilisé brut pour donner, après saponification selon la méthode G (exemple 2d) et après purification dans l'éther isopropylique avec un rendement de 63%, le **composé 8** de formule :

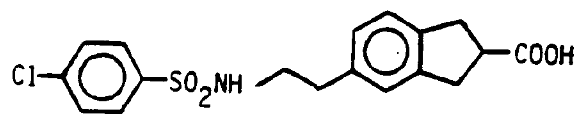

**Composé 8**

- formule brute : $C_{18}H_{18}ClNO_4S$
- masse moléculaire : 379,862
- cristaux blancs
- point de fusion : 140°C
- IR (KBr) : √$SO_2N$ 1158-1320, √COOH 1695, √NH 3250 $cm^{-1}$.
- RMN ($CDCl_3$) δ : 2.7, t, 2H, $ArCH_2CH_2N$ ; 3.03 à 3.32, m, 7H, $ArCH_2CH_2N$ et $CH_2CHCH_2$ ; 6.82 à 6.89, m, 2H, Ar ortho $CH_2CH_2N$ ; 7.05, d, 1H, Ar meta $CH_2CH_2N$ ; 7.4, d, 2H, Ar ortho Cl ; 7.7, d, 2H, Ar ortho $SO_2$.
- CCM : gel de silice 60 Merck F 254
  éluant : chloroforme-méthanol-acide acétique :
  90-09-01
  Rf : 0,40
- Soluble dans le DMSO à 10 %.

**Exemple 9**

**Préparation de l'acide (parachlorobenzènesulfonamido-2 hydroxyimino-1 éthyl)-5 dihydro-2,3 benzofuranecarboxylique-2** XI (R= p-Cl-$C_6H_4$, $R_2$=H, $R_4$=H, A=a, n=1, et pour I $R_1$=$R_3$=H, X=C=N-$OR_4$) (**composé 9**) (méthode H) (Schéma de synthèse I).

Une solution de 1 g (2,5 mmoles) d'acide (parachlorobenzènesulfonamidoacétyl)-5 dihydro-2,3 benzofuranecarboxylique-2 (Cf. exemple 1) dans 10 ml de pyridine est traitée avec 0,26 g (2,75 mmoles) de chlorhydrate d'hydroxylamine et agitée 20 h à 25°C. Le mélange est évaporé à siccité sous vide, repris dans du toluène, réévaporé puis dissous dans l'acétate d'éthyle et lavé à l'eau avec une solution d'acide citrique diluée puis est traité avec une solution diluée de bicarbonate de sodium de façon à obtenir un pH final de 5 et enfin lavé avec une solution d'eau salée. La phase organique est séchée sur sulfate, évaporée à siccité et triturée dans l'éther isopropylique pour donner 700 mg (Rdt = 68 %) de **composé 9** de formule :

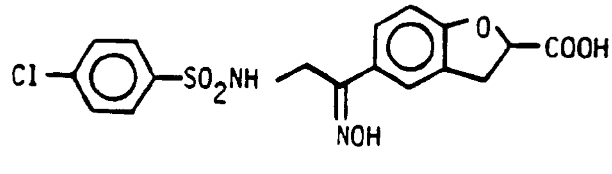

**Composé 9**

- formule brute : $C_{17}H_{15}ClN_2O_6S$
- masse moléculaire : 410,832
- poudre amorphe jaune clair

- point de fusion lent à partir de 90°C
- IR (KBr):√$SO_2$NH 1170-1335, √C=N 1595, √COOH 1730, √NH 3300 $cm^{-1}$.
- RMN ($CDCl_3$) δ : 3.74 et 3.93, 2s, 2H, $NC\underline{H_2}$ ; 7.49 et 7.63, 2d, 2H, Ar ortho $SO_2$.
- CCM : gel de silice 60 Merck F 254
  éluant : chloroforme-méthanol-acide acétique :
  80-18-02
  Rf : 0,32
- Soluble dans le DMSO à 10 %.

**Exemple 10**

**Préparation de l'acide (parachlorobenzènesulfonamido-2 méthoxyimino-1 éthyl)-5 dihydro-2,3 benzofuranecarboxylique-2 XI** (R= p-Cl-$C_6H_4$, $R_2$=H, $R_4$=Me, A=a, n=1, et pour I $R_1$=$R_3$=H, X=C=N=$OR_4$) (**composé 10**) (Schéma de synthèse I).

En utilisant la même méthode que celle décrite dans l'exemple 9 sur une même fraction molaire, mais en partant de la O-méthylhydroxylamine chlorhydratée (310 mg ; 3,8 mmoles) on obtient avec un rendement de 66 % le **composé 10** de formule :

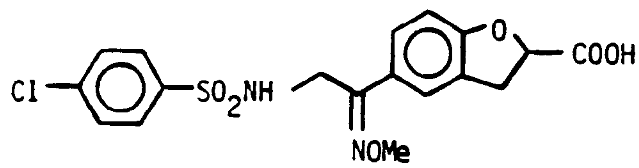

**Composé 10**

- formule brute : $C_{18}H_{17}ClN_2O_6S$
- masse moléculaire : 424,859
- poudre amorphe jaune clair
- point de fusion lent à partir de 64°C
- IR (KBr) : √$SO_2$NH 1170-1340; √C=N 1590; √COOH 1740; √NH 3300 $cm^{-1}$.
- RMN ($CDCl_3$) δ : 3.70 et 3.90, 2s, 3H, $OC\underline{H_3}$ ; 4 et 4.12, 2d, 2H, N $\underline{CH_2}$ ; 5.56 et 5.67, 2t, 1H, NH.
- CCM : gel de silice 60 Merck F 254
  éluant : chloroforme-méthanol-acide acétique :
  80-18-02
  Rf : 0,48
- Soluble dans le DMSO à 10 %.

**Exemple 11**

**Préparation du (parachlorobenzènesulfonamidoacétyl)-5 dihydro-2,3 benzofuranecarboxylate-2 d'éthyle VIII** (R= p-Cl-$C_6H_4$, $R_2$=H, $R_5$=Et, A=a, n=1, et pour I $R_1$=H, $R_3$=Et, X=CO) (**composé 1d**) (méthode K) (Schéma de synthèse I)

Un mélange de 3,95 g (10 mmoles) d'acide (parachlorobenzènesulfonamidoacétyl)-5 dihydro-2,3 benzofuranne carboxylique-2 (Cf. exemple 1) dans 30 ml d'éthanol anhydre et renfermant 0,2 ml d'acide sulfurique concentré est chauffé 2 heures au reflux puis après retour à 25°C, le mélange est évaporé à siccité au rotavapor sous vide. Le résidu est repris dans de l'acétate d'éthyle, lavé à l'eau avec une solution diluée de bicarbonate de sodium, puis à l'eau et à l'eau salée. La phase organique est séchée sur sulfate de sodium, évaporée à siccité et le résidu est trituré dans l'éther isopropylique pour donner avec un rendement de 85 % le **composé 1d** de formule :

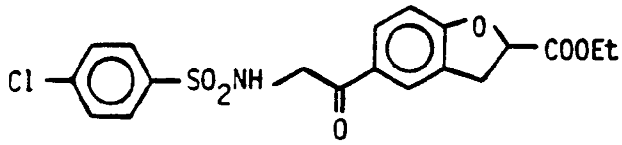

**Composé 1d**

Les caractéristiques physicochimiques de ce composé ont été déjà reportées dans l'exemple 1.

**Exemple 12**

**Préparation de l'acide (N-méthyl paratoluènesulfonamidoacétyl)-5 dihydro-2,3 benzofuranecarboxylique-2 XXV** (R=p-Me-$C_6H_4$, $R_7$=Me, $R_2$=H, A=a, n=1, et pour I $R_1$=Me, X=CO) (**composé 12**)

**a**) (N-méthyl paratoluènesulfonamidoacétyl)-5 dihydro-2,3 benzofuranecarboxylique-2 d'éthyle (**12a**).

En utilisant la méthode I décrite dans l'exemple **2b** mais en l'appliquant au sel de sodium du N-méthyl paratoluènesulfonamide (1 g ; 5,4 mmoles) et en le condensant sur 1,7 g (5,4 mmoles) de bromoacétyl-5 dihydro-2,3 benzofuranecarboxylate-2 d'éthyle, on obtient avec un rendement quantitatif le **composé 12a** de formule :

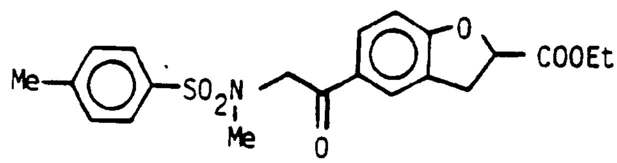

**Composé 12a**

- formule brute : $C_{20}H_{23}NO_6S$
- masse moléculaire : 405,461
- mousse blanchâtre qui n'est pas isolée.

**b**) Saponification du composé **12a** en dérivé ____ (méthode G).

L'ester brut précédent **12a** est saponifié selon la méthode G décrite dans l'exemple **1e** pour donner après purification sur colonne de silice (20 g) et élution avec un mélange chloroforme, méthanol, acide acétique 950/045/005 et cristallisation dans le cyclohexane, 1,1 g (Rdt = 52 %) de **composé 12** de formule :

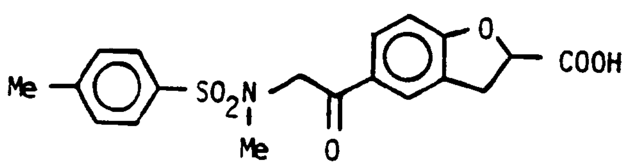

**Composé 12**

- formule brute : $C_{19}H_{19}NO_6S$
- masse moléculaire : 389,426
- cristaux blancs
- point de fusion : 149°C
- IR (KBr) : √$SO_2$N 1160-1335, √CO 1680, √COOH 1740 $cm^{-1}$.
- RMN ($CDCl_3$) δ : 2.39, s, 3H, MeN ; 2.74, s, 3H, MeAr ; 3.30 à 3.63, m, 2H, $OCHCH_2$ ; 4.4, s, 2H, $NCH_2CO$ ; 5.22, q, 1H, $OCHCH_2$ ; 6.87, d, 1H, Ar ortho O ; 7.28, d, 2H, Ar ortho $CH_3$ ; 7.65, d, 2H, Ar ortho $SO_2$ ; 7.8, m, 2H, Ar ortho CO.
- CCM : gel de silice 60 Merck F 254

éluant : chloroforme-méthanol-acide acétique :
80-18-02
Rf : 0,56
- Soluble dans le DMSO à 10 %.

**Exemple 13**

**Acide (parachlorobenzènesulfonamidoacétyl-5 dihydro-2,3 benzofuranecarboxylique XXV** (R= p-Cl-$C_6H_4$, $R_1$=$R_2$=H, n=1, A=a et pour I X=CO, $R_3$=4) (**composé 1**) (Méthode P, schéma de synthèse I).

**a**) Sel de sodium du N-tertiobutoxycarbonyl parachlorobenzènesulfonamide XX (R=p-Cl-$C_6H_4$, $R_7$=t-BuOCO-sel de Na) **composé 13a.**

En utilisant le mode opératoire décrit dans l'exemple **2a** mais en condensant 11,4 g (52 mmoles) de di-tert-butylpyrocarbonate sur 5 g (26 mmoles) de parachlorobenzènesulfonamide, on obtient après purification dans le cyclohexane avec un rendement de 70 % le composé de formule :

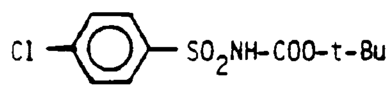

- formule brute : $C_{11}H_{14}ClNO_4S$
- masse moléculaire : 291,753
- cristaux blancs
- point de fusion : 128°C
- IR (KBr) : √$SO_2$N 1170-1345 ; √COO 1740 ; √NH 3270 $cm^{-1}$.

Le sel de sodium de ce composé est préparé à partir de 2 g (6,9 mmoles) de composé précédent selon le mode opératoire **2a** pour donner avec un rendement de 85 % le **composé 13a** de formule :

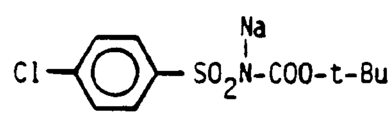

**Composé 13a**

- formule brute : $C_{11}H_{13}ClNNaO_4S$
- masse moléculaire : 313,746
- cristaux blancs
- point de fusion instantané 210-215°C avec décomposition
- IR (KBr) : √NCOO 1640 $cm^{-1}$.

**b**) (N-tertiobutoxycarbonyl parachlorobenzènesulfonamidoacétyl)-5 dihydro-2,3 benzofuranecarboxylate-2 d'éthyle **XXIV** (R= p-Cl-$C_6H_4$, $R_7$=COOtBu, $R_2$=H, n=1, A=a, $R_5$=Et) (**composé 13b**)

En partant de 1 g (3,2 mmoles) de sel de sodium du N-terbutoxycarbonyl parachlorobenzènesulfonamide et en le condensant avec 0,90 g (2,85 mmoles) de bromoacétyl-5 dihydro-2,3 benzofuranecarboxylate-2 d'éthyle (**composé 1a**) selon le procédé décrit dans l'exemple **2b**, on obtient avec un rendement de 90 % le **composé 13b** brut de formule :

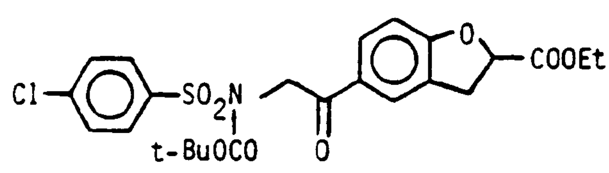

**Composé 13b**

- formule brute : $C_{24}H_{26}ClNO_8S$
- masse moléculaire : 523,99
- huile ambrée visqueuse
- RMN ($CDCl_3$) δ : 1.3, s, 9H, $(C\underline{H_3})_3C$ ; 3.36, t, 3H, $C\underline{H_3}CH_2$ ; 3.44 à 3.63, m, 2H, $OCHC\underline{H_2}$ ; 4.3, q, 2H, $C\underline{H_2}CH_3$ ; 5.24, s, 2H, $NC\underline{H_2}$ ; 5.31, q, 1H, $OC\underline{H}$ ; 6.98, d, 1H, Ar ortho O ; 7.52, d, 2H, Ar ortho Cl ; 7.85, m, 2H, Ar ortho CO ; 8.07, d, 2H, Ar ortho $SO_2$.

**c**) (parachlorobenzènesulfonamidoacétyl)-5 dihydro-2,3 benzofuranecarboxylate-2 d'éthyle XXV (R= p-Cl-$C_6H_4$, $R_1$=$R_2$=H, n=1, A=a, $R_3$=Et) (**méthode P**)

Une solution de 500 mg (0,95 mmoles) de composé précédent **13b** dans 20 ml d'acétate d'éthyle renfermant 2 moles d'HCl gazeux par litre est agitée pendant 2 h à 25°C puis évaporée à siccité. Le résidu est repris dans l'éther isopropylique et l'insoluble est récupéré (m = 0,32 g - Rdt = 77 %) et a pour formule :

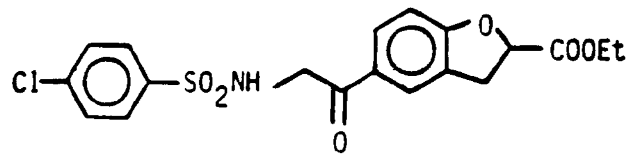

**Composé 1d**

Les caractéristiques physicochimiques de ce dérivé ont déjà été reportées dans l'exemple 1. Il peut être saponifié selon la méthode G pour donner le **composé 1**.

**Exemple 14**

**Préparation du (parachlorobenzènesulfonamidoacétyl)-6 chromanecarboxylate-2 d'éthyle XXV** (R=p-Cl-$C_6H_4$, $R_1$=$R_2$=H, $R_3$=Et, n=1, A=g) et pour I X = CO (**composé 14**) (schéma de synthèse I).

**a**) (N-tertiobutyloxycarbonyl parachlorobenzènesulfonamidoacétyl)-6 chromanecarboxylate-2 d'éthyle XXIV (R=p-Cl-$C_6H_4$, $R_7$=COO-tBu, $R_2$=H, $R_5$=Et, n=1, A=g) (**composé 14a**) (méthode I).

En utilisant le sel de sodium du N-tertiobutyloxycarbonyl parachlorobenzène sulfonamide XX R=p-Cl-$C_6H_4$, $R_7$=t-BuOCO- préparé selon l'exemple **13a** et en condensant 5,51 g (17,5 mmoles) de ce composé avec 5 g (15,2 mmoles) de bromoacétyl-5 chromane carboxylate-2 d'éthyle selon le procédé décrit dans l'exemple **13b**, on obtient avec un rendement de 99 % le composé **14a** engagé brut dans l'étape suivante ayant pour formule :

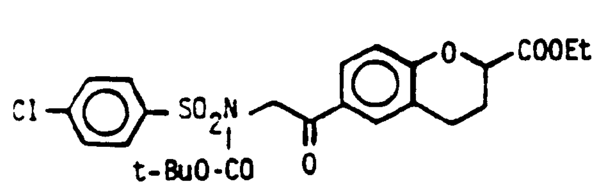

**Composé 14a**

- formule brute : $C_{25}H_{28}ClNO_8S$
- masse moléculaire : 523,99
- poudre blanc cassé
- point de fusion lent : 60°C
- IR (KBr) : √$SO_2$ 1170-1350 ; √CO 1700 ; √COOEt et NCOOtBu 1755 $cm^{-1}$.
- RMN ($CDCl_3$) δ : 1.3, m, 12H, $C(CH_3)_3$, $OCH_2C\underline{H_3}$ ; 3.40, q, 1H, $OCHC\underline{H_2}Ar$ ; 3.63, q, 1H, $OCHC\underline{H_2}Ar$ ; 4.28, q, 2H, $OC\underline{H_2}CH_3$ ; 5.3, m, 3H, $O\underline{CH}CH_2Ar$, $N\underline{HCH_2}CO$ ; 6.97, d, 1H, Ar ortho O ; 7.52, d, 2H, Ar ortho Cl ; 7.84, m, 2H, Ar ortho CO ; 8.03, d, 2H, Ar ortho $SO_2$.

**b**) parachlorobenzènesulfonamidoacétyl-6 chromanecarboxylate-2 d'éthyle XXV (R=p-Cl-$C_6H_4$, $R_1$=$R_2$=H, $R_3$=Et, A=g, n=1) (**composé 14**) (méthode P).

En traitant 8,2 g (15,2 mmoles) de composé **14a** précédent en présence de 100 ml d'acétate d'éthyle renfermant 2 moles d'HCl gazeux par litre selon l'exemple **13c**, et après recristallisation du résidu brut dans 100 ml d'un mélange éther isopropylique/acétate d'éthyle 90/10, on prépare avec un rendement de 73 % le composé **14** de formule :

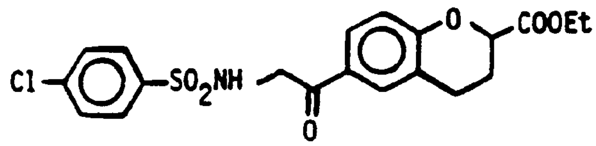

**Composé 14**

- formule brute : $C_{20}H_{20}ClNO_6S$
- masse moléculaire : 437,90
- cristaux blancs pulvérulents
- point de fusion : 150°C
- IR (KBr) : √$SO_2$N 1160-1350 ; √CO 1690 ; √COOEt 1740, √NH 3300cm$^{-1}$.
- RMN ($CDCl_3$) δ : 1.24, t, 3H, $CH_3$ ; 2.18, m, 2H, OCHC$H_2$Ar ; 2.73, m, 2H, OCHCH$_2$C$H_2$Ar ; 4.20, q, 2H, OC$H_2$CH$_3$ ; 4.35, s, 2H, NHC$H_2$CO; 4.78, t, 1H, OC$H$CH$_2$CH$_2$Ar ; 6.06, s, 1H, NH ; 6.90, d, 1H, Ar ortho O ; 7.40, d, 2H, Ar ortho Cl ; 7.58, m, 2H, Ar ortho CO ; 7.79, d, 2H, Ar ortho $SO_2$.
- CCM : gel de silice 60 Merck F 254
  éluant : hexane-acétate d'éthyle : 50-50
  Rf : 0,35
- Solubilité : soluble à 25 % dans le DMSO insoluble dans l'eau.

**Exemple 15**

**Préparation de l'acide (parachlorobenzènesulfonamidoacétyl)-6 chromanecarboxylique-2 IX** (R=p-Cl-$C_6H_4$, $R_2$=H, n=1, A=g) (**composé 15**) (schéma de synthèse I) (méthode G).

En partant de 3,9 g (8,9 mmoles) de parachlorobenzènesulfonamidoacétyl-6 chromanecarboxylate-2 d'éthyle (composé 15) et en saponifiant cet ester selon le procédé décrit dans l'exemple **1e**, et en purifiant sur une colonne de 20 g de silice/éluant $CHCl_3$/MeOH/AcOH 95/4,5/0,5, on prépare avec un rendement de 40 % le composé 15 ayant pour formule :

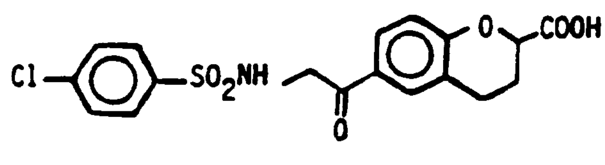

**Composé 15**

- formule brute : $C_{18}H_{16}ClNO_6S$
- masse moléculaire : 409,84
- cristaux blancs
- point de fusion : 174-175°C
- IR (KBr) : √COOH 1740 ; √CO 1690 ; √NH 3300 cm$^{-1}$.
- RMN ($CDCl_3$) δ : 2.18, m, 2H, OCHC$H_2$CH$_2$Ar ; 2.76, m, 2H, OCHCH$_2$C$H_2$Ar; 4.32, s, 2H, NHC$H_2$CO ; 4.74, t, 1H, OC$H$CH$_2$CH$_2$Ar ; 6.23, s, 1H, NH ; 6.90, d, 1H, Ar ortho O 7.40, d, 2H, Ar ortho Cl ; 7.57, m, 2H, Ar ortho CO ; 7.78, d, 2H, Ar ortho $SO_2$.
- CCM : gel de silice 60 Merck F 254
  éluant : chloroforme, méthanol, acide acétique
  80/18/02

Rf : 0,35

- Solubilité : soluble à 25 % dans le DMSO insoluble dans l'eau.

**Exemple 16**

**Préparation de l'acide (benzènesulfonamidoacétyl)-5 dihydro-2,3 benzofuranecarboxylique-2 IX** (R=$C_6H_5$, $R_2$=H, n=1, A=a) et pour I (X=CO, $R_1$=$R_3$=H) (**composé 16**) (schéma de synthèse I).

**a**) (benzènesulfonamidoacétyl)-5 dihydro-2,3 benzofuranecarboxylate-2 d'éthyle **VII** (**composé 16a**) (R=$C_6H_5$, $R_2$=H, A=a, $R_5$=Et) (méthode D)

En traitant 3 g (10,4 mmoles) de chlorhydrate d'(aminoacétyl)-5 dihydro-2,3 benzofuranecarboxylate-2 d'éthyle (composé **1c**) avec 2,8 g (15,7 mmoles) de chlorure de benzènesulfonyle selon le mode opératoire **1d**, on obtient après recristallisation dans l'alcool isopropylique avec un rendement de 75 % le composé **16a** de formule :

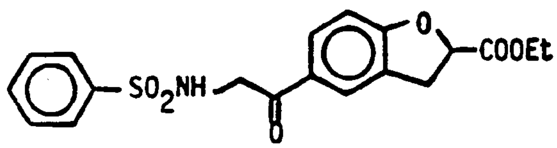

**Composé 16a**

- formule brute : $C_{19}H_{19}NO_6S$
- masse moléculaire : 389,43
- cristaux blanc cassé
- point de fusion : 109°C
- RMN ($CDCl_3$) δ : 1.30, t, 3H, $OCH_2CH_3$; 3.53, q, 1H, $OCHCH_2Ar$ ; 3.81, q, 1H, $OCHCH_2Ar$ ; 4.27, q, 2H, $OCH_2CH_3$ 4.63, d, 2H, $NHCH_2CO$ ; 5.93, q, 1H, $OCHCH_2Ar$ ; 6.71, t, 1H, NH ; 7.01, d, 1H, Ar ortho O ; 7.62 à 8.10, m, 7H, Ar.

**b**) acide benzènesulfonamidoacétyl-5 dihydro-2,3 benzofuranecarboxylique-2 **IX** (**composé 16**) (R=$C_6H_5$, $R_2$=H, n=1, A=a) (méthode G)

En opérant comme dans l'exemple **1e** mais en partant de 2,95 g (7,57 mmoles) d'ester précédent **16a**, on obtient après purification sur colonne de silice avec un rendement de 40 % le composé **16** de formule :

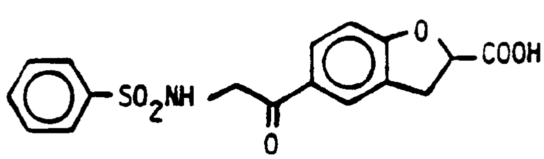

**Composé 16**

- formule brute : $C_{17}H_{15}NO_6S$
- masse moléculaire : 361,37
- cristaux blanc cassé
- point de fusion : 179°C
- IR (KBr) : √$SO_2$ 1180-1346 ; √CO 1690 ; √COOH 1750 ; √NH 3300 $cm^{-1}$
- RMN ($CDCl_3$) δ : 3.53, q, 1H, $OCHCH_2Ar$ ; 3.81, q, 1H, $OCHCH_2Ar$ ; 4.63, d, 2H, $NHCH_2CO$ ; 5.93, q, 1H, $OCHCH_2Ar$ 6.71, t, 1H, NH ; 7.01, d, 1H, Ar ortho O ; 7.62 à 8.10, m, 7H, Ar.
- CCM : gel de silice 60 Merck F 254

éluant : chloroforme, méthanol, acide acétique

80/18/02

Rf : 0,30

- Solubilité : soluble à 25 % dans le DMSO insoluble dans l'eau.

**Exemple 17**

**Préparation de l'acide paraméthoxybenzènesulfonamidoacétyl-5 dihydro-2,3 benzofuranecarboxylique-2 IX** (R=p-$CH_3O$-$C_6H_4$-, $R_2$=H, n=1, A=a) et pour I ($R_1$=$R_3$=H et X=CO) (**composé 17**) (schéma de synthèse I) (méthode D)

**a**) paraméthoxybenzènesulfonamidoacétyl-5 dihydro-2,3 benzofuranecarboxylate-2 d'éthyle **VIII** (**composé 17a**) (R=p-$CH_3O$-$C_6H_4$-, $R_2$=H, A=a, $R_5$=Et, n=1)

En mettant en réaction 3 g (10,4 mmoles) de chlorhydrate d'aminoacétyl-5 dihydro-2,3 benzofuranecarboxylate-2 d'éthyle (composé 1c) avec 3 g (14,5 mmoles) de chlorure de paraméthoxybenzènesulfonyle selon le procédé décrit dans l'exemple **1d**, on obtient quantitativement le composé **17a** de formule :

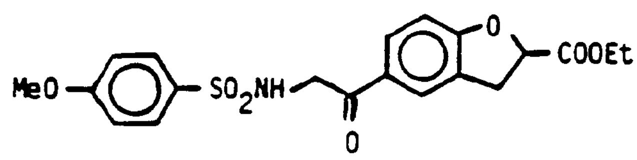

**Composé 17a**

- formule brute : $C_{20}H_{21}NO_7S$
- masse moléculaire : 421,46
- huile visqueuse ambrée

**b**) acide paraméthoxybenzènesulfonamidoacétyl-5 dihydro-2,3 benzofuranecarboxylique-2 **IX** (**composé 17**) (R=p-$CH_3O$-$C_6H_4$-, $R_2$=H, n=1, A=a) (méthode G)

En opérant de la même manière que dans l'exemple **1e** mais en partant de 4,42 g (10,4 mmoles) d'ester brut précédent (**composé 17a**), on obtient après recristallisation dans le chloroforme, avec un rendement de 35 % le composé 17 de formule :

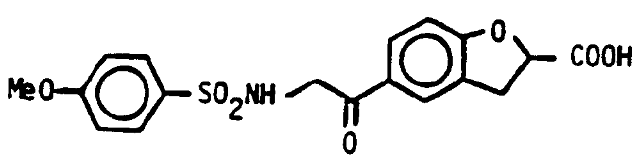

**Composé 17**

- formule brute : $C_{18}H_{17}NO_7S$
- masse moléculaire : 391,40
- cristaux blancs
- point de fusion : 162°C
- IR (KBr) : √$SO_2$ 1160-1350 ; √CO 1695 ; √COOH 1755 : √NH 3300 $cm^{-1}$
- RMN ($CDCl_3$) δ : 6.95, m, 3H, Ar ortho O ; 7.65 à 7.75, m, 4H, Ar.
- CCM : gel de silice 60 Merck F 254
  - éluant : chloroforme, méthanol, acide acétique
    - 80/18/02
  - Rf : 0,30
- Solubilité : soluble à 30 % dans le DMSO

**Exemple 18**

**Acide (pentafluorobenzènesulfonamidoacétyl)-5 dihydro-2,3 benzofuranecarboxylique-2 IX** ($R=C_6F_5$, $R_2=H$, n=1, A=a) et pour I (X=CO, $R_1=R_3=H$) (**composé 18**) (schéma de synthèse I) (méthode D)

**a**) (pentafluorobenzènesulfonamidoacétyl)-5 dihydro-2,3 benzofuranecarboxylate-2 d'éthyle **VIII** ($R=C_6F_5$, $R_2=H$, n=1, $R_5=Et$, A=a).

En partant de 3 g (10,4 mmoles) de chlorhydrate d'(aminoacétyl)-5 dihydro-2,3 benzofuranecarboxylate-2 d'éthyle (composé **1c**) et en le condensant selon l'exemple **1d** avec 4 g (15 mmoles) de chlorure de pentafluorobenzènesulfonyle, on obtient après purification sur silice et cristallisation de l'éther isopropylique 1,30 g (Rdt 26 %) le composé **18a** de formule :

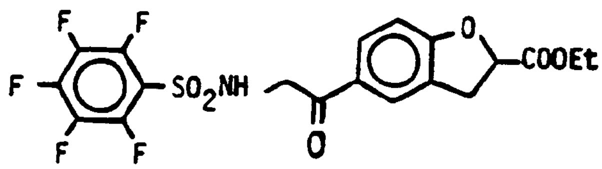

**Composé 18a**

- formule brute : $C_{19}H_{14}F_5NO_6S$
- masse moléculaire : 479,38
- cristaux jaunes
- point de fusion : 127°C

**b**) acide pentafluorobenzènesulfonamidoacétyl-5 dihydro-2,3 benzofuranecarboxylique-2 (**composé 18**)

Une solution de 1,1 g (2,3 mmoles) d'ester précédent dans un mélange méthanol-eau renfermant du bicarbonate de sodium est saponifiée selon le procédé décrit dans l'exemple **1e** pour donner, après purification sur silice et cristallisation de l'éther isopropylique, 0,55 g (Rdt 58 %) de composé **24** de formule :

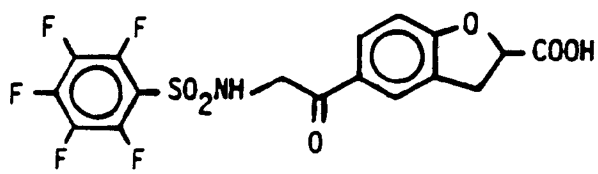

**Composé 18**

- formule brute : $C_{17}H_{10}F_5NO_6S$
- masse moléculaire : 451,32
- cristaux blanc cassé
- point de fusion : 200°C inst.
- IR (KBr) : $\sqrt{}SO_2$ 1180-1330 ; $\sqrt{}CO$ 1690 ; $\sqrt{}COOH$ 1735 ; $\sqrt{}NH$ 3240 $cm^{-1}$
- RMN ($CDCl_3$) δ : 3.54, q, 1H, $OCHCH_2Ar$ ; 3.82, q, 1H, $OCHCH_2Ar$ ; 4.93, s, 2H, $NHCH_2CO$ ; 5.54, q, 1H, $OCHCH_2Ar$ 7.04, d, 1H, Ar ortho O ; 7.73, s, 1H, NH ; 7.98, m, 2H, Ar ortho CO.
- CCM : gel de silice 60 Merck F 254
  éluant : chloroforme, méthanol, acide acétique
  80/18/02
  Rf : 0,42
- Solubilité : soluble dans le DMSO à 20 %. insoluble dans l'eau.

**Exemple 19**

**Trihydrate de parachlorobenzènesulfonamidoacétyl-5 dihydro-2,3 benzofuranecarboxylate-2 de (d1) lysine I** (R=p-Cl-$C_6H_4$-, $R_1$=$R_2$=$R_3$=H, n=1, A=a, X=CO)

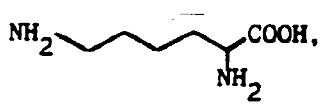

$3H_2O$) (**composé 19**)

Une solution de 6,65 g (22,7 mmoles) de lysine dl à 50 % est diluée avec 18 ml d'eau puis traitée à 20°C avec 6,65 g (16,8 mmoles) d'acide parachlorobenzènesulfonamidoacétyl-5 dihydro-2,3 benzofuranecarboxylique-2 (**composé 1**). Le mélange devient homogène puis le sel cristallise. La cristallisation est parachevée par addition de 150 ml d'alcool isopropylique. Après 1 heure d'agitation supplémentaire, le sel est filtré, essoré et séché puis recristallisé de 300 ml d'un mélange alcool-eau 75-25 pour donner avec un rendement de 70 % le composé 19 de formule :

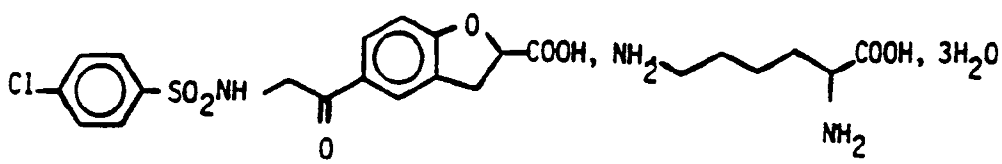

**Composé 19**

- formule brute : $C_{23}H_{34}ClN_3O_{11}S$
- masse moléculaire : 596,05
- cristaux blanc cassé
- point de fusion lent : 130°C
- IR (KBr) : √$SO_2$ 1173-1335-1360 ; √CO, $COO^-$ bande large centrée sur 1600, √NH 3300 $cm^{-1}$
- Solubilité : soluble à 1 % dans l'eau.

**Exemple 20**

**(parachlorobenzènesulfonamido-2 éthyl)-6 chromanecarboxylate-2 de (d1) lysine I** (R=p-Cl-$C_6H_4$-, $R_1$=$R_2$=$R_3$=H, n=1, A=g, X=$CH_2$),

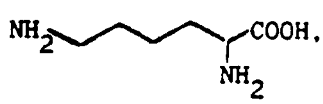

$3H_2O$ (**composé 20**)

En opérant de la même manière que pour le procédé décrit dans l'exemple 19 mais en partant de 3,45 g (8,7 mmoles) d'acide (parachlorobenzènesulfonamido-2 éthyl)-6 chromanecarboxylique-2, on obtient directement (sans recristallisation) avec un rendement de 95 % le **composé 20** de formule :

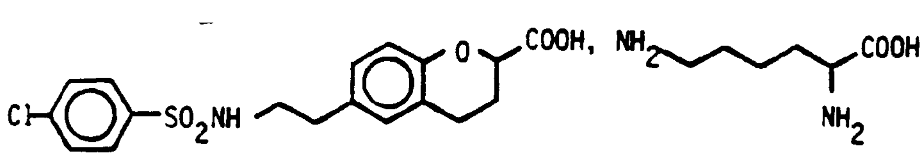

**Composé 20**

- formule brute : $C_{24}H_{32}ClN_3O_7S$

- masse moléculaire : 542,05
- cristaux blancs
- point de fusion : 214°C
- IR (KBr) : √$SO_2$ 1180-1330-1355 ; √CO, $COO^-$ bande large centrée sur 1600 $cm^{-1}$
- Solubilité : soluble à 1 % dans l'eau.

**Exemple 21**

**(parachlorobenzènesulfonamidoacétyl)-6 chromanecarboxylate-2 de d1 lysine I** (R=p-Cl-$C_6H_4$-, $R_1$=$R_2$=$R_3$=H, n=1, A=g, X=CO),

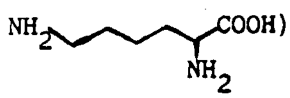

(**composé 21**)

En opérant comme décrit dans l'exemple **19** mais en partant de 4,45 g (10,8 mmoles) d'acide (parachlorobenzènesulfonamido-2 éthyl)-6 chromanecarboxylique-2, et de 4,28 g de dl lysine à 50 % dans l'eau, on obtient directement avec un rendement de 92 % le **composé 21** de formule :

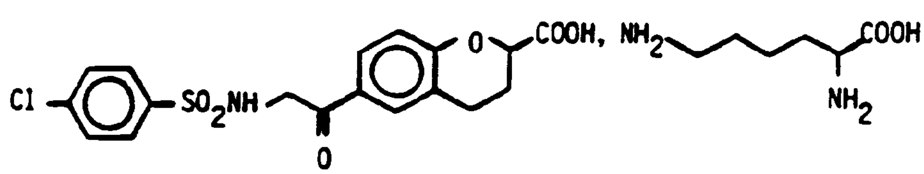

**Composé 21**

- formule brute : $C_{24}H_{30}ClN_3O_8S$
- masse moléculaire : 556,034
- cristaux blanc cassé
- point de fusion : 226°C
- IR (KBr) : √$SO_2$ 1180-1330-1355 ; √CO, $COO^-$ bande large centrée à 1610 $cm^{-1}$
- Solubilité : soluble à 1 % dans l'eau.

**Exemple 22**

**acide (parachlorobenzènesulfonamidoacétyl)-5 indanecarboxylique-2 I** (R=p-Cl-$C_6H_4$-, $R_1$=$R_2$=$R_3$=H, n=1, A=f, X=CO) (**composé 22**) (Méthode **P** modifiée).

En utilisant le mode opératoire de l'exemple **13a** mais en partant de 15 g (50,5 mmoles) de bromoacétyl-5 indane carboxylate-2 d'éthyle préparé selon **9a** et en le condensant sur 17,4 g (55 mmoles) de sel de sodium du N-tertiobutyloxycarbonyl parachlorobenzènesulfonamide préparé selon **13a**, on obtient avec un rendement quantitatif l'intermédiaire de synthèse de formule :

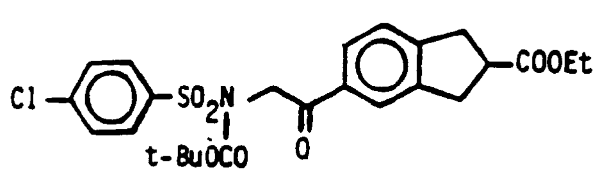

qui n'est pas isolé et est hydrolysé pendant 1 h à 60°C avec 250 ml d'une solution aqueuse d'acide chlorhydrique concentré. Ce mélange réactionnel est dilué avec 600 ml d'eau et le précipité formé est filtré, essoré, lavé à l'eau, séché et recristallisé dans l'acétate d'éthyle bouillant pour donner 6 g (Rdt = 70 %) de cristaux blancs de formule :

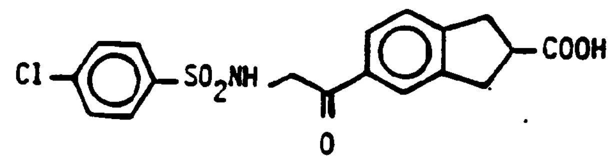

**Composé 22**

- formule brute : $C_{18}H_{16}ClNSO_6$
- masse moléculaire : 393,845
- cristaux blancs
- point de fusion : 150°C puis recristallise et refond à 181°C
- IR (KBr) : √$SO_2$ 1163-1350 ; √CO et COOH 1700 ; √NH 3290 $cm^{-1}$
- RMN (DMSO $d_6$) : de 3.1 à 3.35, m, 5H, $CH_2CHCH_2$ ; 4.48, d, 2H, $NCH_2CO$ ; 7.34, d, 1H, Ar meta du CO ; 7.64, d 2H, Ar ortho Cl ; 7.84, d, 2H, Ar ortho $SO_2$ ; 7.6 à 7.8, m, 2H, Ar ortho CO ; 8.18, t, 1H, NH ; 12.4, s, 1H, COOH.
- CCM : gel de silice Merck F 254
  éluant : chloroforme, méthanol, acide acétique :
  90.09.01
  Rf = 0,46
- Insoluble dans l'eau, soluble à 15 % dans le DMSO.

**Exemple 23**

**(parachlorobenzènesulfonamidoacétyl)-5 indanecarboxylate-2 de d1 lysine I** (R=p-Cl-$C_6H_4$-, $R_1$=$R_2$=$R_3$=H, n=1, A=f, X=CO),

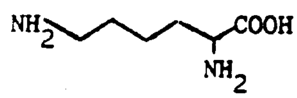

(**composé 23**)

En opérant comme décrit dans l'exemple **19** mais en partant de 5 g (12,5 mmoles) d'acide parachlorobenzènesulfonamidoacétyl)-5 indane carboxylique-2 et de 3,42 g d'une solution aqueuse de lysine à 50 %, on obtient 6 g de sel brut qui est recristallisé dans une solution éthanol/eau 60/40 pour donner avec un rendement de 66 % le **composé 23** de formule :

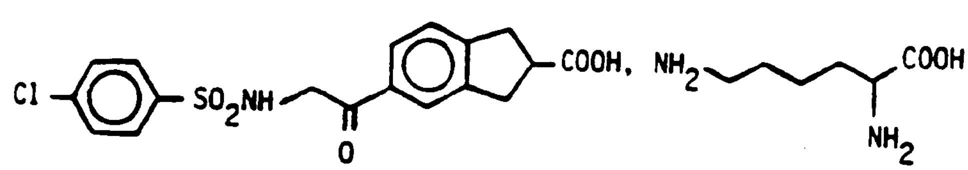

**Composé 23**

- formule brute : $C_{24}H_{30}ClN_3O_7S$
- masse moléculaire : 540,035
- cristaux blanc cassé
- point de fusion avec décomposition : 165°C
- IR (KBr) : √$SO_2$ 1160-1350 ; √CO 1690 ; √NH 3300 $cm^{-1}$
- Soluble dans l'eau à 0,5 %

**EXPERIMENTATIONS BIOLOGIQUES**

Les composés de la présente invention de formule générale I et leurs sels d'acides thérapeutiquement acceptables présentent d'intéressantes propriétés pharmacodynamiques. Ces composés sont fortement antiagrégants par inhibition selective de l'action du thromboxane $A_2$. L'activité de ces composés se situe aussi bien au niveau des plaquettes qu'au niveau du tissu endothélial. Ces composés s'opposent à l'agrégation plaquettaire induite par le composé thromboxamimétique U.46619 ainsi qu'à l'agrégation plaquettaire induite par l'acide arachidonique. Cette action se manifeste sur les tests in vitro ou ex vivo. Ces composés peuvent être utilisés à titre préventif dans l'ischémie cardiovasculaire et comme traitement adjuvant des thromboses ou dans l'infarctus du myocarde.

## 1) Toxicologie

Les composés chimiques précédemment décrits ont été soumis à des contrôles de toxicité. Cette étude a été réalisée sur la souris conventionnelle pesant de 20 à 22 g. Les composés de formule générale I ont été administrés par voie intrapéritonéale. A 500 mg par kilo, les **composés 1, 2** et **3** donnés à titre d'exemple non limitatif se révèlent atoxiques et on ne dénombre aucun mort à cette dose.

## 3) Etude pharmacologique

Les expérimentations pharmacologiques auxquelles ont été soumises les molécules chimiques objet de la présente invention, ont permis de mettre en évidence une intéressante activité sur le système cardiovasculaire à la fois sur des tests in vivo ou in vitro. Les composés de formule générale I se sont révélés capables de réduire les effets agrégants et vasoconstricteurs du composé U.46619 à des doses remarquablement faibles.

### a) Action in vitro :

Du sang est prélevé sur des lapins mâles blancs néozélandais n'ayant reçu aucun traitement susceptible de perturber les fonctions plaquettaires. Après avoir laissé s'écouler librement les premières gouttes, le sang est recueilli dans des tubes plastiques contenant du citrate trisodique 5,5 $H_2O$ à 3,8 % (p/v), le rapport citrate/sang étant de 1 volume de citrate pour 9 volumes de sang.

L'étude de l'agrégation des plaquettes sanguines est réalisé à partir des plasmas riches en plaquettes obtenus par centrifugation à basse vitesse des sangs précédents. Elle est mesurée à l'aide d'un agrégomètre de Mustard à la longueur d'onde de 609 nM avec une vitesse d'agitation de 1100 tours/mn.

Les produits à étudier sont mis en solution dans du polyéthylène glycol 300 et préincubés durant 1 mn à 37°C sous un volume de 10 microlitres, dans un mélange de 300 microlitres de plasma riche en plaquettes et 90 microlitres de solution de tyrode sans calcium pH = 7,4. (Des témoins correspondants sont réalisés en préincubant le véhicule seul à la place des solutions de produits à étudier).

L'agrégation des plaquettes sanguines est déclenchée par l'addition soit de U.46619 (concentrations finales : 1,05 à 2,44 micromoles) soit d'acide arachidonique (concentrations finales : 50 à 100 micromoles).

A partir du paramètre, pourcentage d'agrégation maximum, l'activité antiagrégante d'un produit est calculée par la formule :

$$\left[ \frac{\text{Agrégation Max (témoin)} - \text{Agrégation Max (produit)}}{\text{Agrégation Max (témoin)}} \right] \times 100$$

Une courbe : activité antiagrégante = f (concentration finale) est tracée pour chaque produit et la concentration inhibitrice 50% déterminée graphiquement.

Dans le cas de l'agrégation au U.46619, les $CI_{50}$ sont données dans le tableau I pour les **composés 1, 3, 7** à titre d'exemple non limitatif :

**Tableau I**

| composés | $CI_{50}$ |
|---|---|
| 1 | $6 \times 10^{-7}$ M |
| 3 | $5 \times 10^{-6}$ M |
| 7 | $5 \times 10^{-7}$ M |

Le pourcentage de l'inhibition de l'agrégation à l'acide arachidonique est donné dans le tableau II pour le **composé 3** à titre d'exemples non limitatifs :

**Tableau II**

| composé | concentration finale en composé étudié | % d'inhibition de l'agrégation plaquettaire |
|---|---|---|
| | $10^{-4}$ M | - 86% |
| 3 | $5.10^{-5}$ M | - 78% |
| | $10^{-5}$ M | - 7% |

**b) Action ex vivo :** inhibition des effets antiagrégants au U.46619 chez le lapin.

Du fait de leur puissante activité in vitro, les effets antiagrégants ex vivo ont été recherchés chez le lapin. Les substances de formule générale I objet de la présente invention sont administrées par voie orale en suspension dans la carboxyméthylcellulose à 1 % à des lapins mâles, naïfs, néo-zélandais, d'un poids de 2,5 à 3 kg à jeun de nourriture depuis 18 h environ. Aux temps 0 et après 90 mn, les prélèvements de sang sont effectués par ponction cardiaque. L'agent agrégant U.46619 a été utilisé dans les mêmes conditions que précédemment et la mesure de l'agrégation a été pratiquée selon la même technique.

Les résultats sont exprimés dans le tableau III en termes de pourcentage d'inhibition de l'amplitude maximale de la courbe d'agrégation à t = +90 mn par rapport à la courbe témoin à t = 0 et sont donnés pour le **composé 3** à titre d'exemple non limitatif :

**Tableau III**

| composés I | dose mg/kg | % d'inhibition de l'agrégation |
|---|---|---|
| 3 | 5 | - 43% |
| | 10 | - 74% |

## 3) Applications thérapeutiques

Compte tenu de leur activité pharmacologique, les dérivés de la présente invention peuvent être utilisés en thérapeutique humaine et animale dans le traitement des troubles cardiovasculaires. Les composés inhibent l'agrégation plaquettaire et les effets vasoconstricteurs dûs à une activation du thromboxane $A_2$ au niveau des plaquettes et du tissu vasculaire, en agissant par antagonisme de ses récepteurs sur ces cellules. Les composés de la présente invention sont actifs sur l'agrégation induite au collagène, à l'acide arachidonique et à un degré moindre à celle induite au PAF et à l'ADP. Ces propriétés permettent donc de réduire l'ischémie myocardique et, en particulier, les resténoses après angioplastie ou après injection de steptokinase. Par suite de leur bonne biodisponibilité par voie orale, ils peuvent être utilisés pour traiter les ischémies cérébrales ou périphériques (artérite) ainsi que l'athérosclérose. Ces composés ont aussi une indication secondaire dans le traitement de l'asthme, dans le choc bactériémique, les glomérulonéphrites, dans les maladies à virus et permettent aussi de lutter contre la dissémination des cellules cancéreuses.

Les composés de la présente invention sont utilisés pour préparer des médicaments pouvant être administrés chez les animaux à sang chaud ou chez l'homme. L'administration peut être réalisée par voie orale, parentérale ou rectale, chaque dose est constituée d'un adjuvant pharmaceutique inerte facilitant la préparation, l'absorption du médicament et du principe actif pouvant être aussi associé à un autre. Ces médicaments peuvent se présenter sous forme de comprimés, gélules, suspensions, émulsions, sirops, suppositoires, solutions ou analogues. L'administration du principe actif se fait à une dose moyenne comprise entre 0,5 et 25 mg/kg du poids du corps.

Trois préparations sont données à titre d'exemple non limitatif. Les ingrédients ainsi que d'autres pouvant être introduits en d'autres proportions sans modifier la portée de l'invention.

## Exemple 24 : solution injectable

1 flacon à injection en verre inactinique renfermant du (parachlorobenzènesulfonamidoacétyl)-5 dihydro-2,3 benzofuranecarboxylate-2 de lysine pulvérulent......... 40 mg
1 ampoule scellée renfermant une solution stérile apyrogène d'eau distillée + NaCl q.s.p. une solution isotonique finale de................................................ 4 ml
Préparation extemporanée de la solution avant l'injection.

**Exemple 25 : comprimés**

| | |
|---|---|
| Acide (parachlorobenzènesulfonamidoacétyl)-5 dihydro-2,3 benzofuranecarboxylique-2 | 100 mg |
| Lactose | 60 mg |
| Amidon de maïs | 20 mg |
| Polyvinylpyrrolidone | 18 mg |
| Stéarate de magnésium | 2 mg |
| comprimé de | 200 mg |

**Exemple 26 : suppositoires**

| | |
|---|---|
| Acide (parachlorobenzènesulfonamidoacétyl)-5 dihydro-2,3 benzofuranecarboxylique-2 | 100 mg |
| Base pour suppositoire (beurre de cacao)...q.s.p. | 2,5 g |

à conserver à l'abri de la lumière, de la chaleur et de l'humidité.

**Revendications**

**Revendications pour les Etats contractants suivants : AT, BE, CH, LI, DE, DK, FR, GB, IT, LU, NL, SE**

**1**) Nouveaux sulfonamides dérivés d'acides carbocycliques ou benzohétérocycliques de formule générale I

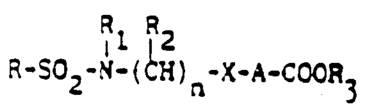

I

dans laquelle les radicaux et substituants sont définis comme suit :

**R** - représente un alcoyle léger linéaire ou ramifié renfermant de 1 à 9 atomes de carbone, des radicaux phényle ou naphtyle substitués ou non par un ou plusieurs groupements : alcoyle inférieur (1 à 4 C), halogéno, alcoyloxy inférieur (1 à 4 C), nitro, amino, dialkylamino léger (1 à 4 C); trifluorométhyle,

**$R_1$** - représente un hydrogène ou un alcoyle inférieur (1 à 4 C) linéaire ou ramifié ou un benzyle ;

**$R_2$** - représente un hydrogène ou un alcoyle léger (1 à 6 C) linéaire ou ramifié, un groupement phényle substitué ou non par un chlore ou un méthoxyle, un groupement arylalcoyle renfermant de 7 à 9 atomes de carbone ;

**$R_3$** - représente un hydrogène ou un alcoyle inférieur ramifié ou non (1 à 6 C) ;

**-X-** représente un radical fonctionnel divalent choisi parmi les suivants:

$-CH_2-$ ; $-CH-OR_4$ ; -C=O ; $-C=N-OR_4$ ;

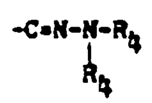

avec $R_4$=H, Me **-X-** est différent de $-CH_2-$ quand -A- représente les radicaux divalents a, b, d, et e ci-après ;

**-A-** représente un radical bivalent benzocyclique ou benzohétérocyclique choisi parmi les suivants : (a-j)

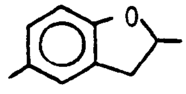 ; 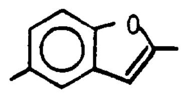 ; 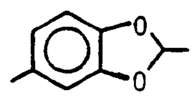 ; 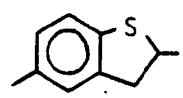 ;

(a) (b) (c) (d)

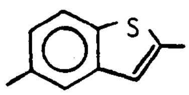 ; 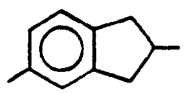 ; 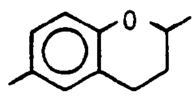 ; 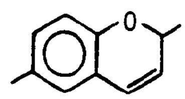 ;

(e) (f) (g) (h)

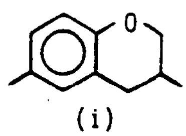 ; 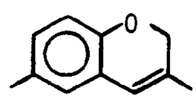

(i) (j)

**n** - peut prendre les valeurs de 1 à 4 inclus.

Les formes hydratées des acides de formule générale I ainsi que les sels organiques ou minéraux de I ($R_3$=H) thérapeutiquement acceptables et en particulier un sel choisi parmi : sodium, calcium, zinc, hydroxy-2 éthylammoniumm, bis(hydroxy-2 éthyl)ammonium, tris(hydroxy-2 éthyl) ammonium, morpholinium, et leur forme hydratée, les composés de formule générale I peuvent se présenter sous forme de mélange racémique ou être isolés sous forme d'énantiomères, de diastéréoisomères ou leur mélange en toute proportion.

**2**) Composé selon la revendication 1 caractérisé en ce qu'il est choisi parmi les composés suivants :

- acide (parachlorobenzènesulfonamidoacétyl)-5 dihydro-2,3 benzofuranecarboxylique-2
- acide (parachlorobenzènesulfonamido-2 hydroxy-1 éthyl)-5 dihydro-2,3 benzofuranecarboxylique-2
- acide (parachlorobenzènesulfonamido-2 hydroxyimino-1 éthyl)-5 dihydro-2,3 benzofuranecarboxylique-2
- acide (parachlorobenzènesulfonamido-2 méthoxyimino-1 éthyl)-5 dihydro-2,3 benzofuranecarboxylique-2
- acide (N-méthyl parachlorobenzènesulfonamidoacétyl)-5 dihydro-2,3 benzofuranecarboxylique-2
- acide ($\alpha$-méthyl parachlorobenzènesulfonamidoacétyl)-5 dihydro-2,3 benzofuranecarboxylique-2
- acide (parachlorobenzènesulfonamido-2 hydroxy-1 propyl)-5 dihydro-2,3 benzofuranecarboxylique-2
- (parachlorobenzènesulfonamidoacétyl dihydro-2,3 benzofuranecarboxylate-2 d'éthyle
- acide (parachlorobenzènesulfonamido-2 éthyl)-5 benzodioxole-1,3 carboxylique-2
- acide (parachlorobenzènesulfonamido-2 éthyl)-5 indanecarboxylique-2
- acide (parachlorobenzènesulfonamido-2 éthyl)-6 chromanecarboxylique-2
- acide (parachlorobenzènesulfonamidoacétyl)-6 chromanecarboxylique-2
- (parachlorobenzènesulfonamidoacétyl)-6 chromanecarboxylate-2 d'éthyle
- acide (benzènesulfonamidoacétyl)-5 dihydro-2,3 benzofuranecarboxylique-2
- acide (paraméthoxybenzènesulfonamidoacétyl)-5 dihydro-2,3 benzofuranecarboxylique-2
- acide (pentafluorobenzènesulfonamidoacétyl)-5 dihydro-2,3 benzofuranecarboxylique-2
- acide (parachlorobenzènesulfonamidoacétyl)-5 indanecarboxylique-2
- (parachlorobenzènesulfonamidoacétyl)-5 dihydro-2,3 benzofuranecarboxylate-2 de lysine
- (parachlorobenzènesulfonamido-2 éthyl)-6 chromanecarboxylate-2 de lysine
- (parachlorobenzènesulfonamidoacétyl)-6 chromanecarboxylate-2 de lysine
- (parachlorobenzènesulfonamidoacétyl)-5 indanecarboxylate-2 de lysine.

**3**) Procédé de préparation des composés chimiques selon les revendications **1** et **2** avec A différent de b,e,h,j, caractérisé en ce que l'on fait réagir un ester benzocyclique ou benzohétérocyclique de formule **II**

$$H\text{-}A\text{-}COOR_5 \qquad \mathbf{II}$$

avec un halogénure d'acyle fonctionnel de structure **III**

$$Y-(\overset{R_2}{\overset{|}{C}}H)_n-COY_1 \qquad \underline{III}$$

selon les conditions de Friedel-Crafts en utilisant un solvant compatible, de préférence chloré et plus précisément le chlorure de méthylène ou le dichloro-1,2 éthane en présence d'un acide de Lewis comme le chlorure d'aluminium à titre d'exemple non limitatif et à une température variant de -5°C à +50°C de façon à obtenir le **composé IVa** de formule :

$$Y-(\overset{R_2}{\overset{|}{C}}H)_n-CO-A-COOR_5 \qquad \underline{IVa}$$

qui est lui-même condensé avec l'azoture de sodium dans un mélange hydroalcoolique à une température comprise entre +10°C et +50°C pour donner le composé de formule :

$$N_3-(\overset{R_2}{\overset{|}{C}}H)_n-CO-A-COOR_5 \qquad \underline{V}$$

qui est réduit sélectivement par l'hydrogène dans un alcool léger et renfermant une solution aqueuse d'acide fort -chlorhydrique par exemple- en présence de catalyseur d'hydrogénation déposé sur un support inerte et plus précisément le palladium sur charbon par exemple pour donner à une température comprise entre +10°C et +50°C l'aminocétoester chlorhydraté de formule **VI** :

$$NH_2-(\overset{R_2}{\overset{|}{C}}H)_n-CO-A-COOR_5,\ HCl \qquad \underline{VI}$$

lui-même condensé avec un halogénure de sulfonyle (fluorure, chlorure, bromure) convenablement substitué de formule :

$R-SO_2Z$ **VII**

dans un solvant basique organique comme la pyridine par exemple à une température comprise entre -15°C et +40°C pour donner le **composé I** avec X=CO et $R_1$=H, $R_3$ différent de H, de formule **VIII** :

$$R-SO_2NH-(\overset{R_2}{\overset{|}{C}}H)_n-\overset{O}{\overset{\|}{C}}-A-COOR_5 \qquad \underline{VIII}$$

enfin, cet ester est saponifié par une solution aqueuse alcaline comme la soude ou la potasse en présence d'un solvant organique miscible comme un alcool ou un éther et plus précisément le méthanol, l'éthanol, le dioxanne ou le tétrahydrofuranne à une température comprise entre +10°C et celle d'ébullition du mélange pour donner après acidification le **composé I** acide avec X=CO, $R_1$=H, $R_3$=H de formule **IX**

$$R-SO_2NH-(\overset{R_2}{\overset{|}{C}}H)_n-\overset{O}{\overset{\|}{C}}-A-COOH \qquad \underline{IX}$$

les radicaux des formules générales de **IVa** à **IX** ont la même signification que dans la revendication **1** et $R_5$=$R_3$ sauf H ; Y et $Y_1$ représentent un chlore ou un brome et Z un fluor ou un chlore ou un brome.

**4**) Procédé de préparation des composés chimiques selon les revendications **1** et **2** et caractérisé en ce que l'on réduit un composé de formule **V** préparé selon la revendication **3**

$$N_3-(\overset{R_2}{CH})_n-CO-A-COOR_5 \qquad \underline{V}$$

sous atmosphère d'hydrogène dans un alcool léger et renfermant une solution aqueuse d'acide fort en présence de catalyseur d'hydrogénation déposé sur un support inerte et plus précisément le palladium sur charbon par exemple pour donner à une température comprise entre +10°C et +60°C et pendant plusieurs heures - en contrôlant l'avancement de la réaction par chromatographie - le composé de formule **XII**

$$NH_2-(\overset{R_2}{CH})_n-\overset{OH}{CH}-A-COOR_5 \qquad \underline{XII}$$

qui est condensé avec un halogénure de sulfonyle (fluorure, chlorure, bromure) convenablement substitué de formule :

$$R-SO_2Z$$

dans un solvant basique organique comme la pyridine par exemple à une température comprise entre -15°C et +40°C pour donner le **composé I** avec X=CHOH et $R_1$=H et $R_3$ différent de H de formule **XIII**

$$R-SO_2NH-(\overset{R_2}{CH})_n-\overset{OH}{CH}-A-COOR_5 \qquad \underline{XIII}$$

enfin cet ester peut être saponifié par une solution aqueuse alcaline comme la soude ou la potasse en présence d'un solvant organique miscible alcoolique ou éthéré et plus précisément le méthanol, l'éthanol, le dioxanne ou le tétrahydrofuranne à une température comprise entre +10°C et +60°C pour donner après acidification le **composé I** acide avec X=CHOH, $R_1$=H, $R_3$=H de formule **XIV**

$$R-SO_2NH-(\overset{R_2}{CH})_n-\overset{OH}{CH}-A-COOH \qquad \underline{XIV}$$

les radicaux des formules générales **V**, **XII** à **XIV** ont la même signification que dans les revendications **1** et **3**.

**5**) Procédé de préparation des composés chimiques selon les revendications 1 et 2 et caractérisé en ce que l'on réduit un composé de formule **VI** préparé selon la revendication **3** soit sous surpression d'hydrogène

$$HCl-NH_2-(\overset{R_2}{CH})_n-\overset{O}{\overset{\|}{C}}-A-COOR_5 \qquad \underline{VI}$$

en présence d'un acide fort comme l'acide sulfurique ou perchlorique en utilisant un catalyseur d'hydrogénation comme le palladium sur charbon dans un acide carboxylique comme solvant et plus particulièrement l'acide acétique en opérant à une température comprise entre 20 et 90°C sous une pression d'hydrogène comprise entre 0,1 et 5 atmosphères ; soit en réduisant le **composé VI** par un trialkyl silane et plus précisément le triéthyl silane en présence d'acide trifluoroacétique à une température comprise entre +10 et +40°C, soit en réduisant le **composé VI** par l'hydrogène naissant produit par l'action d'un acide (chlorhydrique ou acétique) sur le zinc amalgamé ou non en chauffant entre 40 et 120°C dans un solvant organique comme le toluène par exemple pour donner le **composé XV** sous forme de base ou de sel de formule :

$$NH_2-(\overset{R_2}{CH})_n-CH_2-A-COOR_5 \qquad \underline{XV}$$

qui est ensuite condensé avec un halogénure de sulfonyle défini et dans les mêmes conditions que celles décrites dans les revendications 3 et 4 pour donner le **composé I** avec $R_1$=H et X=$CH_2$ de formule **XVI** homologue saturé de **VIII** et **XIII**

$$RSO_2NH-(\overset{\overset{R_2}{|}}{C}H)_n-CH_2-A-COOR_5 \qquad \underline{XVI}$$

qui est saponifié selon le procédé décrit dans les revendications 3 et 4 pour préparer les dérivés **IX** et **XIV**, pour donner le **composé I** avec $R_1$=$R_3$=H et X=$CH_2$ de formule **XVII**

$$RSO_2NH-(\overset{\overset{R_2}{|}}{C}H)_n-CH_2-A-COOH \qquad \underline{XVII}$$

où les radicaux des formules générales **VI**, **XV** à **XVII** ont la même signification que dans les revendications **1** et **3**.

**6**) Procédé de préparation des composés chimiques définis selon les revendications **1** et **2** caractérisé en ce que l'on réduit un composé de formule **IVa** préparé selon la revendication **3**, selon une des méthodes

$$Y-(\overset{\overset{R_2}{|}}{C}H)_n-\overset{\overset{O}{\|}}{C}-A-COOR_5 \qquad \underline{IVa}$$

de la revendication **5** pour donner le composé halogéné équivalent de **XV** ayant pour formule **XIX**

$$Y-(\overset{\overset{R_2}{|}}{C}H)_n-CH_2-A-COOR_5 \qquad \underline{XIX}$$

qui est condensé avec le sel de sodium préparé in situ d'un sulfonamide secondaire convenablement substitué de formule **XX**

$$R-SO_2NH-R_7 \qquad \underline{XX}$$

dans un solvant organique et plus particulièrement le diméthylformamide, le diméthylacétamide, l'acétone, la butanone, le tétrahydrofuranne ou le dioxanne à une température comprise entre +5°C et +60°C pour donner le composé de formule **XXI**

$$R-SO_2-\overset{\overset{R_7}{|}}{N}-(\overset{\overset{R_2}{|}}{C}H)_n-CH_2-A-COOR_5 \qquad \underline{XXI}$$

ce dérivé est ensuite saponifié selon le procédé décrit dans les revendications **3** et **4** relatif aux dérivés **IX** et **XIV** pour donner le composé équivalent saturé **I** avec X=$CH_2$ et $R_3$=H de formule **XXII**

$$R-SO_2-\overset{\overset{R_1}{|}}{N}-(\overset{\overset{R_2}{|}}{C}H)_n-CH_2-A-COOH \qquad \underline{XXII}$$

dans laquelle les radicaux des composés de formule **IVa**, **XIX** à **XXII** ont la même signification que dans les revendications **1** à **3** et où $R_7$ représente soit le radical $R_1$ différent de l'hydrogène soit le radical $R_6$COO- où $R_6$ représente un benzyle ou un alcoyle léger ramifié ou non renfermant de 1 à 6 carbones inclus et à titre d'exemple non limitatif les radicaux : méthyle, éthyle, tertiobutyle.

**7**) Procédé de préparation des composés chimiques définis selon les revendications **1** et **2** caractérisé en ce que l'on condense un composé de formule **IVa** préparé selon la revendication **3** avec le sel de sodium du **composé XX**

$R-SO_2NHR_7$ XX

préparé in situ selon le même procédé que pour préparer le **composé XXI** selon la revendication **6** pour donner le céto sulfamidoester I avec X=CO de formule **XXIV**

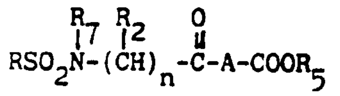

XXIV

qui est saponifié soit directement dans le cas où $R_7$ est différent de $COOR_6$ d'après le procédé décrit pour les **composés VIII et XIII** selon les revendications **3** et **4** pour donner ici le **composé I** avec X=CO, $R_3$=H et $R_1$ différent de H, de formule **XXV** ; soit dans le cas où $R_7$=COO-tBu est soumis en premier lieu à un clivage du groupement $R_7$ en milieu acide fort et plus précisément l'acide chlorhydrique dissous dans un solvant organique comme l'acétate d'éthyle par exemple pour donner le **composé XXV** avec $R_1$=H et $R_5$=$R_3$ différent de H qui peut être ensuite saponifié comme ci-dessus en acide pour donner le **composé I** avec $R_1$=$R_3$=H, X=CO de formule **XXV**

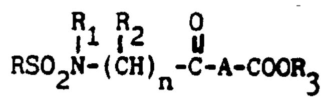

XXV

où les radicaux des formules des composés **IVa**, **XX**, **XXIV**, **XXV** ont la même signification que dans les revendications **1**, **3** et **6**.

**8**) Procédé de préparation des composés chimiques définis selon les revendications **1** et **2** et caractérisé en ce que l'on réduit un dérivé de structure générale **XXIV** préparé selon la revendication **7**

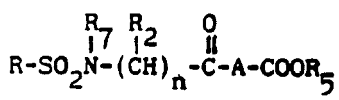

XXIV

selon une des trois méthodes décrites dans la revendication **5** pour préparer le **composé XV** pour donner ici l'homologue sulfonamido de formule **XXI** déjà décrit dans la revendication **6**

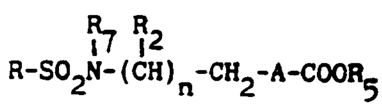

XXI

et saponifié en acide correspondant I avec X=$CH_2$ et $R_3$=H de formule **XXII** selon le procédé déjà décrit dans la revendication **6** et où les radicaux et variables des formules **XXIV** et **XXI** ont la même signification que dans les revendications **1**, **3** et **6**.

**9**) Procédé de préparation des composés chimiques définis selon les revendications **1** et **2** et caractérisé en ce que l'on réduit un composé de formule générale I avec X=CO pour donner, selon un des procédés décrits dans la revendication **5**, le sulfonamide réduit homologue de formule I avec X=$CH_2$.

**10**) Procédé de préparation des composés chimiques définis selon les revendications **1** et **2** et caractérisé en ce que l'on réduit un composé de formule générale I où X=CO en présence de borohydrure de sodium ou de potassium dans un solvant protique comme un alcool léger de préférence de même condensation en carbone que $R_3$ lorsque $R_3$ est différent de H (de façon à éviter une transestérification) à une température comprise entre +5° C et +50°C pour donner les composés de formule générale I avec X=CHOH.

**11**) Procédé de préparation des composés chimiques selon les revendications **1** et **2** et caractérisé en ce que l'on fait réagir un composé de formule I avec $R_3$=H avec un alcool de formule générale $R_3OH$ en présence de catalyseur acide comme l'acide sulfurique ou paratoluènesulfonique en chauffant à une température comprise entre 40°C et 110°C pour donner les composés de formule générale I avec $R_3$ différent de H.

**12**) Procédé de préparation des composés chimiques selon les revendications **1** et **2** et caractérisé en ce que l'on condense un composé de formule générale I où X=CO et $R_3$=H avec un chlorhydrate d'hydroxylamine substitué ou non de formule $R_4ONH_2$ pour donner dans la pyridine à une température comprise entre 10°C et 50°C les oximes correspondantes I avec X=C=N-$OR_4$ et $R_3$=H avec $R_4$ défini comme dans la revendication **1**.

**13**) Procédé de préparation des composés chimiques selon les revendications **1** et **2** et caractérisé en ce que

que l'on condense un dérivé de formule générale I où X=CO et $R_3$=H avec une hydrazine de formule générale

$$\begin{array}{l} NH_2-N-R_4 \\ \qquad\;| \\ \qquad R_4 \end{array}$$

pour donner dans un solvant organique protique comme l'éthanol en présence d'un acide organique comme l'acide acétique par exemple, à une température comprise entre 25°C et 120°C, les hydrazones correspondantes I avec

$$\begin{array}{l} X= \quad C=N-N-R_4 \\ \qquad\qquad\qquad\;| \\ \qquad\qquad\qquad R_4 \end{array}$$

et $R_3$=H avec $R_4$ défini comme dans la revendication **1**.

**14**) Procédé de préparation des composés chimiques selon les revendications **1** et **2** et caractérisé en ce que l'on fait réagir un ester de formule générale I où le radical bicyclique -A- est saturé sur le cycle rattaché à la fonction carboxylate (c'est-à-dire où A = a,d,g,i) avec un halogénoimide et plus particulièrement le N-bromo ou N-chlorosuccinimide dans un solvant halogéné et plus précisément le tétrachlorure de carbone à une température comprise entre +20°C et +80°C pour donner les composés insaturés correspondants de formule générale I (c'est-à-dire où A = b,e,h) avec $R_3$ différent de H qui sont ensuites saponifiés en leurs acides précurseurs de formule générale I avec $R_3$=H selon le protocole décrit dans la revendication **3**.

**15**) Procédé de préparation des composés chimiques selon les revendications **1** et **2** et caractérisé en ce que l'on fait réagir un composé de formule générale I dans laquelle $R_1$=H avec un dérivé activé de formule $(R_1)_2SO_4$ ou $R_1Z$ avec Z=Br ou I en présence d'une base forte comme l' hydrure de sodium ou un carbonate ou un hydroxyde de sodium ou de potassium dans un solvant comme le DMF, le diméthyl acétamide, la butanone, l'acétone et à une température comprise entre +20°C et +70°C pour donner le composé équivalent de formule générale I dans laquelle $R_1$ et $R_3$ sont différents de H et où les radicaux variables sont définis comme dans la revendication **1**.

**16**) Procédé de préparation des composés chimiques selon les revendications **1** et **2** avec A différent de b,e,h et j caractérisé en ce que l'on fait réagir un ester benzocarbocyclique ou benzohétérocyclique de formule générale II

$$H-A-COOR_5 \qquad \text{II}$$

avec un halogénure d'acyle fonctionnel de structure **IIIa**

$$\begin{array}{l} \quad\;\; O \;\; R_1 \;\; R_2 \\ \quad\;\; \| \;\;\; | \;\;\;\;\; | \\ R_8OC-N-(CH)_n-COY \end{array} \qquad \textbf{IIIa}$$

selon les conditions de Friedel et Crafts en utilisant un solvant compatible de préférence chloré et plus précisément le chlorure de méthylène ou le dichloro-1,2 éthane en présence d'un acide de Lewis comme le chlorure d'aluminium à titre d'exemple non limitatif et à une température comprise entre -5°C et +50°C de façon à obtenir le **composé IVb** de formule :

$$\begin{array}{l} \qquad\quad\;\; R_1 \;\; R_2 \\ R_8OCON-(CH)_n-CO-A-COOR_5 \end{array} \qquad \textbf{IVb}$$

qui est lui-même réduit par un borohydrure alcalin selon le procédé décrit dans la revendication **10** de façon à obtenir le dérivé XXIX de formule :

$$\begin{array}{l} \qquad\quad\; R_1 \;\; R_2 \\ \qquad\quad\; | \;\;\;\;\; | \\ R_8OCO-N-(CH)_n-CHOH-A-COOR_5 \end{array} \qquad \textbf{XXIX}$$

pour conduire par hydrolyse basique au sel de sodium de l'aminohydroxy acide de formule <u>XXX</u> selon la méthode décrite dans la revendication **3** pour la préparation du **composé** **<u>IX</u>**

$$R_1NH-(\overset{R_2}{\overset{|}{C}}H)_n-CHOH-A-COOH \qquad \underline{XXX}$$

et enfin en condensant un halogénure de sulfonyle convenablement substitué de formule $RSO_2Z$ sur le sel de sodium brut de <u>XXX</u> précédemment obtenu dans la même phase aqueuse organique à un pH compris entre 11 et 13 et à une température comprise entre 0 et +40°C pour donner le **composé** **<u>I</u>** avec $R_3$=H et Y=CHOH de formule <u>XXVII</u>

$$ArSO_2\overset{R_1}{\overset{|}{N}}-(\overset{R_2}{\overset{|}{C}}H)-CHOH-A-COOH \qquad \underline{XXVII}$$

dans laquelle les radicaux des formules générales **<u>IVb</u>**, **<u>XXIX</u>**, **<u>XXX</u>**, **<u>XXVII</u>** ont la même signification que dans **1** et **3** et $R_8$ représente un radical alcoyle linéaire léger de 1 à 6 carbones inclus.

**17**) Procédé de préparation des composés chimiques selon les revendications **1** et **2** et caractérisé en ce que l'on réduit selon un des procédés décrits dans la revendication **5** la cétone d'un composé de formule générale **<u>IVb</u>** préparé comme décrit dans la revendication **16**

$$R_8OCO\overset{R_1}{\overset{|}{N}}-(\overset{R_2}{\overset{|}{C}}H)_n-CO-A-COOR_5 \qquad \underline{IVb}$$

en groupement méthylène pour donner le dérivé **<u>XXXI</u>** de formule:

$$R_8OCO\overset{R_1}{\overset{|}{N}}-(\overset{R_2}{\overset{|}{C}}H)_n-CH_2-A-COOR_5 \qquad \underline{XXXI}$$

qui est ensuite saponifié selon le procédé décrit dans la revendication **16** pour conduire au sel de sodium de l'amino acide de formule **<u>XXXII</u>**

$$R_1NH-(\overset{R_2}{\overset{|}{C}}H)_n-CH_2-A-COOH \qquad \underline{XXXII}$$

qui est condensé sans être isolé avec l'halogénure de sulfonyle de formule $RSO_2Z$ selon la méthode décrite dans la revendication **16** pour donner après acidification le **composé** **<u>I</u>** avec X=$CH_2$ et $R_3$=H de formule **<u>XXIII</u>**

$$RSO_2\overset{R_1}{\overset{|}{N}}-(\overset{R_2}{\overset{|}{C}}H)_n-CH_2-A-COOH \qquad \underline{XXIII}$$

dans laquelle les radicaux des formules **<u>IVb</u>**, **<u>XXXI</u>**, **<u>XXXII</u>**, **<u>XXIII</u>** ont la même signification que dans les revendications **1**, **3** et **16**.

**18**) Procédé de préparation des sels des composés chimiques selon les revendications **1** et **2** caractérisé en ce que l'on fait réagir l'acide de formule générale **<u>I</u>** avec $R_3$=H avec une quantité stoëchiométrique de base organique ou minérale dans un solvant protique comme l'eau, le méthanol, l'éthanol ou cétonique comme la butanone ou l'acétone à une température comprise entre 0°C et +60°C.

**19**) Procédé de préparation des composés chimiques selon les revendications **1**, **2** et **7** caractérisé en ce que l'on hydrolyse un composé de formule générale **<u>I</u>** avec $R_1$ = COO-tBu, $R_3$ différent de H, en versant le mélange réactionnel brut ainsi obtenu dans une solution aqueuse concentrée d'acide fort et plus précisément l'acide chlorhydrique, en chauffant à une température variant de 40 à 100°C et plus précisément comprise entre 50 et 70°C pour donner le **composé I** où $R_1 = R_3 = H$ et où les autres radicaux ont la même signification que dans 1.

**20**) A titre de médicaments nouveaux, utiles en particulier dans le traitement des troubles du système cardiovasculaire et plus particulièrement comme antiagrégant ; anti-ischémique au niveau du myocarde, cérébral ou périphérique ; antiasthmatique, antiathéromateux et enfin, comme antimigraineux, anticancéreux et antiviral, les composés définis selon l'une des revendications **1** et **2**.

**21**) Compositions pharmaceutiques caractérisées en ce qu'elles contiennent comme principe actif au moins un composé défini selon l'une des revendications **1** et **2** associées à un support pharmaceutique inerte.

**Revendications pour les Etats contractants suivants : ES, GR**

1) Procédé de préparation de sulfonamides dérivés d'acides carbocycliques ou benzohétérocycliques de formule générale I

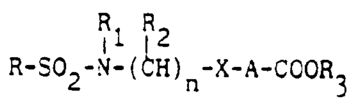

I

dans laquelle les radicaux et substituants sont définis comme suit :

**R** - représente un alcoyle léger linéaire ou ramifié renfermant de 1 à 9 atomes de carbone, des radicaux phényle ou naphtyle substitués ou non par un ou plusieurs groupements : alcoyle inférieur (1 à 4 C), halogéno, alcoyloxy inférieur (1 à 4 C), nitro, amino, dialkylamino léger (1 à 4 C), trifluorométhyle;

**$R_1$** - représente un hydrogène ou un alcoyle inférieur (1 à 4 C) linéaire ou ramifié ou un benzyle ;

**$R_2$** - représente un hydrogène ou un alcoyle léger (1 à 6 C) linéaire ou ramifié, un groupement phényle substitué ou non par un chlore ou un méthoxyle et un groupement arylalcoyle renfermant de 7 à 9 atomes de carbone ;

**$R_3$** - représente un hydrogène ou un alcoyle inférieur ramifié ou non (1 à 6 C) ;

**-X-** représente un radical fonctionnel divalent choisi parmi les suivants:

$-CH_2-$ ; $-CH-OR_4-$ ; $-C{=}O$ ; $-C{=}N-OR_4$ ;

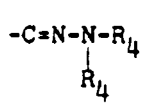

avec $R_4$=H,Me **-X-** est différent de $-CH_2-$ quand -A- représente les radicaux divalents a, b, d, et e ci-après ;

**-A-** représente un radical bivalent benzocyclique ou benzohétérocyclique choisi parmi les suivants de (a) à (j) :

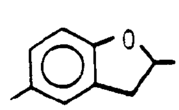 ; 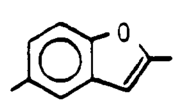 ; 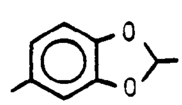 ; 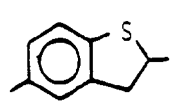 ;

(a) (b) (c) (d)

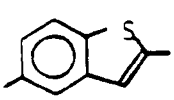 ; 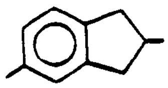 ; 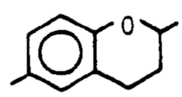 ; 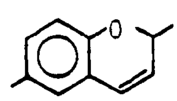 ;

(e) (f) (g) (h)

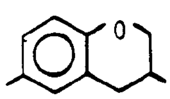 ; 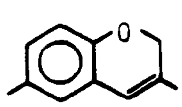

(i) (j)

**n -** peut prendre les valeurs de 1 à 4 inclus.

Les formes hydratées des acides de formule générale I ainsi que les sels organiques ou minéraux de I ($R_3$=H) thérapeutiquement acceptables et en particulier un sel choisi parmi : sodium, calcium, zinc, hydroxy-2 éthylammoniumm, bis(hydroxy-2 éthyl) ammonium, tris(hydroxy-2 éthyl) ammonium, morpholinium, et leur forme hydratée, les composés de formule générale I peuvent se présenter sous forme de mélange racémique ou être isolés sous forme d'énantiomères, de diastéréoisomères ou leur mélange en toute proportion ; caractérisé en ce qu'on effectue l'opération suivante pour A différent de b, e, h, j :

on fait réagir un ester benzocyclique ou benzohétérocyclique de formule **II**

$$H\text{-}A\text{-}COOR_5 \qquad \textbf{II}$$

avec un halogénure d'acyle fonctionnel de structure **III**

$$Y\text{-}(\overset{R_2}{\overset{|}{C}}H)_n\text{-}COY_1 \qquad \textbf{III}$$

selon les conditions de Friedel-Crafts en utilisant un solvant compatible, de préférence chloré et plus précisément le chlorure de méthylène ou le dichloro-1,2 éthane en présence d'un acide de Lewis comme le chlorure d'aluminium à titre d'exemple non limitatif et à une température variant de -5°C à +50°C de façon à obtenir le **composé IVa** de formule :

$$Y\text{-}(\overset{R_2}{\overset{|}{C}}H)_n\text{-}CO\text{-}A\text{-}COOR_5 \qquad \textbf{IVa}$$

qui est lui-même condensé avec l'azoture de sodium dans un mélange hydroalcoolique à une température comprise entre +10°C et +50°C pour donner le composé de formule :

$$N_3\text{-}(\overset{R_2}{\overset{|}{C}}H)_n\text{-}CO\text{-}A\text{-}COOR_5 \qquad \textbf{V}$$

qui est réduit sélectivement par l'hydrogène dans un alcool léger et renfermant une solution aqueuse d'acide fort -chlorhydrique par exemple- en présence de catalyseur d'hydrogénation déposé sur un support inerte et plus précisément le palladium sur charbon par exemple pour donner à une température comprise entre +10°C et +50°C l'aminocétoester chlorhydraté de formule **VI** :

$$NH_2\text{-}(\overset{R_2}{\overset{|}{C}}H)_n\text{-}CO\text{-}A\text{-}COOR_5,\ HCl \qquad \textbf{VI}$$

lui-même condensé avec un halogénure de sulfonyle (fluorure, chlorure, bromure) convenablement substitué de formule :

$$R\text{-}SO_2Z \qquad \textbf{VII}$$

dans un solvant basique organique comme la pyridine par exemple à une température comprise entre -15°C et +40°C pour donner le **composé I** avec X=CO et $R_1$=H, $R_3$ différent de H, de formule **VIII** :

$$R\text{-}SO_2NH\text{-}(\overset{R_2}{\overset{|}{C}}H)_n\text{-}\overset{O}{\overset{\|}{C}}\text{-}A\text{-}COOR_5 \qquad \textbf{VIII}$$

enfin, cet ester est saponifié par une solution aqueuse alcaline comme la soude ou la potasse en présence d'un solvant organique miscible comme un alcool ou un éther et plus précisément le méthanol, l'éthanol, le dioxanne ou le tétrahydrofuranne à une température comprise entre +10°C et celle d'ébullition du mélange pour donner après acidification le **composé I** acide avec X=CO, $R_1$=H, $R_3$=H de formule **IX**

$$R-SO_2NH-(\overset{R_2}{\overset{|}{C}H})_n-\overset{O}{\overset{\|}{C}}-A-COOH \qquad \underline{IX}$$

les radicaux des formules générales de **IVa** à **IX** ont la même signification que dans la revendication 1 et $R_5$=$R_3$ sauf H ; Y et $Y_1$ représentent un chlore ou un brome et Z un fluor ou un chlore ou un brome.

puis en ce que l'on effectue éventuellement au moins une transformation des composés de formules, I, IV, V ou VI obtenus précédemment, notamment par des opérations de réduction ou de condensation.

2) Procédé selon la revendication 1, caractérisé en ce que l'on réduit un composé de formule V

$$N_3-(\overset{R_2}{\overset{|}{C}H})_n-CO-A-COOR_5 \qquad \underline{V}$$

sous atmosphère d'hydrogène dans un alcool léger et renfermant une solution aqueuse d'acide fort en présence de catalyseur d'hydrogénation déposé sur un support inerte et plus précisément le palladium sur charbon par exemple pour donner à une température comprise entre +10°C et +60°C et pendant plusieurs heures - en contrôlant l'avancement de la réaction par chromatographie - le composé de formule **XII**

$$NH_2-(\overset{R_2}{\overset{|}{C}H})_n-\overset{OH}{\overset{|}{C}H}-A-COOR_5 \qquad \underline{XII}$$

qui est condensé avec un halogénure de sulfonyle (fluorure, chlorure, bromure) convenablement substitué de formule :

$R-SO_2Z$

dans un solvant basique organique comme la pyridine par exemple à une température comprise entre -15°C et +40°C pour donner le **composé I** avec X=CHOH et $R_1$=H et $R_3$ différent de H de formule **XIII**

$$R-SO_2NH-(\overset{R_2}{\overset{|}{C}H})_n-\overset{OH}{\overset{|}{C}H}-A-COOR_5 \qquad \underline{XIII}$$

enfin cet ester peut être saponifié par une solution aqueuse alcaline comme la soude ou la potasse en présence d'un solvant organique miscible alcoolique ou éthéré et plus précisément le méthanol, l'éthanol, le dioxanne ou le tétrahydrofuranne à une température comprise entre +10°C et +60°C pour donner après acidification le **composé I** acide avec X=CHOH, $R_1$=H, $R_3$=H de formule **XIV**

$$R-SO_2NH-(\overset{R_2}{\overset{|}{C}H})_n-\overset{OH}{\overset{|}{C}H}-A-COOH \qquad \underline{XIV}$$

les radicaux des formules générales **V**, **XII** à **XIV** ont la même signification que dans la revendication 1.

3) Procédé selon la revendication 1, caractérisé en ce que l'on réduit un composé de formule VI sous surpression d'hydrogène

$$HCl-NH_2-(\overset{R_2}{\overset{|}{C}H})_n-\overset{O}{\overset{\|}{C}}-A-COOR_5 \qquad \underline{VI}$$

en présence d'un acide fort comme l'acide sulfurique ou perchlorique en utilisant un catalyseur d'hydrogénation comme le palladium sur charbon dans un acide carboxylique comme solvant et plus particulièrement l'acide acétique en opérant à une température comprise entre 20 et 90°C sous une pression d'hydrogène comprise entre 0,1 et 5 atmosphères ; soit en réduisant le **composé VI** par un trialkyl silane et plus précisément

le triéthyl silane en présence d'acide trifluoroacétique à une température comprise entre +10°C et +40°C, soit en réduisant le **composé VI** par l'hydrogène naissant produit par l'action d'un acide (chlorhydrique ou acétique) sur le zinc amalgamé ou non en chauffant entre 40 et 120°C dans un solvant organique comme le toluène par exemple pour donner le **composé XV** sous forme de base ou de sel de formule :

$$NH_2-(\overset{R_2}{\overset{|}{C}}H)_n-CH_2-A-COOR_5 \qquad \underline{XV}$$

qui est ensuite condensé avec un halogénure de sulfonyle défini et dans les mêmes conditions que celles décrites dans les revendications 3 et 4 pour donner le **composé I** avec $R_1$=H et X=$CH_2$ de formule **XVI** homologue saturé de **VIII** et **XIII**

$$RSO_2NH-(\overset{R_2}{\overset{|}{C}}H)_n-CH_2-A-COOR_5 \qquad \underline{XVI}$$

qui est saponifié selon le procédé décrit dans les revendications 3 et 4 pour préparer les dérivés **IX** et **XIV**, pour donner le **composé I** avec $R_1$=$R_3$=H et X=$CH_2$ de formule **XVII**

$$RSO_2NH-(\overset{R_2}{\overset{|}{C}}H)_n-CH_2-A-COOH \qquad \underline{XVII}$$

où les radicaux des formules générales **VI**, **XV** à **XVII** ont la même signification que dans la revendication 1.

4) Procédé selon la revendication 1, caractérisé en ce que l'on réduit un composé de formule **IVa**,

$$Y-(\overset{R_2}{\overset{|}{C}}H)_n-\overset{O}{\overset{\|}{C}}-A-COOR_5 \qquad \underline{IVa}$$

selon l'une des méthodes de la revendication 3, pour donner le composé halogéné équivalent de **XV** ayant pour formule **XIX**

$$Y-(\overset{R_2}{\overset{|}{C}}H)_n-CH_2-A-COOR_5 \qquad \underline{XIX}$$

qui est condensé avec le sel de sodium préparé in situ d'un sulfonamide secondaire convenablement substitué de formule **XX**

$$R-SO_2NH-R_7 \qquad \underline{XX}$$

dans un solvant organique et plus particulièrement le diméthylformamide, le diméthylacétamide, l'acétone, la butanone, le tétrahydrofuranne ou le dioxanne à une température comprise entre +5°C et +60°C pour donner le composé de formule **XXI**

$$R-SO_2-\overset{R_7}{\overset{|}{N}}-(\overset{R_2}{\overset{|}{C}}H)_n-CH_2-A-COOR_5 \qquad \underline{XXI}$$

ce dérivé est ensuite saponifié selon le procédé décrit dans les revendications 3 et 4 relatif aux dérivés **IX** et **XIV** pour donner le composé équivalent saturé **I** avec X=$CH_2$ et $R_3$=H de formule **XXII**

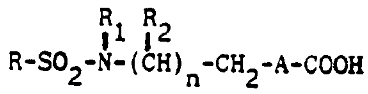

XXII

dans laquelle les radicaux des composés de formule **IVa**, **XIX** à **XXII** ont la même signification que dans la revendication 1 et où $R_7$ représente soit le radical $R_1$ différent de l'hydrogène soit le radical $R_6COO$- où $R_6$ représente un benzyle ou un alcoyle lèger ramifié ou non renfermant de 1 à 6 carbones inclus et à titre d'exemple non limitatif les radicaux : méthyle, éthyle, tertiobutyle.

5) Procédé selon la revendication 1, caractérisé en ce que l'on condense un composé de formule **IVa** préparé selon la revendication 1 avec le sel de sodium du **composé XX**

$R$-$SO_2NHR$ **XX**

préparé in situ selon le même procédé que pour préparer le **composé XXI** selon la revendication 4 pour donner le céto sulfamidoester **I** avec X=CO de formule **XXIV**

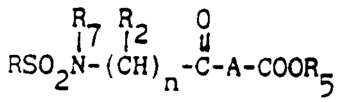

XXIV

qui est saponifié soit directement dans le cas où $R_7$ est différent de $COOR_6$ d'après le procédé décrit pour les **composés VIII** et **XIII** selon les revendications 1 et 2 pour donner ici le **composé I** avec X=CO, $R_3$=H et $R_1$ différent de H, de formule **XXV** ; soit dans le cas où $R_7$=COO-tBu est soumis en premier lieu à un clivage du groupement $R_7$ en milieu acide fort et plus précisément l'acide chlorhydrique dissous dans un solvant organique comme l'acétate d'éthyle par exemple pour donner le **composé XXV** avec $R_1$=H et $R_6$=$R_3$ différent de H qui peut être ensuite saponifié comme ci-dessus en acide pour donner le **composé I** avec $R_1$=$R_3$=H, X=CO de formule **XXV**

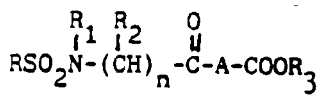

XXV

où les radicaux des formules des composés **IVa**, **XX**, **XXIV**, **XXV** ont la même signification que dans les revendications 1 et 4.

6) Procédé selon la revendication 1, caractérisé en ce que l'on réduit un dérivé de structure générale **XXIV** préparé selon la revendication 5

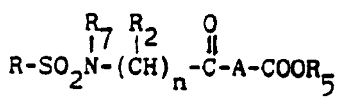

XXIV

selon une des trois méthodes décrites dans la revendication 3 pour préparer le **composé XV** pour donner ici l'homologue sulfonamido de formule **XXI** déjà décrit dans la revendication 4

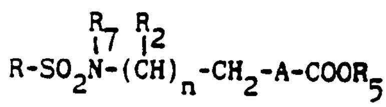

XXI

et saponifié en acide correspondant I avec X=$CH_2$ et $R_3$=H de formule XXII selon le procédé déjà décrit dans la revendication 4 et où les radicaux et variables des formules XXIV et XXI ont la même signification que dans les revendications 1 et 4.

7) Procédé selon la revendication 1, caractérisé en ce que l'on réduit un composé de formule générale I avec X=CO pour donner, selon un des procédés décrits dans la revendication 3, le sulfonamide réduit homologue I avec X=$CH_2$.

8) Procédé selon la revendication 1, caractérisé en ce que l'on réduit un composé de formule générale I où X=CO en présence de borohydrure de sodium ou de potassium dans un solvant protique comme un alcool léger de préférence de même condensation en carbone que $R_3$ lorsque $R_3$ est différent de H (de façon à éviter

une transestérification) à une température comprise entre +5°C et +50°C pour donner les composés de formule générale I avec X=CHOH.

9) Procédé selon la revendication 1, caractérisé en ce que l'on fait réagir un composé de formule I avec $R_3$=H avec un alcool de formule générale $R_3OH$ en présence de catalyseur acide comme l'acide sulfurique ou paratoluènesulfonique en chauffant à une température comprise entre 40°C et 110°C pour donner les composés de formule générale I avec $R_3$ différent de H.

10) Procédé selon la revendication 1, caractérisé en ce que l'on condense un composé de formule générale I où X=CO et $R_3$=H avec un chlorhydrate d'hydroxylamine substitué ou non de formule $R_4ONH_2$ pour donner dans la pyridine à une température comprise entre 10°C et 50°C les oximes correspondantes I avec X=C=N=O$R_4$ et $R_3$=H avec $R_4$ défini comme dans la revendication 1.

11) Procédé selon la revendication 1, caractérisé en ce que l'on condense un dérivé de formule générale I où X=CO et $R_3$=H avec une hydrazine de formule générale

$$NH_2\text{-}N\text{-}R_4$$
$$R_4$$

pour donner dans un solvant organique protique comme l'éthanol en présence d'unacide organique comme l'acide acétique par exemple, à une température comprise entre 25°C et 120°C, les hydrazones correspondantes I avec

$$X= C=N\text{-}N\text{-}R_4$$
$$R_4$$

et $R_3$=H avec $R_4$ défini comme dans la revendication 1.

12) Procédé selon la revendication 1, caractérisé en ce que l'on fait réagir un ester de formule générale I où le radical bicyclique -A- est saturé sur le cycle rattaché à la fonction carboxylate (c'est-à-dire où A=a,d,g,i) avec un halogénoimide et plus particulièrement le N-bromo ou N-chlorosuccinimide dans un solvant halogéné et plus précisément comprise entre +20°C et +80°C pour donner les composés insaturés correspondants de formule générale I (c'est-à-dire où A=b,e,h) avec $R_3$ différent de H qui sont ensuite saponifiés en leurs acides précurseurs de formule générale <u>I</u> avec $R_3$=H selon le protocole décrit dans la revendication 1.

13) Procédé selon la revendication 1, caractérisé en ce que l'on fait réagir un composé de formule générale I dans laquelle $R_1$=H avec un dérivé activé de formule $(R_1)_2SO_4$ ou $R_1Z$ avec Z=Br ou I en présence d'une base forte comme l'hydrure de sodium ou un carbonate ou un hydroxyde de sodium ou de potassium dans un solvant comme le DMF, le diméthyl acétamide, la butanone, l'acétone et à une température comprise entre +20°C et +70°C pour donner le composé équivalent de formule générale I dans laquelle $R_1$ et $R_3$ sont différents de H et où les radicaux variables sont définis comme dans la revendication 1.

14) Procédé selon la revendication 1, avec A différent de b, e, h et j caractérisé en ce que l'on fait réagir un ester benzocarbocyclique ou benzohétérocyclique de formule générale **<u>II</u>**

avec un halogènure d'acyle fonctionnel de structure **<u>IIIa</u>**

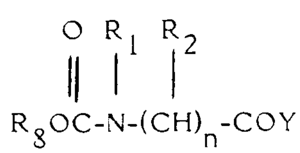

<u>IIIa</u>

selon les conditions de Friedel et Crafts en utilisant un solvant compatible de préférence chloré et plus précisément le chlorure de méthylène ou le dichloro-1,2 éthane en présence d'un acide de Lewis comme le chlorure d'aluminium à titre d'exemple non limitatif et à une température comprise entre -5°C et +50°C de façon à obtenir le composé **<u>IVb</u>** de formule :

$$\begin{array}{l} \qquad\quad R_1 \;\; R_2 \\ R_8OCON\text{-}(CH)_n\text{-}CO\text{-}A\text{-}COOR_5 \end{array}$$ IVb

qui est lui-même réduit par un borohydrure alcalin selon le procédé décrit dans la revendication 8 de façon à obtenir le dérivé **XXIX** de formule :

$$\begin{array}{l} \qquad\qquad R_1 \;\; R_2 \\ \qquad\qquad | \quad\; | \\ R_8OCO\text{-}N\text{-}(CH)_n\text{-}CHOH\text{-}A\text{-}COOR_5 \end{array}$$ XXIX

pour conduire par hydrolyse basique au sel de sodium de l'aminohydroxy acide de formule **XXX** selon la méthode décrite dans la revendication 1 pour la préparation du composé **IX**

$$\begin{array}{l} \qquad\qquad R_2 \\ \qquad\qquad | \\ R_1NH\text{-}(CH)_n\text{-}CHOH\text{-}A\text{-}COOH \end{array}$$ XXX

et enfin en condensant un halogénure de sulfonyle convenablement substitué de formule $RSO_2Z$ sur le sel de sodium brut de **XXX** précédemment obtenu dans la même phase aqueuse organique à un pH compris entre 11 et 13 et à une température comprise entre 0 et +40°C pour donner le **composé I** avec $R_3$=H et Y=CHOH de formule **XXVII**

$$\begin{array}{l} \qquad\qquad R_1 \;\; R_2 \\ \qquad\qquad | \quad\; | \\ ArSO_2N\text{-}(CH)\text{-}CHOH\text{-}A\text{-}COOH \end{array}$$ XXVII

dans laquelle les radicaux des formules générales **IVb**, **XXIX**, **XXX**, **XXVII** ont la même signification que dans 1 et $R_8$ représente un radical alcoyle linéaire léger de 1 à 6 carbone inclus.

15) Procédé selon la revendication 1, caractérisé en ce que l'on réduit selon un des procédés décrits dans la revendication 3 la cétone d'un composé de formule générale **IVb** préparé comme décrit dans la revendication 14

$$\begin{array}{l} \qquad\qquad R_1 \;\; R_2 \\ \qquad\qquad | \quad\; | \\ R_8OCON\text{-}(CH)_n\text{-}CO\text{-}A\text{-}COOR_5 \end{array}$$ IVb

en groupement méthylène pour donner le dérivé **XXXI** de formule:

$$\begin{array}{l} \qquad\qquad R_1 \;\; R_2 \\ \qquad\qquad | \quad\; | \\ R_8OCON\text{-}(CH)_n\text{-}CH_2\text{-}A\text{-}COOR_5 \end{array}$$ XXXI

qui ensuite saponifié selon le procédé décrit dans la revendication 14 pour conduire au sel de sodium de l'amino acide de formule **XXXII**

$$\begin{array}{l} \qquad\qquad R_2 \\ \qquad\qquad | \\ R_1NH\text{-}(CH)_n\text{-}CH_2\text{-}A\text{-}COOH \end{array}$$ XXXII

qui est condensé sans être isolé avec l'halogénure de sulfonyle de formule $RSO_2Z$ selon la méthode décrite dans la revendication 14 pour donner après acidification le composé I avec $X=CH_2$ et $R_3=H$ de formule **XXIII**

$$RSO_2\overset{R_1}{\overset{|}{N}}-(\overset{R_2}{\overset{|}{C}}H)_n-CH_2-A-COOH \qquad \underline{XXIII}$$

dans laquelle les radicaux des formules **IVb**, **XXXI**, **XXXII**, **XXIII** ont la même signification que dans les revendications 1 et 14.

16) Procédé selon la revendication 1, caractérisé en ce que l'on fait réagir l'acide de formule générale I avec $R_3=H$ avec une quantité stoëchiométrique de base organique ou minérale dans un solvant protique comme l'eau, le méthanol, l'éthanol ou cétonique comme la butanone ou l'acétone à une température comprise entre 0°C et +60°C.

17) Procédé selon les revendications 1 et 5, caractérisé en ce que l'on hydrolyse un composé de formule générale I avec $R_1$=COO-tBu, $R_3$ différent de H, en versant le mélange réactionnel brut ainsi obtenu dans une solution aqueuse concentrée d'acide fort et plus précisément l'acide chlorydrique, en chauffant à une température variant de 40 à 100°C et plus précisément comprise entre 50 et 70°C pour donner le composé I où $R_1=R_3=H$ et où les autres radicaux ont la même signification que dans 1.

## Patentansprüche

## Patentansprüche für folgende Vertragsstaaten : AT, BE, CH, LI, DE, DK, FR, GB, IT, LU, NL, SE

1. Neue Sulfonamid-Derivate von carbocyclischen oder benzoheterocyclischen Säuren der allgemeinen Formel (I)

$$R-SO_2-\overset{R_1}{\overset{|}{N}}-(\overset{R_2}{\overset{|}{C}}H)_n-X-A-COOR_3 \qquad (I)$$

in der die Reste und Substituenten wie folgt definiert sind:

R steht für einen linearen oder verzweigten leichten Alkylrest mit 1 bis 9 Kohlenstoffatomen, Phenyl- oder Naphthylreste, die unsubstituiert oder substituiert sind durch eine oder mehrere der folgenden Gruppen: niederes Alkyl (mit 1 bis 4 C), Halogen, niederes Alkyloxy (mit 1 bis 4 C), Nitro, Amino, leichtes Dialkylamino (mit 1 bis 4 C);

$R_1$ steht für ein Wasserstoffatom oder einen linearen oder verzweigten niederen Alkylrest (mit 1 bis 4 C) oder einen Benzylrest;

$R_2$ steht für ein Wasserstoffatom oder einen linearen oder verzweigten leichten Alkylrest (mit 1 bis 6 C), eine Phenylgruppe, die unsubstituiert oder substituiert ist durch ein Chlor oder ein Methoxyl, eine Arylalkylgruppe mit 7 bis 9 Kohlenstoffatomen;

$R_3$ steht für ein Wasserstoffatom oder einen verzweigten oder unverzweigten niederen Alkylrest (mit 1 bis 6 C) ;

-X- steht für einen divalenten funktionellen Rest, der ausgewählt wird unter den folgenden:
$-CH_2-$ ; $-CH-OR_4$ ; $-C=O$ ; $-C=N-OR_4$ ;

$$-C=N-\underset{R_4}{\underset{|}{N}}-R_4$$

mit $R_4$=H,Me wobei -X- verschieden ist von $-CH_2-$, wenn -A- die nachstehend angegebenen divalenten Reste a, b, d und e darstellt;

-A- steht für einen bivalenten benzocyclischen oder benzoheterocyclischen Rest, der ausgewählt wird unter den folgenden Resten (a-j):

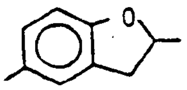 ; 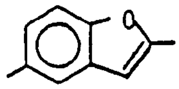 ; 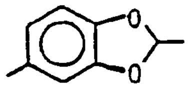 ; 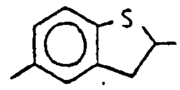

(a) (b) (c) (d)

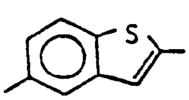 ; 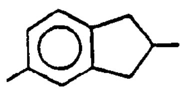 ; 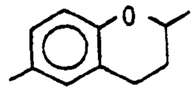 ; 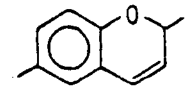

(e) (f) (g) (h)

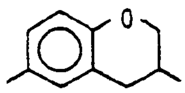 ; 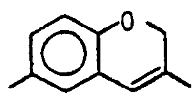

(i) (j)

und

n kann die Werte 1 bis einschließlich 4 annehmen,

die hydratisierten Formen der Säuren der allgemeinen Formel (I) sowie die therapeutisch akzeptablen organischen oder mineralischen Salze von (I) ($R_3$=H) und insbesondere ein Salz, das ausgewählt wird aus den Salzen von Natrium, Calcium, Zink, 2-Hydroxyethylammonium, Bis(2-hydroxyethyl)ammonium, Tris(2-hydroxyethyl)ammonium, Morpholinium und ihrer hydratisierten Form, wobei die Verbindungen der allgemeinen Formel (I) in Form einer racemischen Mischung vorliegen können oder isoliert sein können in Form ihrer Enantiomeren, Diastereoisomeren oder ihrer Mischung in einem beliebigen Mengenverhältnis.

**2.** Verbindung nach Anspruch 1 dadurch gekennzeichnet, daß sie unter den folgenden Verbindungen ausgewählt wird:

- 5-(p-Chlorobenzolsulfonamidoacetyl)-2,3-dihydro-2-benzofurancarbonsäure
- 5-(2-p-Chlorobenzolsulfonamido-1-hydroxy-ethyl)-2,3-dihydro-2-benzofurancarbonsäure
- 5-(2-p-Chlorobenzolsulfonamido-1-hydroxyimino-ethyl)-2,3-dihydro-2-benzofurancarbonsäure
- 5-(2-p-Chlorobenzolsulfonamido-1-methoxyimino-ethyl)-2,3-dihydro-2-benzofurancarbonsäure
- 5-(N-Methyl-p-chlorobenzolsulfonamidoacetyl)-2,3-dihydro-2-benzofurancarbonsäure
- 5-($\alpha$-Methyl-p-chlorobenzolsulfonamidoacetyl)-2,3-dihydro-2-benzofurancarbonsäure
- 5-(2-p-Chlorobenzolsulfonamido-1-hydroxy-propyl)-2,3-dihydro-2-benzofurancarbonsäure
- p-Chlorobenzolsulfonamidoacetyl-2,3-dihydro-2-benzofuranethylcarboxylat
- 5-(2-p-Chlorobenzolsulfonamido-ethyl)-1,3-benzodioxol-2-carbonsäure
- 5-(2-p-Chlorobenzolsulfonamido-ethyl)-2-indancarbonsäure
- 6-(2-p-Chlorobenzolsulfonamido-ethyl)-2-chromancarbonsäure
- 6-(p-Chlorobenzolsulfonamidoacetyl)-2-chromancarbonsäure
- 6-(p-Chlorobenzolsulfonamidoacetyl)-2-chroman-ethylcarboxylat
- 5-(Benzolsulfonamidoacetyl)-2,3-dihydro-2-benzofurancarbonsäure
- 5-(p-Methoxybenzolsulfonamidoacetyl)-2,3-dihydro-2-benzofurancarbonsäure
- 5-(Pentafluorobenzolsulfonamidoacetyl)-2,3-dihydro-2-benzofurancarbonsäure
- 5-(p-Chlorobenzolsulfonamidoacetyl)-2-indancarbonsäure
- 5-(p-Chlorobenzolsulfonamidoacetyl)-2,3-dihydro-2-benzofurancarboxylat von Lysin
- 6-(2-p-Chlorobenzolsulfonamido-ethyl)-2-chromancarboxylat von Lysin
- 6-(p-Chlorobenzolsulfonamidoacetyl)-2-chromancarboxylat von Lysin
- 5-(p-Chlorobenzolsulfonamidoacetyl)-2-indancarboxylat von Lysin.

**3.** Verfahren zur Herstellung der chemischen Verbindungen nach den Ansprüchen 1 und 2, worin A verschieden ist von b, e, h und j, dadurch gekennzeichnet, daß man einen benzocyclischen oder benzoheterocyclischen Ester der Formel (II)

$$H\text{-}A\text{-}COOR_5 \qquad (II)$$

reagieren läßt mit einem funktionellen Acylhalogenid der Struktur (III)

$$Y\text{-}(\overset{R_2}{\overset{|}{C}}H)_n\text{-}COY_1 \qquad \text{(III)}$$

unter den Friedel-Crafts-Bedingungen unter Verwendung eines kompatiblen, vorzugsweise chlorierten Lösungsmittels und insbesondere unter Verwendung von Methylenchlorid oder 1,2-Dichlorethan, in Gegenwart einer Lewis-Säure wie beispielsweise, jedoch nicht beschränkend, Aluminiumchlorid und bei einer Temperatur, die von -5°C bis zu +50°C variiert, in der Weise, daß man die Verbindung (IVa) der Formel erhält:

$$Y\text{-}(\overset{R_2}{\overset{|}{C}}H)_n\text{-}CO\text{-}A\text{-}COOR_5 \qquad \text{(IVa)}$$

die selbst kondensiert wird mit Natriumnitrid in einem Wasser/Alkohol-Gemisch bei einer Temperatur zwischen +10°C und +50°C unter Bildung der Verbindung der Formel:

$$N_3\text{-}(\overset{R_2}{\overset{|}{C}}H)_n\text{-}CO\text{-}A\text{-}COOR_5 \qquad \text{(V)}$$

die selektiv reduziert wird mit Wasserstoff in einem leichten Alkohol, der eine wäßrige Lösung einer starken Säure - beispielsweise Chlorwasserstoffsäure - enthält, in Gegenwart eines Hydrierungskatalysators, der auf einem inerten Träger abgeschieden ist, insbesondere beispielsweise in Gegenwart von Palladium auf Kohle, unter Bildung bei einer Temperatur zwischen +10°C und +50°C des Aminoketoesterhydrochlorids der Formel (VI):

$$NH_2\text{-}(\overset{R_2}{\overset{|}{C}}H)_n\text{-}CO\text{-}A\text{-}COOR_5,\ HCl \qquad \text{(VI)}$$

das selbst kondensiert wird mit einem in geeigneter Weise substituierten Sulfonylhalogenid (-fluorid, -chlorid, -bromid) der Formel:

$$R\text{-}SO_2Z \qquad \text{(VII)}$$

in einem basischen organischen Lösungsmittel, wie beispielsweise Pyridin, bei einer Temperatur zwischen -15°C und +40°C unter Bildung der Verbindung (I) mit X = CO und $R_1$ = H, $R_3$ = verschieden von H, der Formel (VIII):

$$R\text{-}SO_2NH\text{-}(\overset{R_2}{\overset{|}{C}}H)_n\text{-}\overset{O}{\overset{\|}{C}}\text{-}A\text{-}COOR_5 \qquad \text{(VIII)}$$

wobei anschließend dieser Ester mit einer wäßrigen alkalischen Lösung, wie einer Natriumcarbonatlösung oder Kaliumcarbonatlösung, verseift wird in Gegenwart eines damit mischbaren organischen Lösungsmittels, wie eines Alkohols oder eines Äthers und insbesondere in Gegenwart von Methanol, Ethanol, Dioxan oder Tetrahydrofuran, bei einer Temperatur zwischen +10°C und dem Siedepunkt der Mischung, wobei man nach dem Ansäuern die Säure-Verbindung (I) mit X = CO, $R_1$ = H, $R_3$ = H der Formel (IX) erhält

$$R-SO_2NH-(\overset{R_2}{\overset{|}{C}}H)_n-\overset{O}{\overset{\|}{C}}-A-COOH \qquad (IX)$$

wobei die Reste der allgemeinen Formeln (IVa) bis (IX) die gleiche Bedeutung wie im Anspruch 1 haben und $R_5 = R_3$ ausgenommen H; Y und $Y_1$ Chlor oder Brom und Z Fluor oder Chlor oder Brom darstellen.

4. Verfahren zur Herstellung der chemischen Verbindungen nach den Ansprüchen 1 und 2, dadurch gekennzeichnet, daß man eine nach Anspruch 3 hergestellte Verbindung der Formel (V)

$$N_3-(\overset{R_2}{\overset{|}{C}}H)_n-CO-A-COOR_5 \qquad (V)$$

reduziert unter einer Wasserstoffatomosphäre in einem leichten Alkohol, der eine wäßrige Lösung einer starken Säure enthält, in Gegenwart eines Hydrierungskatalysators, der auf einem inerten Träger abgeschieden ist, insbesondere in Gegenwart von beispielsweise Palladium auf Kohle, unter Bildung bei einer Temperatur zwischen +10°C und +60°C und während mehrerer Stunden - wobei man das Fortschreiten der Reaktion durch Chromatographie kontrolliert - der Verbindung der Formel (XII)

$$NH_2-(\overset{R_2}{\overset{|}{C}}H)_n-\overset{OH}{\overset{|}{C}}H-A-COOR_5 \qquad (XII)$$

die kondensiert wird mit einem in geeigneter Weise substituierten Sulfonylhalogenid (-fluorid, -chlorid, -bromid) der Formel:

$$R-SO_2Z$$

in einem organischen basischen Lösungsmittel, wie beispielsweise Pyridin, bei einer Temperatur zwischen -15°C und +40°C unter Bildung der Verbindung (I) mit X = CHOH und $R_1$ = H und $R_3$ = verschieden von H der Formel (XIII):

$$R-SO_2NH-(\overset{R_2}{\overset{|}{C}}H)_n-\overset{OH}{\overset{|}{C}}H-A-COOR_5 \qquad (XIII)$$

wobei dieser Ester dann verseift werden kann mit einer alkalischen wäßrigen Lösung, wie einer Natriumcarbonatoder Kaliumcarbonatlösung, in Gegenwart eines damit mischbaren alkoholischen oder etherischen organischen Lösungsmittels, insbesondere in Gegenwart von Methanol, Ethanol, Dioxan oder Tetrahydrofuran, bei einer Temperatur zwischen +10°C und +60°C, wobei man nach dem Ansäuern erhält die Säure-Verbindung (I) mit X = CHOH, $R_1$ = H, $R_3$ = H der Formel (XIV)

$$R-SO_2NH-(\overset{R_2}{\overset{|}{C}}H)_n-\overset{OH}{\overset{|}{C}}H-A-COOH \qquad (XIV)$$

worin die Reste der allgemeinen Formeln (V), (XII) bis (XIV) die gleiche Bedeutung wie in den Ansprüchen 1 und 3 haben.

5. Verfahren zur Herstellung der chemischen Verbindung nach den Ansprüchen 1 und 2, dadurch gekennzeichnet, daß man eine nach Anspruch 3 hergestellte Verbindung der Formel (VI)

$$HCl-NH_2-(\overset{R_2}{\overset{|}{C}}H)_n-\overset{O}{\overset{\|}{C}}-A-COOR_5 \qquad (VI)$$

reduziert entweder unter einem Wasserstoffüberdruck in Gegenwart einer starken Säure, wie Essigsäure oder Perchlorsäure, unter Verwendung eines Hydrierungskatalysators, wie Palladium auf Kohle, in einer Carbonsäure als Lösungsmittel und insbesondere in Essigsäure als Lösungsmittel, wobei man bei einer Temperatur zwischen 20 und 90°C unter einem Wasserstoffdruck zwischen 0,1 und 5 Atmosphären arbeitet, oder indem man die Verbindung (VI) mit einem Trialkylsilan und insbesondere mit Triethylsilan reduziert in Gegenwart von Trifluoressigsäure bei einer Temperatur zwischen +10°C und +40°C oder indem man die Verbindung (VI) mit naszierendem Wasserstoff reduziert, der gebildet worden ist durch Einwirkung einer Säure (Chlorwasserstoffsäure oder Essigsäure) auf amalgamiertes oder nichtamalgamiertes Zink, wobei man auf eine Temperatur zwischen 40 und 120°C erwärmt, in einem organischen Lösungsmittel, wie beispielsweise Toluol, unter Bildung der Verbindung (XV) in Form einer Base oder eines Salzes der Formel:

$$NH_2-(\overset{R_2}{\overset{|}{C}}H)_n-CH_2-A-COOR_5 \qquad (XV)$$

die anschließend kondensiert wird mit einem Sulfonylhalogenid, wie es definiert ist in und unter den Bedingungen, wie sie beschrieben sind in den Ansprüchen 3 und 4, unter Bildung der Verbindung (I) mit $R_1$ = H und X = $CH_2$ der Formel (XVI), die ein gesättigtes Homologes von (VIII) und (XIII) ist

$$RSO_2NH-(\overset{R_2}{\overset{|}{C}}H)_n-CH_2-A-COOH \qquad (XVII)$$

die verseift wird nach dem Verfahren, wie es in den Ansprüchen 3 und 4 beschrieben ist zur Herstellung der Derivate (IX) und (XIV) unter Bildung der Verbindung (I) mit $R_1 = R_3$ = H und X = $CH_2$ der Formel (XVII)

$$RSO_2NH-(\overset{R_2}{\overset{|}{C}}H)_n-CH_2-A-COOH \qquad (XVII)$$

wobei die Reste der allgemeinen Formeln (VI), (XV) bis (XVII) die gleiche Bedeutung wie in den Ansprüchen 1 und 3 haben.

**6.** Verfahren zur Herstellung der chemischen Verbindungen, wie sie in den Ansprüchen 1 und 2 definiert sind, dadurch gekennzeichnet, daß man eine nach Anspruch 3 hergestellte Verbindung der Formel (IVa)

$$Y-(\overset{R_2}{\overset{|}{C}}H)_n-\overset{O}{\overset{\|}{C}}-A-COOR_5 \qquad (IVa)$$

reduziert nach einer der Methoden des Anspruchs 5 unter Bildung der halogenierten Verbindung, äquivalent zu (XV) mit der Formel (XIX)

$$Y-(\overset{R_2}{\overset{|}{C}}H)_n-CH_2-A-COOR_5 \qquad (XIX)$$

die kondensiert wird mit dem in situ hergestellten Natriumsalz eines in geeigneter Weise substituierten sekundären Sulfonamids der Formel (XX)

$R\text{-}SO_2NH\text{-}R_7$ (XX)

in einem organischen Lösungsmittel und insbesondere in Dimethylformamid, Dimethylacetamid, Aceton, Butanon, Tetrahydrofuran oder Dioxan, bei einer Temperatur zwischen +5°C und +60°C unter Bildung der Verbindung der Formel (XXI)

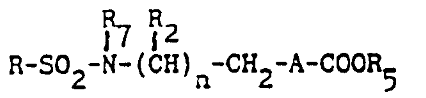 (XXI)

wobei dieses Derivat anschließend nach dem in den Ansprüchen 3 und 4 in bezug auf die Derivate (IX) und (XIV) beschriebenen Verfahren verseift wird unter Bildung der äquivalenten gesättigten Verbindung (I) mit $X = CH_2$ und $R_3 = H$ der Formel (XXII)

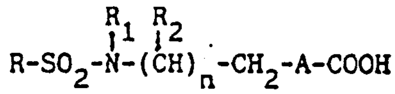 (XXII)

wobei die Reste der Verbindungen der Formeln (IVa), (XIX) bis (XXII) die gleiche Bedeutung haben wie in den Ansprüchen 1 bis 3 und $R_7$ entweder den Rest $R_1$, der von Wasserstoff verschieden ist, oder den Rest $R_6COO$- darstellt, worin $R_6$ eine Benzylgruppe oder eine verzweigte oder unverzweigte leichte Alkylgruppe mit 1 bis einschließlich 6 Kohlenstoffatomen, wie beispielsweise, aber nicht begrenzend, Methyl-, Ethyl-, tert-Butylreste bedeutet.

**7.** Verfahren zur Herstellung der chemischen Verbindungen, wie sie in den Ansprüchen 1 und 2 definiert sind, dadurch gekennzeichnet, daß man eine nach Anspruch 3 hergestellte Verbindung der Formel (VIa) kondensiert mit dem Natriumsalz der Verbindung (XX)

$R\text{-}SO_2NHR_7$ (XX)

das in situ hergestellt worden ist nach dem gleichen Verfahren wie für die Herstellung der Verbindung (XXI) in Anspruch 6 beschrieben, unter Bildung des Ketosulfamidoesters (I) mit X = CO der Formel (XXIV)

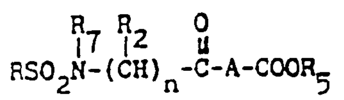 (XXIV)

der verseift wird entweder direkt für den Fall, daß $R_7$ von $COOR_6$ verschieden ist, nach dem für die Verbindungen (VIII) und (XIII) in den Ansprüchen 3 und 4 beschriebenen Verfahren, wobei man hier die Verbindung (I) erhält mit X = CO, $R_3 = H$ und $R_1$ = verschieden von H der Formel (XXV); oder für den Fall, daß $R_7$ = COO-t-Bu, indem man es an erster Stelle einer Abspaltung der Gruppe $R_7$ im Milieu einer starken Säure, insbesondere Chlorwasserstoffsäure, gelöst in einem organischen Lösungsmittel, wie beispielsweise Ethylacetat, unterwirft unter Bildung der Verbindung (XXV) mit $R_1 = H$ und $R_5 = R_3$, das von H verschieden ist, das anschließend wie oben angegeben in einer Säure verseift werden kann unter Bildung der Verbindung (I) mit $R_1 = R_3 = H$, X = CO der Formel (XXV)

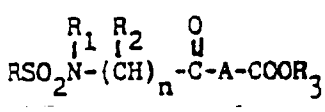 (XXV)

wobei die Reste der Formeln der Verbindungen (IVa), (XX), (XXIV), (XXV) die gleiche Bedeutung wie in den Ansprüchen 1, 3 und 6 haben.

**8.** Verfahren zur Herstellung der chemischen Verbindungen, wie sie in den Ansprüchen 1 und 2 definiert sind,

dadurch gekennzeichnet, daß man ein nach Anspruch 7 hergestelltes Derivat mit der allgemeinen Struktur (XXIV)

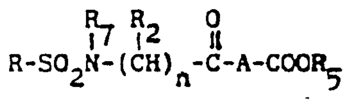

(XXIV)

reduziert nach einer der drei Methoden, wie sie im Anspruch 5 beschrieben sind zur Herstellung der Verbindung (XV), wobei man hier das Sulfonamid-Homologe der Formel (XXI) erhält, wie bereits im Anspruch 6 beschrieben

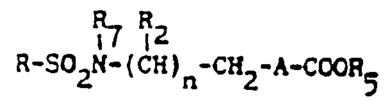

(XXI)

und zu einer entsprechenden Säure (I) mit X = $CH_2$ und $R_3$ = H der Formel (XII) verseift nach dem in Anspruch 6 bereits beschriebenen Verfahren, wobei die Reste und Variablen der Formeln (XXIV) und (XXI) die gleichen Bedeutungen wie in den Ansprüchen 1, 3 und 6 haben.

**9.** Verfahren zur Herstellung der chemischen Verbindungen, wie sie in den Ansprüchen 1 und 2 definiert sind, dadurch gekennzeichnet, daß man eine Verbindung der allgemeinen Formel (I) mit X = CO reduziert unter Bildung nach einem der in Anspruch 5 beschriebenen Verfahren des reduzierten Sulfonamid-Homologen der Formel (I) mit X = $CH_2$.

**10.** Verfahren zur Herstellung der chemischen Verbindungen, wie sie in den Ansprüchen 1 und 2 definiert sind, dadurch gekennzeichnet, daß man eine Verbindung der allgemeinen Formel (I), worin X = CO, reduziert in Gegenwart von Natrium- oder Kaliumborhydrid in einem protischen Lösungsmittel, wie einem leichten Alkohol, vorzugsweise mit der gleichen Anzahl von an Kohlenstoffatomen wie $R_3$, wenn $R_3$ von H verschieden ist (um eine Umesterung zu vermeiden) bei einer Temperatur zwischen +5°C und +50°C unter Bildung der Verbindungen der allgemeinen Formel (I) mit X = CHOH).

**11.** Verfahren zur Herstellung der chemischen Verbindungen nach den Ansprüchen 1 und 2, dadurch gekennzeichnet, daß man eine Verbindung der Formel (I) mit $R_3$ = H reagieren läßt mit einem Alkohol der allgemeinen Formel $R_3OH$ in Gegenwart eines Säurekatalysators, wie Schwefelsäure oder p-Toluolsulfonsäure, wobei man auf eine Temperatur zwischen 40 und 110°C erwärmt, unter Bildung der Verbindungen der allgemeinen Formel (I) mit $R_3$ = verschieden von H.

**12.** Verfahren zur Herstellung der chemischen Verbindungen nach den Ansprüchen 1 und 2, dadurch gekennzeichnet, daß man eine Verbindung der allgemeinen Formel (I), worin X = CO und $R_3$ = H, kondensiert mit einem unsubstituierten oder substituierten Hydroxylaminhydrochlorid der Formel

$$R_4ONH_2$$

unter Bildung in Pyridin bei einer Temperatur zwischen 10°C und 50°C der entsprechenden Oxime (I) mit X = C=N-$OR_4$ und $R_3$ = H, wobei $R_4$ wie in Anspruch 1 definiert ist.

**13.** Verfahren zur Herstellung der chemischen Verbindungen nach den Ansprüchen 1 und 2, dadurch gekennzeichnet, daß man ein Derivat der allgemeinen Formel (I), worin X = CO und $R_3$ = H, kondensiert mit einem Hydrazin der allgemeinen Formel

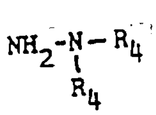

unter Bildung in einem protischen organischen Lösungsmittel wie Ethanol in Gegenwart einer organischen Säure wie beispielsweise Essigsäure bei einer Temperatur zwischen 25 und 120°C der entsprechenden Hydrazone (I) mit X =

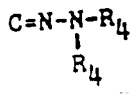

und $R_3$ = H, wobei $R_4$ wie in Anspruch 1 definiert ist.

**14.** Verfahren zur Herstellung der chemischen Verbindungen nach den Ansprüchen 1 und 2, dadurch gekennzeichnet, daß man einen Ester der allgemeinen Formel (I), in der der bicyclische Rest -A- gesättigt ist an dem an die Carboxylatfunktion gebundenen Cyclus (d.h. worin A = a, d, g, i), reagieren läßt mit einem Halogenimid und insbesondere mit N-Brom- oder N-Chlorsuccinimid in einem halogenierten Lösungsmittel und insbesondere in Tetrachlorkohlenstoff bei einer Temperatur zwischen +20°C und +80°C unter Bildung der entsprechenden ungesättigten Verbindungen der allgemeinen Formel (I) (d.h. worin A = b, e, h) mit $R_3$ = verschieden von H, die anschließend zu ihren Vorläufer-Säuren der allgemeinen Formel (I) mit $R_3$ = H verseift werden nach der in Anspruch 3 beschriebenen Vorschrift.

**15.** Verfahren zur Herstellung der chemischen Verbindungen nach den Ansprüchen 1 und 2, dadurch gekennzeichnet, daß man eine Verbindung der allgemeinen Förmel (I), in der $R_1$ = H, reagieren läßt mit einem aktiven Derivat der Formel

$$(R_1)_2SO_4 \text{ oder } R_1Z$$

mit Z = Br oder J in Gegenwart einer starken Base, wie Natriumhydrid oder eines Carbonats oder Hydroxids von Natrium oder Kalium, in einem Lösungsmittel wie DMF, Dimethylacetamid, Butanon, Aceton bei einer Temperatur zwischen +20°C und +70°C unter Bildung der äquivalenten Verbindung der allgemeinen Formel (I), worin $R_1$ und $R_3$ von H verschieden sind und worin die variablen Reste wie in Anspruch 1 definiert sind.

**16.** Verfahren zur Herstellung der chemischen Verbindungen nach den Ansprüchen 1 und 2, worin A verschieden ist von b, e, h und j, dadurch gekennzeichnet, daß man einen benzocarbocyclischen oder benzoheterocyclischen Ester der allgemeinen Formel (II)

$$H - A - COOR_5 \qquad (II)$$

reagieren läßt mit einem funktionellen Acylhalogenid der Struktur (IIIa)

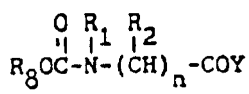

(IIIa)

unter den Friedel-Crafts-Bedingungen unter Verwendung eines kompatiblen, vorzugsweise chlorierten Lösungsmittels und insbesondere unter Verwendung von Methylenchlorid oder 1,2-Dichlorethan in Gegenwart einer Lewis-Säure wie beispielsweise, jedoch nicht beschränkend, Aluminiumchlorid bei einer Temperatur zwischen -5°C und +50°C, so daß man die Verbindung der Formel (IVb) erhält

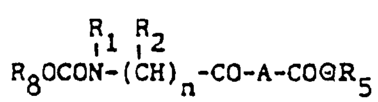

(IVb)

die selbst durch ein Alkaliborhydrid nach dem in Anspruch 10 beschriebenen Verfahren so reduziert wird, daß man das Derivat der Formel (XXIX) erhält:

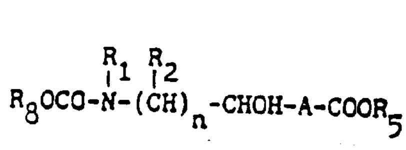

(XXIX)

die durch basische Hydrolyse zu dem Natriumsalz der Aminohydoxysäure der Formel (XXX) führt nach dem in Anspruch 3 führt zur Herstellung der Verbindung (IX) beschriebenen Verfahren

$$R_1NH-(\overset{R_2}{\overset{|}{C}}H)_n-CHOH-A-COOH \qquad (XXX)$$

und wobei man schließlich ein in geeigneter Weise substituiertes Sulfonylhalogenid der Formel $RSO_2Z$ kondensiert mit dem oben erhaltenen rohen Natriumsalz der Formel (XXX) in der gleichen wäßrig-organischen Phase bei einem pH-Wert zwischen 11 und 13 und bei einer Temperatur zwischen 0 und +40°C unter Bildung der Verbindung (I) mit $R_3$ = H und Y = CHOH der Formel (XXVII)

$$ArSO_2\overset{R_1}{\overset{|}{N}}-(\overset{R_2}{\overset{|}{C}}H)-CHOH-A-COOH \qquad (XXVII)$$

wobei die Reste der allgemeinen Formeln (IVb), (XXIX), (XXX), (XXVII) die gleiche Bedeutung wie in den Ansprüchen 1 und 3 haben und $R_8$ einen leichten linearen Alkylrest mit 1 bis einschließlich 6 Kohlenstoffatomen darstellt.

**17.** Verfahren zur Herstellung der chemischen Verbindung nach den Ansprüchen 1 und 2, dadurch gekennzeichnet, daß man nach einem der in Anspruch 5 beschriebenen Verfahren das Keton einer Verbindung der allgemeinen Formel (IVb), hergestellt wie in Anspruch 16 beschrieben,

$$R_8OCO\overset{R_1}{\overset{|}{N}}-(\overset{R_2}{\overset{|}{C}}H)_n-CO-A-COOR_5 \qquad (IVb)$$

zu einer Methylengruppe reduziert unter Bildung des Derivats (XXXI) der Formel

$$R_8OCO\overset{R_1}{\overset{|}{N}}-(\overset{R_2}{\overset{|}{C}}H)_n-CH_2-A-COOR_5 \qquad (XXXI)$$

das anschließend verseift wird nach dem im Anspruch 16 beschriebenen Verfahren, was zu dem Natriumsalz der Aminosäure der Formel (XXXII) führt

$$R_1NH-(\overset{R_2}{\overset{|}{C}}H)_n-CH_2-A-COOH \qquad (XXXII)$$

das, ohne isoliert zu werden, mit dem Sulfonylhalogenid der Formel $RSO_2Z$ nach dem in Anspruch 16 beschriebenen Verfahren kondensiert wird, wobei man nach dem Ansäuern die Verbindung (I) mit X = $CH_2$ und $R_3$ = H der Formel (XXIII) erhält

$$RSO_2\overset{R_1}{\overset{|}{N}}-(\overset{R_2}{\overset{|}{C}}H)_n-CH_2-A-COOH \qquad (XXIII)$$

wobei die Reste der Formeln (IVb), (XXXI), (XXXII), (XXIII) die gleiche Bedeutung wie in den Ansprüchen 1, 3 und 16 haben.

**18.** Verfahren zur Herstellung der Salze der chemischen Verbindungen nach den Ansprüchen 1 und 2, dadurch gekennzeichnet, daß man die Säure der allgemeinen Formel (I) mit $R_3$ = H mit einer stöchiometrischen Menge einer organischen oder mineralischen Base in einem protischen Lösungsmittel wie Wasser,

Methanol, Ethanol oder in einem Keton, wie Butanon oder Aceton, bei einer Temperatur zwischen 0°C und 60°C reagieren läßt.

**19.** Verfahren zur Herstellung der chemischen Verbindungen nach den Ansprüchen 1, 2 und 7, dadurch gekennzeichnet, daß man eine Verbindung der allgemeinen Formel (I) mit $R_1$ = COO-t-Bu, $R_3$ = verschieden von H, hydrolysiert, indem man die so erhaltene rohe Reaktionsmischung in eine konzentrierte wäßrige Lösung einer starken Säure und insbesondere Chlorwasserstoffsäure, gießt, auf eine Temperatur erwämt, die von 40 bis 100°C und insbesondere zwischen 50 und 70°C variiert, unter Bildung der Verbindung (I), worin $R_1$ = $R_3$ = H und worin die anderen Reste die gleiche Bedeutung wie in Anspruch 1 haben.

**20.** Als neue Arzneimittel, die insbesondere verwendbar sind für die Behandlung von Störungen des cardiovasculären Systems und insbesondere als Antiaggregationsmittel, antiischämisches Mittel im Myocard-, Cerebral- oder peripheren Bereich, als Antiasthma-Mittel, als Antiatheromatosemittel und schließlich als Antimigränemittel, Antikrebsmittel und Antivirenmittel, die Verbindungen, wie sie in einem der Ansprüche 1 und 2 definiert sind.

**21.** Pharmazeutische Zusammensetzungen, dadurch gekennzeichnet, daß sie als Wirkstoff (aktives Prinzip) mindestens eine Verbindung, wie sie in einem der Ansprüche 1 und 2 definiert ist, in Assoziation mit einem inerten pharmazeutischen Träger enthalten.

**Patentansprüche für folgende Vertragsstaaten : ES, GR**

**1.** Verfahren zur Herstellung von Sulfonamid-Derivaten von carbocyclischen oder benzoheterocyclischen Säuren der allgemeinen Formel (I)

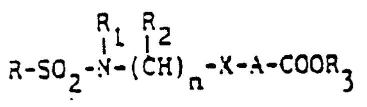

(I)

in der die Reste und Substituenten wie folgt definiert sind:

R steht für ein lineares oder verzweigtes leichtes Alkyl mit 1 bis 9 Kohlenstoffatomen, für Phenyl- oder Naphthylreste, die unsubstituiert sind oder substituiert sind durch eine oder mehrere der folgenden Gruppen: niederes Alkyl (mit 1 bis 4 C), Halogen, niederes Alkyloxy (mit 1 bis 4 C), Nitro, Amino, leichtes Dialkylamino (mit 1 bis 4 C) und Trifluoromethyl,

$R_1$ steht für ein Wasserstoffatom oder ein lineares oder verzweigtes niederes Alkyl (mit 1 bis 4 C) oder ein Benzyl;

$R_2$ steht für ein Wasserstoffatom oder ein lineares oder verzweigtes leichtes Alkyl (mit 1 bis 6 C), eine Phenylgruppe, die unsubstituiert ist oder substituiert ist durch ein Chlor oder ein Methoxyl, und eine Arylalkylgruppe mit 7 bis 9 Kohlenstoffatomen;

$R_3$ steht für ein Wasserstoffatom oder ein verzweigtes oder unverzweigtes niederes Alkyl (mit 1 bis 6 C);

-X- steht für einen divalenten funktionellen Rest, der ausgewählt wird aus:

$-CH_2-$ ; $-CH-OR_4$ ; -C=O ; $-C=N-OR_4$ ;

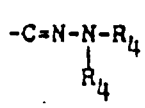

mit $R_4$=H,Me wobei -X- verschieden ist von $-CH_2-$, wenn -A- die nachstehend angegebenen divalenten Reste a, b, d und e darstellt;

-A- steht für einen benzocyclischen oder benzoheterocyclischen bivalenten Rest, der ausgewählt wird aus den folgenden Resten (a) bis (j) :

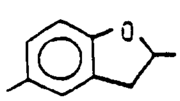 ; 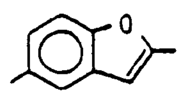 ; 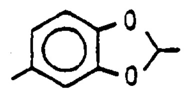 ; 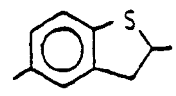

(a) (b) (c) (d)

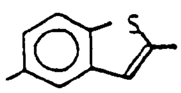 ; 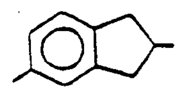 ; 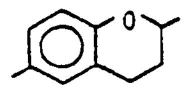 ; 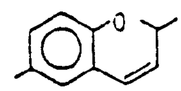

(e) (f) (g) (h)

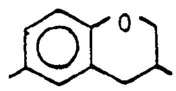 ; 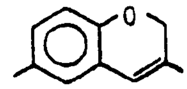

(i) (j)

und

n kann die Werte 1 bis einschließlich 4 annehmen,

der hydratisierten Formen der Säuren der allgemeinen Formel (I) sowie der therapeutisch akzeptablen organischen oder mineralischen Salze von (I) ($R_3$=H) und insbesondere eines Salzes, ausgewählt aus Natrium, Calcium, Zink, 2-Hydroxyethylammonium, Bis(2-hydroxyethyl)ammonium, Tris(2-hydroxyethyl)ammonium, Morpholinium und ihrer hydratisierten Form, wobei die Verbindungen der allgemeinen Formel (I) in Form einer racemischen Mischung vorliegen können oder isoliert sein können in Form von Enantiomeren, Diastereoisomeren oder ihrer Mischung in jedem Mengenverhältnis, dadurch gekennzeichnet, daß man die folgende Operation durchführt für A, das von b, e, h und j verschieden ist:

man läßt einen benzocyclischen oder benzoheterocyclischen Ester der Formel (II)

$$H\text{-}A\text{-}COOR_5 \qquad (II)$$

mit einem funktionellen Acylhalogenid mit der Struktur (III)

$$Y\text{-}(\overset{R_2}{\overset{|}{C}}H)_n\text{-}COY_1 \qquad (III)$$

unter Friedel-Crafts-Bedingungen reagieren unter Verwendung eines kompatiblen Lösungsmittels, das vorzugsweise chloriert ist, und insbesondere unter Verwendung von Methylenchlorid oder 1,2-Dichlorethan in Gegenwart einer Lewis-Säure wie Aluminiumchlorid, als nicht-beschränkendes Beispiel bei einer Temperatur, die von -5°C bis +50°C variiert, in der Weise, daß man die Verbindung (IVa) der Formel erhält:

$$Y\text{-}(\overset{R_2}{\overset{|}{C}}H)_n\text{-}CO\text{-}A\text{-}COOR_5 \qquad (IVa)$$

die selbst mit Natriumnitrid in einem Alkohol/Wasser-Gemisch bei einer Temperatur zwischen +10°C und +50°C kondensiert wird unter Bildung der Verbindung der Formel:

$$N_3\text{-}(\overset{R_2}{\overset{|}{C}}H)_n\text{-}CO\text{-}A\text{-}COOR_5 \qquad (V)$$

die selektiv reduziert wird mit Wasserstoff in einem leichten Alkohol, der eine wäßrige Lösung einer starken Säure, wie z.B. Chlorwasserstoffsäure, enthält, in Gegenwart eines auf einem inerten Träger abgeschiedenen Hydrierungskatalysators und insbesondere in Gegenwart von beispielsweise Palladium auf Kohle, unter Bildung bei einer Temperatur zwischen +10°C und +50°C des Aminoketoesterhydrochlorids der Formel (VI):

$$NH_2-(\overset{R_2}{\overset{|}{C}}H)_n-CO-A-COOR_5,\ HCl \qquad (VI)$$

das selbst kondensiert wird mit einem in geeigneter Weise substituierten Sulfonylhalogenid (-fluorid, -chlorid, -bromid) der Formel:

$R-SO_2Z$ (VII)

in einem organischen basischen Lösungsmittel, wie beispielsweise Pyridin, bei einer Temperatur zwischen -15°C und +40°C, unter Bildung der Verbindung (I) mit X = CO und $R_1$ = H, $R_3$ = verschieden von H, der Formel (VIII):

$$R-SO_2NH-(\overset{R_2}{\overset{|}{C}}H)_n-\overset{O}{\overset{\|}{C}}-A-COOR_5 \qquad (VIII)$$

wobei dieser Ester dann verseift wird mit einer alkalischen wäßrigen Lösung, wie einer Natriumcarbonatlösung oder Kaliumcarbonatlösung, in Gegenwart eines damit mischbaren organischen Lösungsmittels, wie eines Alkohols oder eines Äthers und insbesondere in Gegenwart von Methanol, Ethanol, Dioxan oder Tetrahydrofuran, bei einer Temperatur zwischen +10°C und dem Siedepunkt der Mischung, wobei man nach dem Ansäuern die Säure-Verbindung (I) mit X = CO, $R_1$ = H, $R_3$ = H der Formel (IX) erhält

$$R-SO_2NH-(\overset{R_2}{\overset{|}{C}}H)_n-\overset{O}{\overset{\|}{C}}-A-COOH \qquad (IX)$$

worin die Reste der allgemeinen Formeln (IVa) bis (IX) die gleichen Bedeutungen wie im Anspruch 1 haben und $R_5 = R_3$ ausgenommen H; Y und $Y_1$ Chlor oder Brom und Z Fluor oder Chlor oder Brom darstellen,

wobei man anschließend gegebenenfalls mindestens eine Umwandlung der oben erhaltenen Verbindungen der Formeln (I), (IV), (V) oder (VI) durchführt, insbesondere durch Reduktions- oder Kondensationsoperationen.

2. Verfahren nach Anspruch 1 dadurch gekennzeichnet, daß man eine Verbindung der Formel (V)

$$N_3-(\overset{R_2}{\overset{|}{C}}H)_n-CO-A-COOR_5 \qquad (V)$$

reduziert unter einer Wasserstoffatomosphäre in einem leichten Alkohol, der eine wäßrige Lösung einer starken Säure enthält, in Gegenwart eines Hydrierungskatalysators, der auf einem inerten Träger abgeschieden ist, und insbesondere in Gegenwart von beispielsweise Palladium auf Kohle, unter Bildung bei einer Temperatur zwischen +10°C und +60°C und während mehrerer Stunden - wobei man das Fortschreiten der Reaktion durch Chromatographie kontrolliert - der Verbindung der Formel (XII)

$$NH_2-(\overset{R_2}{\overset{|}{C}}H)_n-\overset{OH}{\overset{|}{C}}H-A-COOR_5 \qquad (XII)$$

die kondensiert wird mit einem in geeigneter Weise substituierten Sulfonylhalogenid (-fluorid, -chlorid, -bromid) der Formel:

$R-SO_2Z$

in einem organischen basischen Lösungsmittel, wie beispielsweise Pyridin, bei einer Temperatur zwischen -15°C und +40°C unter Bildung der Verbindung (I) mit X = CHOH und $R_1$ = H und $R_3$ = verschie-

den von H der Formel (XIII):

$$R\text{-}SO_2NH\text{-}(\overset{R_2}{\overset{|}{C}}H)_n\text{-}\overset{OH}{\overset{|}{C}}H\text{-}A\text{-}COOR_5 \qquad (XIII)$$

wobei schließlich dieser Ester verseift werden kann durch eine alkalische wäßrige Lösung, wie eine Natriumcarbonatoder Kaliumcarbonatlösung, in Gegenwart eines damit mischbaren alkoholischen oder etherischen organischen Lösungsmittels und insbesondere in Gegenwart von Methanol, Ethanol, Dioxan oder Tetrahydrofuran, bei einer Temperatur zwischen +10°C und +60°C, wobei nach dem Ansäuern die Säure-Verbindung (I) erhalten wird mit X = CHOH, $R_1$ = H, $R_3$ = H der Formel (XIV)

$$R\text{-}SO_2NH\text{-}(\overset{R_2}{\overset{|}{C}}H)_n\text{-}\overset{OH}{\overset{|}{C}}H\text{-}A\text{-}COOH \qquad (XIV)$$

worin die Reste der allgemeinen Formeln (V), (XII) bis (XIV) die gleiche Bedeutung wie im Anspruch 1 haben.

**3.** Verfahren nach Anspruch 1, dadurch gekennzeichnet, daß man eine Verbindung der Formel (VI)

$$HCl\text{-}NH_2\text{-}(\overset{R_2}{\overset{|}{C}}H)_n\text{-}\overset{O}{\overset{\|}{C}}\text{-}A\text{-}COOR_5 \qquad (VI)$$

unter einem Wasserstoffüberdruck reduziert in Gegenwart einer starken Säure, wie Schwefelsäure oder Perchlorsäure, unter Verwendung eines Hydrierungskatalysators, wie Palladium auf Kohle, in einer Carbonsäure als Lösungsmittel und insbesondere in Essigsäure, wobei man bei einer Temperatur zwischen 20 und 90°C unter einem Wasserstoffdruck zwischen 0,1 und 5 Atmosphären arbeitet, oder indem man entweder die Verbindung (VI) mit einem Trialkylsilan und insbesondere mit Triethylsilan in Gegenwart von Trifluoressigsäure bei einer Temperatur zwischen +10°C und +40°C reduziert oder indem man die Verbindung (VI) mit naszierendem Wasserstoff reduziert, der gebildet wird durch Einwirkung einer Säure (Chlorwasserstoffsäure oder Essigsäure) auf amalgamiertes oder nicht-amalgamiertes Zink unter Erwärmen auf eine Temperatur zwischen 40 und 120°C in einem organischen Lösungsmittel, wie beispielsweise Toluol, unter Bildung der Verbindung (XV) in Form einer Base oder in Form eines Salzes der Formel:

$$NH_2\text{-}(\overset{R_2}{\overset{|}{C}}H)_n\text{-}CH_2\text{-}A\text{-}COOR_5 \qquad (XV)$$

die anschließend kondensiert wird mit einem Sulfonylhalogenid, das definiert ist wie und unter den gleichen Bedingungen wie in den Ansprüchen 3 und 4 angegeben, unter Bildung der Verbindung (I) mit $R_1$ = H und X = $CH_2$ der Formel (XVI), die das gesättigte Homologe von (VIII) und (XIII) ist

$$RSO_2NH\text{-}(\overset{R_2}{\overset{|}{C}}H)_n\text{-}CH_2\text{-}A\text{-}COOR_5 \qquad (XVI)$$

die verseift wird nach dem Verfahren, wie es in den Ansprüchen 3 und 4 zur Herstellung der Derivate (IX) und (XIV) beschrieben ist, unter Bildung der Verbindung (I) mit $R_1$ = $R_3$ = H und X = $CH_2$ der Formel (XVII)

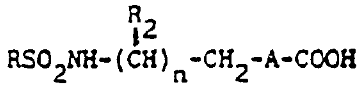

(XVII)

wobei die Reste der allgemeinen Formeln (VI), (XV) bis (XVII) die gleiche Bedeutung wie im Anspruch 1 haben.

4. Verfahren nach Anspruch 1, dadurch gekennzeichnet, daß man eine Verbindung der Formel (IVa) reduziert

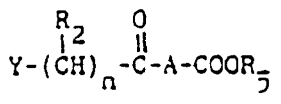

(IVa)

nach einer der Methoden des Anspruchs 3 unter Bildung der halogenierten Verbindung, die äquivalent zu (XV) ist und die Formel (XIX) hat

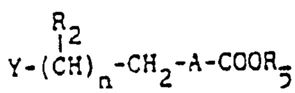

(XIX)

die kondensiert wird mit dem in situ hergestellten Natriumsalz eines in geeigneter Weise substituierten sekundären Sulfonamids der Formel (XX)

$R\text{-}SO_2NH\text{-}R_7$ (XX)

in einem organischen Lösungsmittel und insbesondere in Dimethylformamid, Dimethylacetamid, Aceton, Butanon, Tetrahydrofuran oder Dioxan, bei einer Temperatur zwischen +5°C und +60°C unter Bildung der Verbindung der Formel (XXI)

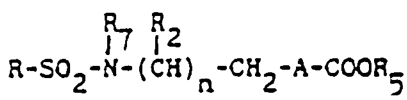

(XXI)

wobei dieses Derivat anschließend nach dem in den Ansprüchen 3 und 4 in bezug auf die Derivate (IX) und (XIV) beschriebenen Verfahren verseift wird unter Bildung der äquivalenten gesättigten Verbindung (I) mit $X = CH_2$ und $R_3 = H$ der Formel (XXII)

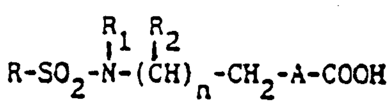

(XXII)

wobei die Reste der Verbindungen der Formeln (IVa), (XIX) bis (XXII) die gleiche Bedeutung wie im Anspruch 1 haben und wobei $R_7$ entweder den Rest $R_1$, der von Wasserstoff verschieden ist, oder den Rest $R_6COO$- darstellt, worin $R_6$ Benzyl oder ein verzweigtes oder unverzweigtes leichtes Alkyl mit 1 bis einschließlich 6 Kohlenstoffatomen wie beispielsweise, jedoch nicht beschränkend, Methyl-, Ethyl-, tert-Butylreste bedeutet.

5. Verfahren nach Anspruch 1, dadurch gekennzeichnet, daß man eine nach Anspruch 1 hergestellte Verbindung der Formel (IVa) mit dem Natriumsalz der Verbindung (XX) kondensiert

$R\text{-}SO_2NHR$ (XX)

die nach dem gleichen Verfahren wie für die Herstellung der Verbindung (XXI) in Anspruch 4 angegeben in situ hergestellt worden ist, unter Bildung des Ketosulfamidoesters (I) mit X = CO der Formel (XXIV)

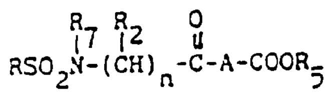 (XXIV)

der verseift wird entweder direkt für den Fall, daß $R_7$ von $COOR_6$ verschieden ist, nach dem für die Verbindungen (VIII) und (XIII) in den Ansprüchen 1 und 2 beschriebenen Verfahren, wobei hier erhalten wird die Verbindung (I) mit X = CO, $R_3$ = H und $R_1$ = verschieden von H der Formel (XXV); oder der für den Fall, daß $R_7$ = COO-t-Bu, an erster Stelle einer Abspaltung der Gruppe $R_7$ in einem stark sauren Milieu und insbesondere in Chlorwasserstoffsäure, gelöst in einem organischen Lösungsmittel, wie beispielsweise Ethylacetat, unterworfen wird unter Bildung der Verbindung (XXV) mit $R_1$ = H und $R_5$ = $R_3$, das von H verschieden ist,
die anschließend wie oben in einer Säure verseift werden kann unter Bildung der Verbindung (I) mit $R_1$ = $R_3$ = H, X = CO der Formel (XXV)

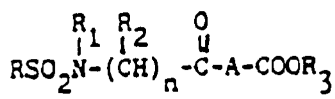 (XXV)

worin die Reste der Formeln der Verbindungen (IVa), (XX), (XXIV), (XXV) die gleichen Bedeutungen wie in den Ansprüchen 1 und 4 haben.

**6.** Verfahren nach Anspruch 1, dadurch gekennzeichnet, daß man ein nach Anspruch 5 hergestelltes Derivat mit der allgemeinen Struktur (XXIV) reduziert

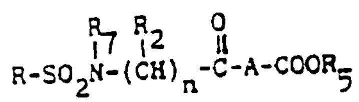 (XXIV)

nach einer der drei Methoden, wie sie im Anspruch 3 zur Herstellung der Verbindung (XV) beschrieben sind, wobei man hier das Sulfonamido-Homologe der im Anspruch 4 bereits beschriebenen Formel (XXI) erhält

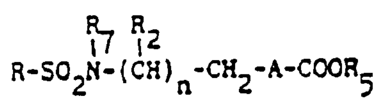 (XXI)

und zu einer entsprechenden Säure (I) mit X = $CH_2$ und $R_3$ = H der Formel (XXII) verseift nach dem in Anspruch 4 bereits beschriebenen Verfahren, wobei die Reste und Variablen der Formeln (XXIV) und (XXI) die gleichen Bedeutungen wie in den Ansprüchen 1 und 4 haben.

**7.** Verfahren nach Anspruch 1, dadurch gekennzeichnet, daß man eine Verbindung der allgemeinen Formel (I) mit X = CO reduziert nach einem der in Anspruch 3 beschriebenen Verfahren unter Bildung des homologen reduzierten Sulfonamids (I) mit X = $CH_2$.

**8.** Verfahren nach Anspruch 1, dadurch gekennzeichnet, daß man eine Verbindung der allgemeinen Formel (I), worin X = CO, reduziert in Gegenwart von Natrium- oder Kaliumborhydrid in einem protischen Lösungsmittel, wie einem leichten Alkohol, vorzugsweise mit der gleichen Anzahl von Kohlenstoffatomen wie $R_3$, wenn $R_3$ von H verschieden ist (um eine Umesterung zu vermeiden), bei einer Temperatur zwischen +5°C und +50°C unter Bildung der Verbindungen der allgemeinen Formel (I) mit X = CHOH.

**9.** Verfahren nach Anspruch 1, dadurch gekennzeichnet, daß man eine Verbindung der Formel (I) mit $R_3$ = H reagieren läßt mit einem Alkohol der allgemeinen Formel $R_3OH$ in Gegenwart eines Säure-Katalysators wie Schwefelsäure oder p-Toluolsulfonsäure, indem man auf eine Temperatur zwischen 40°C und 110°C erwärmt unter Bildung der Verbindung der allgemeinen Formel (I) mit $R_3$ = verschieden von H.

**10.** Verfahren nach Anspruch 1, dadurch gekennzeichnet, daß man eine Verbindung der allgemeinen Formel (I), worin X = CO und $R_3$ = H, kondensiert mit einem substituierten oder unsubstituierten Hydroxylamin-hydrochlorid der Formel

$$R_4ONH_2$$

unter Bildung in Pyridin bei einer Temperatur zwischen 10°C und 50°C der entsprechenden Oxime (I) mit X = C=N=$OR_4$ und $R_3$ = H, wobei $R_4$ wie in Anspruch 1 definiert ist.

**11.** Verfahren nach Anspruch 1, dadurch gekennzeichnet, daß man ein Derivat der allgemeinen Formel (I), worin X = CO und $R_3$ = H, kondensiert mit einem Hydrazin der allgemeinen Formel

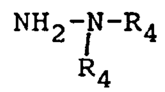

unter Bildung in einem protischen organischen Lösungsmittel wie Ethanol in Gegenwart einer organischen Säure wie beispielsweise Essigsäure bei einer Temperatur zwischen 25 und 120°C der entsprechenden Hydrazone (I) mit

X = 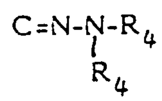

und $R_3$ = H, worin $R_4$ wie in Anspruch 1 definiert ist.

**12.** Verfahren nach Anspruch 1, dadurch gekennzeichnet, daß man einen Ester der allgemeinen Formel (I), worin der bicyclische Rest -A- an dem Cyclus, der an die Carboxylatfunktion gebunden ist, gesättigt ist (d.h. worin A = a, d, g, i), reagieren läßt mit einem Halogenimid und insbesondere mit N-Brom- oder N-Chlorsuccinimid in einem halogenierten Lösungsmittel und insbesondere zwischen +20°C und +80°C unter Bildung der entsprechenden ungesättigten Verbindungen der allgemeinen Formel (I) (d.h. worin A = b, e, h) mit $R_3$ = verschieden von H, die anschließend verseift werden zu ihren Vorläufer-Säuren der allgemeinen Formel (I) mit $R_3$ = H nach der in Anspruch 1 beschriebenen Vorschrift.

**13.** Verfahren nach Anspruch 1, dadurch gekennzeichnet, daß man eine Verbindung der allgemeinen Formel (I), worin $R_1$ = H, reagieren läßt mit einem aktivierten Derivat der Formel

$$(R_1)_2SO_4 \text{ oder } R_1Z$$

mit Z = Br oder J in Gegenwart einer starken Base wie Natriumhydrid oder eines Carbonats oder eines Hydroxids von Natrium oder Kalium in einem Lösungsmittel wie DMF, Dimethylacetamid, Butanon, Aceton und bei einer Temperatur zwischen +20°C und +70°C unter Bildung der äquivalenten Verbindung der allgemeinen Formel (I), in der $R_1$ und $R_3$ von H verschieden sind und in der die variablen Reste wie in Anspruch 1 definiert sind.

**14.** Verfahren nach Anspruch 1 mit A = verschieden von b, e, h und j, dadurch gekennzeichnet, daß man einen benzocarbocyclischen oder benzoheterocyclischen Ester der allgemeinen Formel (II) reagieren läßt mit einem funktionellen Acylhalogenid der Struktur (IIIa)

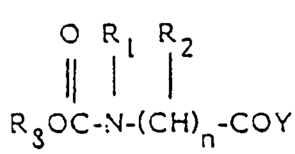

(IIIa)

unter den Friedel-Crafts-Bedingungen unter Verwendung eines kompatiblen, vorzugsweise chlorierten Lösungsmittels, insbesondere unter Verwendung von Methylenchlorid oder 1,2-Dichlorethan, in Gegenwart einer Lewis-Säure, wie beispielsweise, jedoch nicht beschränkend, Aluminiumchlorid bei einer Temperatur zwischen -5°C und +50°C unter Bildung der Verbindung (IVb) der Formel:

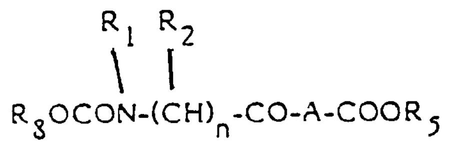

(IVb)

die selbst mit einem Alkaliborhydrid reduziert wird nach dem in Anspruch 8 beschriebenen Verfahren unter Bildung des Derivats (XXIX) der Formel:

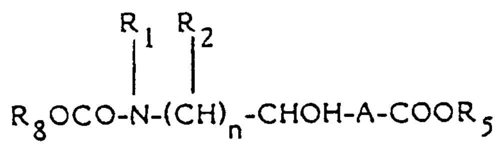

(XXIX)

die durch basische Hydrolyse zu dem Natriumsalz der Aminohydroxysäure der Formel (XXX) führt nach der in Anspruch 1 beschriebenen Methode zur Herstellung der Verbindung (IX)

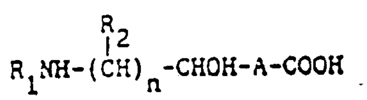

(XXX)

und wobei man schließlich ein in geeigneter Weise substituiertes Sulfonylhalogenid der Formel

$RSO_2Z$

kondensiert mit dem oben erhaltenen rohen Natriumsalz (XXX) in der gleichen wäßrigen organischen Phase bei einem pH-Wert zwischen 11 und 13 und bei einer Temperatur zwischen 0 und +40°C unter Bildung der Verbindung (I) mit $R_3$ = H und Y = CHOH der Formel (XXVII)

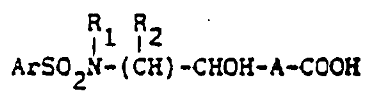

(XXVII)

in der die Reste der allgemeinen Formeln IVb, XXIX, XXX, XXVII die gleichen Bedeutungen wie in Anspruch 1 haben und $R_8$ einen leichten linearen Alkylrest mit 1 bis einschließlich 6 Kohlenstoffatomen bedeutet.

**15.** Verfahren nach Anspruch 1, dadurch gekennzeichnet, daß man nach einem der im Anspruch 3 beschriebenen Verfahren das Keton einer Verbindung der allgemeinen Formel (IVb), die wie im Anspruch 14 angegeben hergestellt worden ist

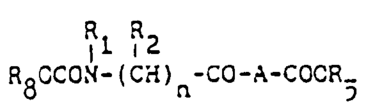

(IVb)

an der Methylengruppe reduziert unter Bildung des Derivats (XXXI) der Formel

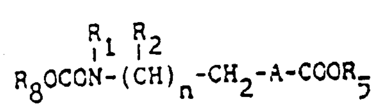

(XXXI)

das anschließend nach dem in Anspruch 14 beschriebenen Verfahren verseift wird unter Bildung des Natriumsalzes der Aminosäure der Formel (XXXII)

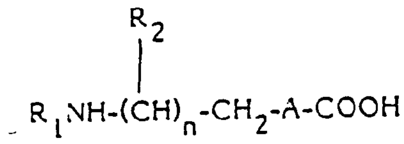

(XXXII)

das, ohne isoliert zu werden, mit dem Sulfonylhalogenid der Formel

$RSO_2Z$

kondensiert wird nach dem im Anspruch 14 beschriebenen Verfahren, wobei man nach dem Ansäuern erhält die Verbindung (I) mit X = $CH_2$ und $R_3$ = H der Formel (XXIII)

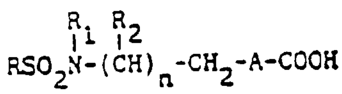

(XXIII)

wobei die Reste der Formeln (IVb, (XXXI), (XXXII), (XXIII) die gleiche Bedeutung wie in den Ansprüchen 1 und 14 haben.

16. Verfahren nach Anspruch 1, dadurch gekennzeichnet, daß man die Säure der allgemeinen Formel (I) mit $R_3$ = H reagieren läßt mit einer stöchiometrischen Menge einer organischen oder mineralischen Base in einem protischen Lösungsmittel wie Wasser, Methanol, Ethanol oder in einem Keton, wie Butanon oder Aceton, bei einer Temperatur zwischen 0°C und +60°C.

17. Verfahren nach den Ansprüchen 1 und 5, dadurch gekennzeichnet, daß man eine Verbindung der allgemeinen Formel (I) mit $R_4$ = COO-t-Bu, $R_3$ = verschieden von H, hydrolysiert, indem man das so erhaltene rohe Reaktionsgemisch in eine konzentrierte wäßrige Lösung einer starken Säure und insbesondere Chlorwasserstoffsäure gießt, wobei man auf eine Temperatur erwärmt, die zwischen 40 und 100°C und insbesondere zwischen 50 und 70°C variiert unter Bildung einer Verbindung (I), in der $R_1$ = $R_3$ = H und in der die anderen Reste die gleiche Bedeutung wie in Anspruch 1 haben.

## Claims

### Claims for the following Contracting States : AT, BE, CH, LI, DE, DK, FR, GB, IT, LU, NL, SE

1. New sulphonamide derivatives of carbocyclic or benzo-heterocyclic acids of general formula I

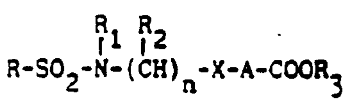

I

in which the radicals and substituents are defined as follows:

R represents a straight-chain or branched lower alkyl containing 1 to 9 carbon atoms, or phenyl or naphthyl radicals which are unsubstituted or substituted by one or more groups: lower alkyl (1 to 4 C), halogeno, lower alkoxy (1 to 4 C), nitro, amino, lower dialkylamino (1 to 4C), trifluoromethyl ;

$R_1$ represents hydrogen or a straight-chain or branched lower alkyl (1 to 4 C) or benzyl;

$R_2$ represents hydrogen or a straight-chain or branched lower alkyl (1 to 6 C), a phenyl group which is unsubstituted or substituted by a chlorine or a methoxy and an aralkyl group containing from 7 to 9 carbon atoms;

$R_3$ represents hydrogen or a straight-chain or branched lower alkyl (1 to 6 C);

-X- represents a divalent functional radical chosen from the following:

$-CH_2-$;

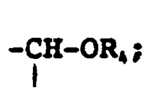 ; 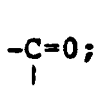 ; 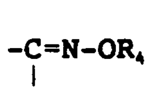 ; 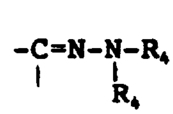

where $R_4$=H,Me with the proviso, however, that X differs from $-CH_2-$ when -A- represents divalent radicals a, b, d and e below, -A- represents a benzo-cyclic or benzo-heterocyclic divalent radical chosen from the following: (a-j)

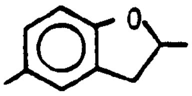 ; 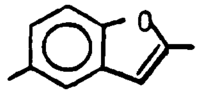 ; 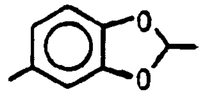 ; 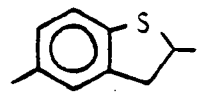 ;

(a) (b) (c) (d)

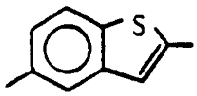 ; 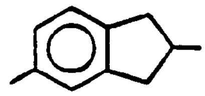 ; 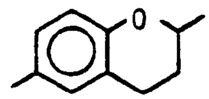 ; 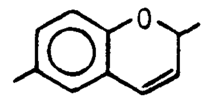

(e) (f) (g) (h)

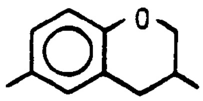 ; 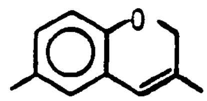

(i) (j)

and n can assume the values 1 to 4 inclusive,
the hydrated forms of acids of general formula I and the therapeutically acceptable organic or inorganic salts of I ($R_3$=H) and in particular a salt chosen from: sodium, calcium, zinc, 2-hydroxyethylammonium, bis(2-hydroxyethyl)ammonium, tris(2-hydroxyethyl)ammonium, morpholinium and their hydrated form,
it being possible for the compounds of general formula I to be in the form of a racemic mixture or to be isolated in the form of enantiomers, diastereoisomers or their mixture in any proportions.

**2.** Compound according to Claim 1, characterized in that it is chosen from the following compounds:
.5-(parachlorobenzenesulphonamidoacetyl)-2-indane-carboxylic acid
.lysine 5-(parachlorobenbenesulphnonamidoacetyl)-2-indanecarboxylate
-5-(parachlorobenzenesulphonamidoacetyl)-2,3-dihydro-2-benzofurancarboxylic acid
-5-(2-parachlorobenzenesulphonamido-1-hydroxyethyl)-2,3-dihydro-2-benzofurancarboxylic acid
-5-(2-parachlorobenzenesulphonamido-1-hydroxyiminoethyl)-2,3-dihydro-2-benzofurancarboxylic acid
-5-(2-parachlorobenzenesulphonamido-1-methoxyiminoethyl)-2,3-dihydro-2-benzofurancarboxylic acid
-5-(N-methyl-parachlorobenzenesulphonamidoacetyl)-2,3-dihydro-2-benzofurancarboxylic acid
-5-(α-methyl-parachlorobenzenesulphonamidoacetyl)-2,3-dihydro-2-benzofurancarboxylic acid
-5-(2-parachlorobenzenesulphonamido-1-hydroxypropyl)-2,3-dihydro-2-benzofurancarboxylic acid
-ethyl-5-(parachlorobenzenesulphonamidoacetyl)-2,3-dihydro -2-benzofurancarboxylate
-5-(2-parachlorobenzenesulphonamidoethyl)-1,3-benzodioxole-2-carboxylic acid
-5-(parachlorobenzenesulphonamidoethyl)-2-indanecarboxylic acid
-6-(2-parachlorobenzenesulphonamidoethyl)-2-chromancarboxylic acid
-6-(parachlorobenzenesulphonamidoacetyl)-2-chromancarboxylic acid
-ethyl 6-(parachlorobenzenesulphonamidoacetyl)-2-chromancarboxylate
-5-(benzenesulphonamidoacetyl)-2,3-dihydro-2-benzofurancarboxylic acid
-5-(paramethoxybenzenesulphonamidoacetyl)-2,3-dihydro -2-benzofurancarboxylic acid
-5-(pentafluorobenzenesulphonamidoacetyl)-2,3-dihydro-2-benzofurancarboxylic acid
-lysine 5-(parachlorobenzenesulphonamidoacetyl)-2,3-dihydro-2-benzofurancarboxylate
-lysine 6-(2-parachlorobenzenesulphonamidoethyl)-2-chromancarboxylate
-lysine 6-(parachlorobenzenesulphonamidoacetyl)-2-chromancarboxylate.

**3.** Process for the preparation of chemical compounds according to Claims 1 and 2 where A differs from b, e, h, and j, characterized in that a benzo-cyclic or benzo-heterocyclic ester of formula II

$H-A-COOR_5$ II

is reacted with a functional acyl halide of structure III

$$Y-(\overset{R_2}{\overset{|}{C}}H)_n-COY_1 \qquad \underline{III}$$

under Friedel-Crafts conditions using a compatible solvent, preferably chlorinated and more precisely methylene chloride or 1,2-dichloroethane, in the presence of a Lewis acid such as aluminium chloride by way of non-limiting example, and at a temperature varying from -5°C to +50°C so as to obtain the compound IVa of formula

$$Y-(\overset{R_2}{\overset{|}{C}}H)_n-CO-A-COOR_5 \qquad \underline{IVa}$$

which itself is subjected to a condensation reaction with sodium nitride in an aqueous-alcoholic mixture at a temperature of between +10°C and +50°C to give the compound of formula:

$$N_3-(\overset{R_2}{\overset{|}{C}}H)_n-CO-A-COOR_5 \qquad \underline{V}$$

which is selectively reduced by hydrogen in a lower alcohol containing an aqueous solution of a strong acid - hydrochloric for example - in the presence of a hydrogenation catalyst deposited on an inert support and more precisely palladium-on-charcoal for example, to give, at a temperature of between +10°C and +50°C, the aminoketoester hydrochloride of formula VI:

$$NH_2-(\overset{R_2}{\overset{|}{C}}H)_n-CO-A-COOR_5,\ HCl \qquad \underline{VI}$$

which is itself subjected to a condensation reaction with a suitably substituted sulphonyl halide (fluoride, chloride, bromide) of formula:

$R-SO_2Z$ VII

in a basic organic solvent such as pyridine for example, at a temperature of between -15°C and +40°C, to give the compound I where X=CO and $R_1$=H, and $R_3$ differs from H, of formula VIII:

$$R-SO_2NH-(\overset{R_2}{\overset{|}{C}}H)_n-\overset{O}{\overset{||}{C}}-A-COOR_5 \qquad \underline{VIII}$$

and, finally, this ester is saponified with an aqueous alkaline solution such as sodium hydroxide solution or potassium hydroxide solution in the presence of a miscible organic solvent such as an alcohol or an ether and more precisely methanol, ethanol, dioxane or tetrahydrofuran, at a temperature of between +10°C and the boiling point of the fixture, to give, after acidification, the acid compound I where X=CO, $R_1$=H and $R_3$=H, of formula IX

$$R-SO_2NH-(\overset{R_2}{\overset{|}{C}}H)_n-\overset{O}{\overset{||}{C}}-A-COOH \qquad \underline{IX}$$

the radicals in the general formulae of IVa to IX having the same meaning as in Claim 1 and $R_5$=$R_3$ with the exception of H; Y and $Y_1$ represent a chlorine or a bromine and Z represents a fluorine or a chlorine or a bromine.

4. Process for the preparation of chemical compounds according to Claims 1 and 2 and characterized in that a compound of formula V, prepared according to Claim 3,

$$N_3-(\overset{R_2}{\overset{|}{C}}H)_n-CO-A-COOR_5 \qquad \underline{V}$$

is reduced under an atmosphere of hydrogen in a lower alcohol containing an aqueous solution of a strong acid in the presence of a hydrogenation catalyst deposited on an inert support and more precisely palladium-on-charcoal for example, to give, at a temperature of between +10°C and +60°C and in the course of several hours - monitoring the progress of the reaction by chromatography - the compound of formula XII

$$NH_2-(\overset{R_2}{\overset{|}{C}}H)_n-\overset{OH}{\overset{|}{C}}H-A-COOR_5 \qquad \underline{XII}$$

which is subjected to a condensation reaction with a suitably substituted sulphonyl halide (fluoride, chloride, bromide) of formula:

$$R-SO_2Z$$

in a basic organic solvent such as pyridine for example, at a temperature of between -15°C and +40°C, to give compound I where X=CHOH and $R_1$=H and $R_3$ differs from H, of formula XIII

$$R-SO_2NH-(\overset{R_2}{\overset{|}{C}}H)_n-\overset{OH}{\overset{|}{C}}H-A-COOR_5 \qquad \underline{XIII}$$

and finally thin ester can be saponified with an aqueous alkaline solution such an sodium hydroxide solution or potassium hydroxide solution in the presence of an alcoholic or ethereal miscible organic solvent and more precisely methanol, ethanol, dioxane or tetrahydrofuran, at a temperature of between +10°C and +60°C to give, after acidification, the acid compound I where X=CHOH, $R_1$=H and $R_3$=H, of formula XIV

$$R-SO_2NH-(\overset{R_2}{\overset{|}{C}}H)_n-\overset{OH}{\overset{|}{C}}H-A-COOH \qquad \underline{XIV}$$

the radicals in general formulae V and XII to XIV having the same meaning as in Claims 1 and 3.

5. Process for the preparation of chemical compounds according to Claims 1 and 2 and characterized in that a compound of formula VI, prepared according to Claim 3,

$$HCl-NH_2-(\overset{R_2}{\overset{|}{C}}H)_n-\overset{O}{\overset{\|}{C}}-A-COOR_5 \qquad \underline{VI}$$

is reduced either under excess hydrogen pressure in the presence of a strong acid such as sulphuric acid or perchloric acid using a hydrogenation catalyst such aspalladium-on-charcoal in a carboxylic acid as the solvent and more particularly acetic acid, carrying out the reaction at a temperature of between 20 and 90°C under a hydrogen pressure of between 0.1 and 5 atmospheres; or by reducing compound VI with a trialkylsilane and more precisely triethylsilane in the presence of trifluoroacetic acid at a temperature of between +10 and +40°C, or by reducing compound VI with nascent hydrogen produced by the action of an acid (hydrochloric or acetic) on zinc, which may be in the form of zinc amalgam, by heating at between 40 and 120°C in an organic solvent such as toluene for example to give the compound XV in the form of the base or the salt of formula:

$$NH_2-(\overset{R_2}{\overset{|}{C}}H)_n-CH_2-A-COOR_5 \qquad \underline{XV}$$

which is then subjected to a condensation reaction with a defined sulphonyl halide and under the

same conditions as those described in Claims 3 and 4, to give the compound I where $R_1$=H and X=$CH_2$, of formula XVI, a saturated homologue of VIII and XIII

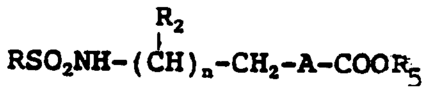 XVI

which is saponified by the process described in Claims 3 and 4 to prepare the derivatives IX and XIV, to give the compound I where $R_1$=$R_3$=H and X=$CH_2$, of formula XVII

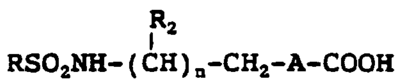 XVII

where the radicals in general formulae VI and XV to XVII have the same meaning as in Claims 1 and 3.

6. Process for the preparation of chemical compounds defined according to Claims 1 and 2, characterized in that a compound of formula IVa, prepared according to Claim 3,

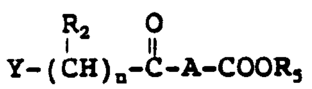 IVa

is reduced by one of the methods of Claim 5 to give the halogenated compound equivalent to XV having the formula XIX

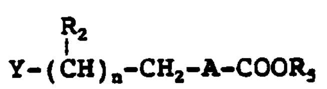 XIX

which is subjected to a condensation reaction with the sodium salt, prepared in situ, of a suitably substituted secondary sulphonamide of formula XX

$R-SO_2NH-R_7$ XX

in an organic solvent and more particularly dimethylformamide, dimethylacetamide, acetone, butanone, tetrahydrofuran or dioxane, at a temperature of between +5°C and +60°C to give the compound of formula XXI

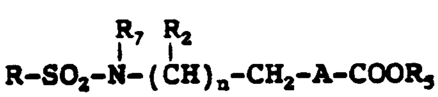 XXI

which derivative is then saponified by the process described in Claims 3 and 4 with respect to derivatives IX and XIV, to give the equivalent saturated compound I where X=$CH_2$ and $R_3$=H, of formula XXII

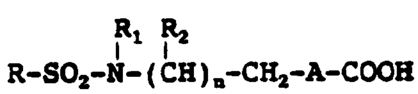 XXII

in which the radicals in the compounds of formulae IVa and XIX to XXII have the same meaning as in Claims 1 to 3 and where $R_7$ represents either the radical $R_1$ other than hydrogen or the radical $R_6COO$-, where $R_6$ represents a benzyl or a straight-chain or branched lower alkyl containing from 1 to 6 carbon atoms inclusive, and by way or non-limiting example the radicals: methyl, ethyl, tert.-butyl.

7. Process for the preparation of chemical compounds defined according to Claims 1 and 2, characterized in that a compound of formula IVa, prepared according to Claim 3, is subjected to a condensation reaction with the sodium salt of compound XX,

$R-SO_2NHR$ XX

prepared in situ by the same process as to prepare compound XXI according to Claim 6, to give the ketosulphamidoester I where X=CO, of formula XXIV

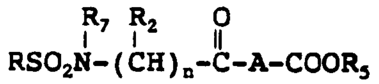

XXIV

which is saponified either directly in the case where $R_7$ differs from $COOR_6$ according to the process described for the compounds VIII and XIII according to Claims 3 and 4, to give, in this case, the compound I where X=CO, $R_3$=H and $R_1$ differs from H, of formula XXV; or in the case where $R_7$=COO-tBu is first subjected to cleavage of the group $R_7$ in a strong acid medium and more precisely hydrochloric acid dissolved in an organic solvent such as ethyl acetate for example, to give the compound XXV where $R_1$=H and $R_5$=$R_3$ and differs from H, which can then be saponified to the acid as above, to give the compound I where $R_1$=$R_3$=H and X=CO, of formula XXV

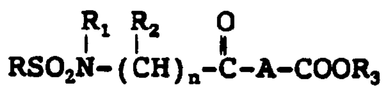

XXV

where the radicals in the formulae of compounds IVa, XX, XXIV and XXV have the same meaning as in Claims 1, 3 and 6.

8. Process for the preparation of chemical compounds defined according to Claims 1 and 2 and characterized in that a derivative of general structure XXIV, prepared according to Claim 7,

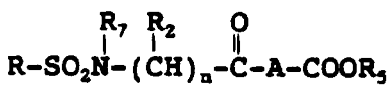

XXIV

is reduced by one of the three methods described in Claim 5 for preparation of the compound XV, to give, in this case, the sulphonamido homologue of formula XXI, already described in Claim 6

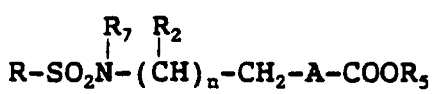

XXI

and saponified to the corresponding acid I where X=$CH_2$ and $R_3$=H, of formula XXII, according to the process already described in Claim 6, and where the radicals and variables in formulae XXIV and XXI have the same meaning as in Claims 1, 3 and 6.

9. Process for the preparation of chemical compounds defined according to Claims 1 and 2 and characterized in that a compound of general formula I where X=co is reduced to give, by one of the processes described in Claim 5, the reduced sulphonamide homologue of formula I where X=$CH_2$.

10. Process for the preparation of chemical compounds defined according to Claims 1 and 2, and characterized in that a compound of general formula I where X=co is reduced in the presence of sodium borohydride or potassium borohydride in a protic solvent such as lower alcohol, preferably of the same condensation with respect to carbon as $R_3$ when $R_3$ differs from H (so as to prevent transesterification), at a temperature of between +5°C and +50°C, to give the compounds of general formula I where X=CHOH.

11. Process for the preparation of chemical compounds according to Claims 1 and 2 and characterized in that a compound or formula I where $R_3$=H is reacted with an alcohol of general formula $R_3OH$ in the presence of an acid catalyst such as sulphuric acid or paratoluenesulphonic acid, heating at a temperature of between 40°C and 110°C, to give the compounds of general formula I where $R_3$ differs from H.

12. Process for the preparation of chemical compounds according to Claims 1 and 2 and characterized in that a compound of general formula I where X=CO and $R_3$=H is subjected to a condensation reaction with an

unsubstituted or substituted hydroxylamine hydrochloride of formula $R_4ONH_2$, to give, in pyridine at a temperature of between 10°C and 50°C, the corresponding oximes I where X= C=N-$OR_4$ and $R_3$=H, where $R_4$ is defined as in Claim 1.

**13.** Process for the preparation of chemical compounds according to Claims 1 and 2 and characterized in that a derivative of general formula I where X=CO and $R_3$=H is subjected to a condensation reaction with a hydrazine of general formula

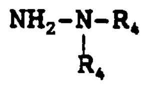

to give, in a protic organic solvent such as ethanol in the presence of an organic acid such as acetic acid for example, at a temperature of between 25°C and 120°C, the corresponding hydrazones I where

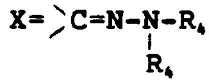

and $R_3$=H, where $R_4$ is defined as in Claim 1.

**14.** Process for the preparation of chemical compounds according to Claims 1 and 2 and characterized in that an ester of general formula I where the bicyclic radical -A- is saturated on the cycle attached to the carboxylate function (that is to say where A = a, d, g or i) is reacted with a halogenoimide and more particularly N-bromo- or N-chlorosuccinimide in a halogenated solvent and more precisely carbon tetrachloride at a temperature of between +20°C and +80°C to give the corresponding unsaturated compounds of general formula I (that is to say where A = b, e or h) where $R_3$ differs from H, which compounds are then saponified to their precursor acids of general formula I where $R_3$=H using the method described in Claim 3.

**15.** Process for the preparation of chemical compounds according to Claims 1 and 2 and characterized in that a compound of general formula I in which $R_1$=H is reacted with an activated derivative of formula $(R_1)_2SO_4$ or $R_1Z$, where Z=Br or I, in the presence of a strong base such as sodium hydride or a sodium carbonate or hydroxide or a potassium carbonate or hydroxide, in a solvent such as DMF, dimethylacetamide, butanone or acetone and at a temperature of between +20°C and +70°C to give the equivalent compound of general formula I in which $R_1$ and $R_3$ differ from H and where the variable radicals are defined as in Claim 1.

**16.** Process for the preparation of chemical compounds according to Claims 1 and 2 where A differs from b, e, h and j, characterized in that a benzo-carbocyclic or benzo-heterocyclic ester of general formula II

H-A-$COOR_5$ II

is reacted with a functional acyl halide of structure IIIa

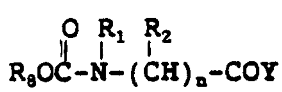

IIIa

under Friedel-Crafts conditions using a compatible solvent, preferably chlorinated and more precisely methylene chloride or 1,2-dichloromethane, in the presence of a Lewis acid, such as aluminium chloride by way of non-limiting example, and at a temperature of between -5°C and +50°C so as to obtain compound IVb of formula

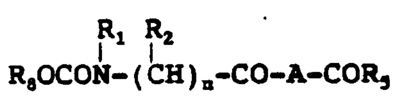

IVb

which is itself reduced by an alkaline metal borohydride by the process described in Claim 10 so is to obtain the derivative is of formula

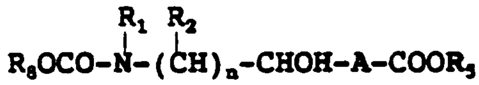 XXIX

to lead, by basic hydrolysis, to the sodium salt of the aminohydroxy acid of formula XXX by the method described in Claim 3 for the preparation or compound IX

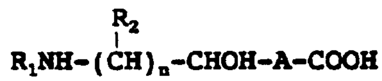 XXX

and finally, by subjecting a suitably substituted sulphonyl halide of formula $RSO_2Z$ to a condensation reaction with the crude sodium salt of XXX obtained above in the same aqueous-organic phase at a pH or between 11 and 13 and at a temperature of between 0 and +40°C, to give the compound I where $R_3$=H and Y=CHOH, of formula XXVII

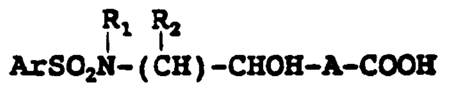 XXVII

in which the radicals in general formulae IVb, XXIX, XXX and XXVII have the same meaning as in Claims 1 and 3 and $R_8$ represents a lower straight-chain alkyl radical having 1 to 6 carbon atoms inclusive.

**17.** Process for the preparation of chemical compounds according to Claims 1 and 2 and characterized in that the ketone of a compound of general formula IVb, prepared as described in Claim 16,

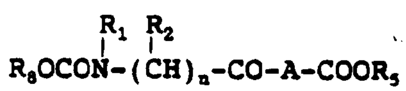 IVb

is reduced by one of the processes described in Claim 5 to give a methylene group, to give the derivative XXXI of formula

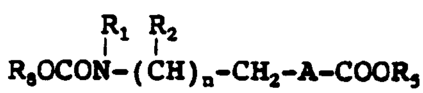 XXXI

which is then saponified by the process described in Claim 16 to lead to the sodium salt of the amino ester of formula XXXII

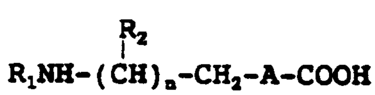 XXXII

which is condensed, without being isolated, with the sulphonyl halide of formula $RSO_2Z$ by the method described in Claim 16 to give, after acidification, the compound I where X=CO and $R_3$=H, of formula XXIII

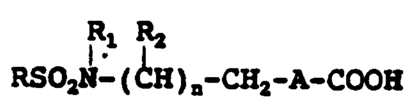 XXIII

in which the radicals in formulae IVb, XXXI, XXXII and XXIII have the same meaning as in Claims 1, 3 and 16.

**18.** Process for the preparation of salts of chemical compounds according to Claims 1 and 2, characterized in that the acid of general formula I where $R_3$=H is reacted with a stoichiometric amount of an inorganic or organic base in a protic solvent such as water, methanol or ethanol or a ketone solvent such as butanone

or acetone, at a temperature between 0°C and +60°C.

**19.** Process for the preparation of chemical compounds according to Claims 1, 2 and 7, characterized in that a compound of general formula I where $R_1$ = COO-tBu and $R_3$ differs from H, is hydrolysed by pouring the crude reaction mixture thus obtained into a concentrated aqueous solution of strong acid and more precisely hydrochloric acid, by heating at a temperature varying from 40 to 100°C and more precisely of between 50 and 70°C, to give the compound I where $R_1 = R_3$ = H and where the other radicals have the same meaning as in Claim 1.

**20.** As new medicaments, which can be used in paticular in the treatment of disorders of the cardiovascular system and more particularly as an anti-aggregant; anti-ischaemic agent at the myocardial, cerebral or peripheral level; anti-asthmatic or anti-atheromatous agents and, finally, as anti-migraine, anti-cancer and anti-viral agents, the compounds defined according to one of Claims 1 and 2.

**21.** Pharmaceutical compositions characterized in that they contain, as active principle, at least one compound defined according to one of Claims 1 and 2 in combination with an inert pharmaceutical carrier.

**Claims for the following Contracting States : ES, GR**

**1.** A process for the preparation of sulphonamido derivatives of carboxylic or benzo-heterocyclic acids of general formula I

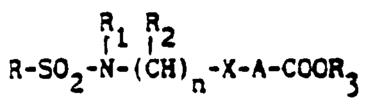 I

in which the radicals and substituents are defined as follows:

R represents a straight-chain or branched lower alkyl containing 1 to 9 carbon atoms, or phenyl or naphtyl radicals which are unsubstituted or substituted by one or more groups: lower alkyl (1 to 4 C), halogeno, lower alkoxy (1 to 4 C), nitro, amino, lower dialkylamino (1 to 4 C), trifluoromethyl;

$R_1$ represents hydrogen or a straight-chain or branched lower alkyl (1 to 4 C) or benzyl;

$R_2$ represents hydrogen or a straight-chain or branched lower alkyl (1 to 6 C), a phenyl group which is unsubstituted or substituted by a chlorine or a methoxy and an aralkyl group containing from 7 to 9 carbon atoms;

$R_3$ represents hydrogen or a straight-chain or branched lower alkyl (1 to 6 C);

-X- represents a divalent functional radical chosen from the following:

$-CH_2-$;

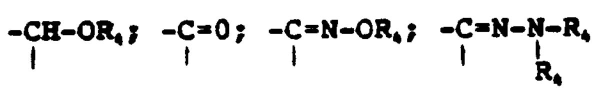

where $R_4$=H, Me with the proviso, however, that X differs from $-CH_2-$ when -A- represents divalent radicals a, b, d and e below, -A- represents a benzo-cyclic or benzo-heterocyclic divalent radical chosen from the following: (a-j)

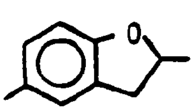 ; 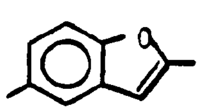 ; 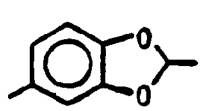 ; 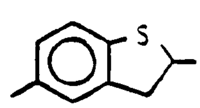

(a) (b) (c) (d)

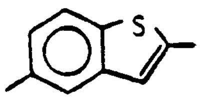 ; 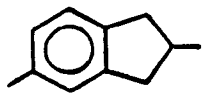 ; 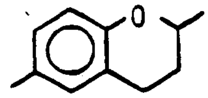 ; 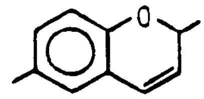

(e) (f) (g) (h)

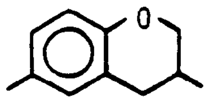 ; 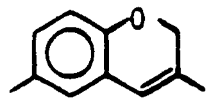

(i) (j)

and n can assume the values 1 to 4 inclusive, the hydrated forms of acids of general formula I and the therapeutically acceptable organic or inorganic salts of I ($R_3$=H) and in particular a salt chosen from: sodium, calcium, zinc, 2-hydroxyethylammonium, bis(2-hydroxyethyl)ammonium, tris(2-hydroxyethyl)ammonium, morpholinium and their hydrated form,
it being possible for the compounds of general formula I to be in form of a racemic mixture or to be isolated in the form of enantiomers, diastereoisomers or their mixture in any proportions.
characterized in that one conduct the following operation for A different from b, e, h, and j : a benzo-cyclic or benzoheterocyclic ester of formula II

$$H-A-COOR_5 \qquad \underline{II}$$

is reacted with a functional acyl halide of structure III

$$Y-(\overset{R_2}{\overset{|}{C}}H)_n-COY_1 \qquad \underline{III}$$

under Friedel-Crafts conditions using a compatible solvent, preferably chlorinated and more precisely methylene chloride or 1,2-dichloroethane, in the presence of a Lewis acid such as aluminium chloride by way of non-limiting example, and at a temperature varying from -5°C to +50°C so as to obtain the compound IVa of formula

$$Y-(\overset{R_2}{\overset{|}{C}}H)_n-CO-A-COOR_5 \qquad \underline{IVa}$$

which itself is subjected to a condensation reaction with sodium nitride in an aqueous-alcoholic mixture at a temperature of between +10°C and +50°C to give the compound of formula:

$$N_3-(\overset{R_2}{\overset{|}{C}}H)_n-CO-A-COOR_5 \qquad \underline{V}$$

which is selectively reduced by hydrogen in a lower alcohol containing an aqueous solution of a strong acid - hydrocloric for example - in the presence of a hydrogenation catalyst deposited on an inert support and more precisely palladium-on-carchoal for example, to give, at a temperature of between +10°C and +50°C, the aminoketoester hydrochloride of formula VI:

$$NH_2-(\overset{R_2}{\overset{|}{C}}H)_n-CO-A-COOR_5,\ HCl \qquad \underline{VI}$$

which is itself subjected to a condensation reaction with a suitably substituted sulphonyl halide (fluoride, chloride, bromide) of formula:

$$R-SO_2Z \qquad \underline{VII}$$

in a basic organic solvent such as pyridine for example, at a temperature of between -15°C and +40°C, to give the compound I where X=CO and $R_1$=H and $R_3$ differs from H, of formula VIII:

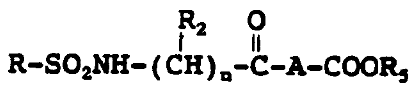

VIII

and finally, this ester is saponified with an aqueous alkaline solution such as sodium hydroxide solution or potassium hydroxide solution in the presence of a miscible organic solvent such as an alcohol or an ether and more precisely methanol, ethanol, dioxane or tetrahydrofuran, at a temperature of between +10°C and the boiling point of the mixture, to give, after acidification, the acid compound I where X=CO, $R_1$=H and $R_3$=H, of formula IX

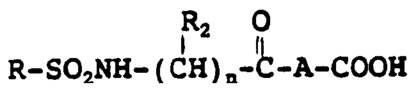

IX

the radicals in the general formulae of IVa to IX having the same meaning as in Claim 1 and $R_5$=$R_3$ with the exception of H; Y and $Y_1$ represent a chlorine or a bromine and Z represents a fluorine or a chlorine or a bromine.

and in that one conduct possibly at least one transformation of the compounds of formulae I, IV, V, or VI, obtained previously, notably by the way of reduction or condensation.

**2.** Process acording to claim 1 and characterized in that a compound of formula V,

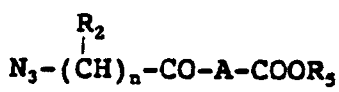

V

is reduced under an atmosphere of hydrogen in a lower alcohol containing an aqueous solution of a strong acid in the presence of a hydrogenation catalyst deposited on an inert support and more precisely palladium-on-charcoal for example, to give, at a temperature or between +10°C and +60°C and in the course of several hours - monitoring the progress of the reaction by chromatography - the compound of formula XII

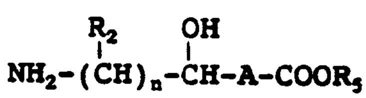

XII

which is subjected to a condensation reaction with a suitably substituted sulphonyl halide (fluoride, chloride, bromide) of formula:

$R\text{-}SO_2Z$

in a basic organic solvent such as pyridine for example, at a temperature of between -15°C and +40°C, to give compound I where X=CHOH and $R_1$=H and $R_3$ differs from H, of formula XIII

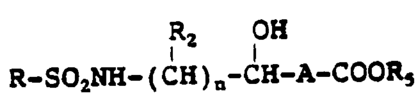

XIII

and finally this ester can be saponified with an aqueous alkaline solution such as sodium hydroxide solution or potassium hydroxide solution in the presence of an alcoholic or ethereal miscible organic solvent and more precisely methanol, ethanol, dioxane or tetrahydrofuran, at a temperature of between +10°C and +60°C to give, after acidification, the acid compound I where X=CHOH, $R_1$=H and $R_3$=H, of formula XIV

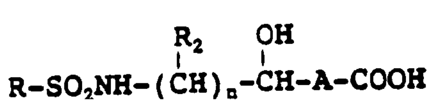

XIV

the radicals in general formulae V and XII to XIV having the same meaning as in Claims 1

**3.** Process according to Claim 1 and characterized in that a compound of formula VI,

$$HCl-NH_2-(\overset{R_2}{\overset{|}{C}}H)_n-\overset{O}{\overset{||}{C}}-A-COOR_5 \qquad \text{VI}$$

is reduced either under excess hydrogen pressure in the presence of a strong acid such as sulphuric acid or perchloric acid using a hydrogenation catalyst such as palladium-on-charcoal in a carboxylic acid as the solvent and more particularly acetic acid, carrying out the reaction at a temperature of between 20 and 90°C under a hydrogen pressure of between 0.1 and 5 atmospheres; or by reducing compound VI with a trialkylsilane and more precisely triethylsilane in the presence of trifluoroacetic acid at a temperature of between +10°C and +40°C, or by reducing compound VI with nascent hydrogen produced by the action of an acid (hydrochloric or acetic) on zinc, which may be in the form of zinc amalgam, by heating at between 40 and 120°C in an organic solvent such as toluene for example to give the compound XV in the form of the base or the salt of formula:

$$NH_2-(\overset{R_2}{\overset{|}{C}}H)_n-CH_2-A-COOR_5 \qquad \text{XV}$$

which is then subjected to a condensation reaction with a defined sulphonyl halide and under the same conditions as those described in Claims 1 and 2, to give the compound I where $R_1$=H and X=$CH_2$, of formula XVI, a saturated homologue of VIII and XIII

$$RSO_2NH-(\overset{R_2}{\overset{|}{C}}H)_n-CH_2-A-COOR_5 \qquad \text{XVI}$$

which is saponified by the process described in Claims 1 and 2 to prepare the derivatives IX and XIV, to give the compound I where $R_1$=$R_3$=H and X=$CH_2$, of formula XVII

$$RSO_2NH-(\overset{R_2}{\overset{|}{C}}H)_n-CH_2-A-COOH \qquad \text{XVII}$$

where the radicals in general formulae VI and XV have the same meaning as in Claim 1.

**4.** Process according to claim 1, characterized in that a compound of formula IVa,

$$Y-(\overset{R_2}{\overset{|}{C}}H)_n-\overset{O}{\overset{||}{C}}-A-COOR_5 \qquad \text{IVa}$$

is reduced by one of the methods of Claim 3 to give the halogenated compound equivalent to XV having the formula XIX

$$Y-(\overset{R_2}{\overset{|}{C}}H)_n-CH_2-A-COOR_5 \qquad \text{XIX}$$

which is subjected to a condensation reaction with the sodium salt, prepared in situ, of a suitably substituted secondary sulphonamide of formula XX

$$R-SO_2NH-R_7$$

in an organic solvent and more particularly dimethylformamide, dimethylacetamide, acetone, butanone, tetrahydrofuran or dioxane, at a temperature or between +5°C and +60°C to give the compound of formula is XXI

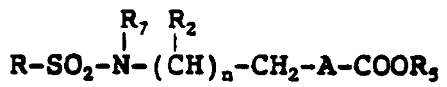

XXI

which derivative is then saponified by the process described in claims 3 and 4 with respect to derivatives IX and XIV, to give the equivalent satured compound I where X=$CH_2$ and $R_3$=H, of formula XXII

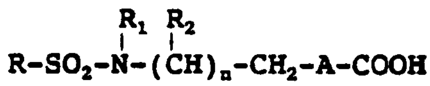

XXII

in which the radicals in the compounds of formulae IVa and XIX to XXII have the same meaning as in Claims 1 to 3 and where $R_2$ represents either the radical $R_1$ other than hydrogen or the radical $R_6COO$-, where $R_6$ represents a benzyl or a straight-chain or branched lower alkyl containing from 1 to 6 carbon atoms inclusive, and by way of non-limiting example the radicals: methyl, ethyl, tert.-butyl.

**5.** Process according to Claim 1 , characterized in that a compound of formula IVa, prepared according to Claims 1, is subjected to a condensation reaction with the sodium salt of compound XX,

$R-SO_2NHR$ XX

prepared in situ by the same process as to prepare compound XXI according to Claim 4, to give the keto-sulphamidoester I where X=CO, of formula XXIV

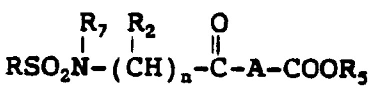

XXIV

which is saponified either directly in the case where $R_7$ differs from $COOR_6$ according to the process described for the compounds is VIII and XIII according to Claims 3 and 4, to give, in this case, the compound I where X=CO, $R_3$=H and $R_1$ differs from H, of formula XXV; or in the case where $R_7$=COO-tBu is first subjected to cleavage of the group $R_7$ in a strong acid medium and more precisely hydrochloric acid dissolved in an organic solvent such as ethyl acetate for example, to give the compound XXV where $R_1$=H and $R_5$=$R_3$ and differs from H, which can then be saponified to the acid as above, to give the compound I where $R_1$=$R_3$=H and X=CO, of formula XXV,

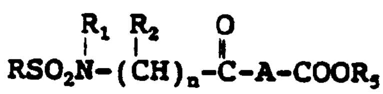

XXV

where the radicals in the formulae of compounds IVa, XX, XXIV and XXV have the same meaning as in Claims 1 and 4.

**6.** Process according to Claim 1 characterized in that a derivative of general structure XXIV, prepared according to Claim 5,

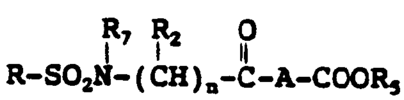

XXIV

is reduced by one of the three methods described in Claim 3 for preparation of the compound XV, to give, in this case, the sulphonamido homologue of formula XXI, already described in Claim 4.

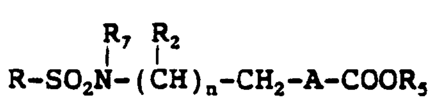

XXI

and saponified to the corresponding acid I where X=$CH_2$ and $R_3$=H, of formula XXII, according to the process already described in Claim 6, and where the radicals and variables in formulae XXX and XXI, have the same meaning as in Claims 1 and 4.

7. Process according to Claim 1 characterized in that a compound of general formula I where X=co is reduced to give, by one of the processes described in Claim 3, the reduced sulphonamide homologue of formula I where $X=CH_2$.

8. Process according to Claim 1 characterized in that a compound of general formula I where X=co is reduced in the presence of sodium borohydride or potassium borohydride in a protic solvent such as lowerr alcohol, preferably of the same condensation with respect to carbon as $R_3$ when $R_3$ differs from H (so as to prevent transesterification), at a temperature of between +5°C and +50°C, to give the compounds of general formula I where X=CHOH.

9. Process for the preparation of chemical compounds according to Claim 1 characterized in that a compound or formula I where $R_3$=H is reacted with an alcohol of general formula $R_3OH$ in the presence of an acid catalyst such as sulphuric acid or paratoluenesulphonic acid, heating at a temperature of between 40°C and 110°C, to give the compounds of general formula I where $R_3$ differs from H.

10. Process according according to Claim 1 characterized in that a compound of general formula I where X=CO and $R_3$=H is subjected to a condensation reaction with an unsubstituted or substituted hydroxylamine hydrochloride of formula $R_4ONH_2$, to give, in pyridine at a temperature of between 10°C and 50°C, the corresponding oximes I where X= C=N-$OR_4$ and $R_3$=H, where $R_4$ is defined as in Claim 1.

11. Process according to Claim 1 characterized in that a derivative of general formula I where X=CO and $R_3$=H is subjected to a condensation reaction with a hydrazine of general formula

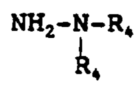

to give, in a protic organic solvent such as ethanol in the presence of an organic acid such as acetic acid for example, at a temperature of between 25°C and 120°C, the corresponding hydrazones I where

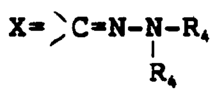

and $R_3$=H, where $R_4$ is defined as in Claim 1.

12. Process according to Claim 1 characterized in that an ester of general formula I where the bicyclic radical -A- im saturated on the cycle attached to the carboxylate function (that is to say where A = a, d, g or i) is reacted with a halogenoimide and more particularly N-bromo- or N-chlorosuccinimide in a halogenated solvent and more precisely carbon tetrachloride at a temperature of between +20°C and +80°C to give the corresponding unsaturated compounds of general formula I (that is to say where A = b, e or h) where $R_3$, differs from H, which compounds are then saponified to their precursor acids of general formula I where $R_3$=H using the method described in Claim 1.

13. Process according to Claim 1 characterized in that a compound or general formula I in which $R_1$=H is reacted with an activated derivative or formula $(R_1)_2SO_4$ or $R_1Z$, where Z=Br or I, in the presence of a strong base such as sodium hydride or a sodium carbonate or hydroxide or a potassium carbonate or hydroxide, in a solvent such as DMF, dimethylacetamide, butanone or acetone and at a temperature of between +20°C and +70°C to give the equivalent compound of general formula I, in which $R_1$ and $R_3$ differ from H and where the variable radicals are defined as in Claim 1.

14. Process according to claim 1 where A differs from b, e, h and j, characterized in that a benzo-carbocyclic or benzo-heterocyclic ester of general formula II

$$H\text{-}A\text{-}COOR_5 \qquad II$$

is reacted with a functional acyl halide of structure IIIa

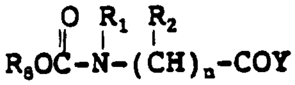

IIIa

under Friedel-Crafts conditions using a compatible solvent, preferably chlorinated and more precisely methylene chloride or 1,2-dichloromethane, in the presence of a Lewis acid, such as aluminium chloride by way or non-limiting example, and at a temperature of between -5°C and +50°C so as to obtain compound IVb of formula

$$R_8OCO\overset{R_1}{\overset{|}{N}}-(\overset{R_2}{\overset{|}{C}}H)_n-CO-A-COR_5 \qquad \underline{IVb}$$

which is itself reduced by an alkaline metal borohydride by the process described in Claim 10 so as to obtain the derivative XXIX of formula

$$R_8OCO-\overset{R_1}{\overset{|}{N}}-(\overset{R_2}{\overset{|}{C}}H)_n-CHOH-A-COOR_5 \qquad \underline{XXIX}$$

to lead, by basic hydrolysis, to the sodium salt of the aminohydroxy acid of formula XXX by the method described in Claim 3 for the preparation of compound IX

$$R_1NH-(\overset{R_2}{\overset{|}{C}}H)_n-CHOH-A-COOH \qquad \underline{XXX}$$

and finally, by subjecting a suitably substituted sulphonyl halide of formula $RSO_2Z$ to a condensation reaction with the crude sodium salt of XXX obtained above in the same aqueous-organic phase at a pH of between 11 and 13 and at a temperature of between 0 and + 40°C, to give the compound I where $R_3$=H and Y=CHOH, of formula XXVII

$$ArSO_2\overset{R_1}{\overset{|}{N}}-(\overset{R_2}{\overset{|}{C}}H)-CHOH-A-COOH \qquad \underline{XXVII}$$

in which the radicals in general formulae IVb, XXIX, XXX and XXVII have the same meaning as in Claims 1 and $R_8$ represents a lower straight-chain alkyl radical having 1 to 6 carbon atoms inclusive.

**15.** Process according to Claim 1 characterized in that the ketone of a compound of general formula IVb, prepared as described in Claim 16,

$$R_8OCO\overset{R_1}{\overset{|}{N}}-(\overset{R_2}{\overset{|}{C}}H)_n-CO-A-COOR_5 \qquad \underline{IVb}$$

is reduced by one of the processes described in Claim 5 to give a methylene group, to give the derivative XXXI of formula

$$R_8OCO\overset{R_1}{\overset{|}{N}}-(\overset{R_2}{\overset{|}{C}}H)_n-CH_2-A-COOR_5 \qquad \underline{XXXI}$$

which is then saponified by the process described in Claim 14 to lead to the sodium salt of the amino ester of formula XXXII

$$R_1NH-(\overset{R_2}{\overset{|}{C}}H)_n-CH_2-A-COOH \qquad \underline{XXXII}$$

which is condensed, without being isolated, with the sulphonyl halide of formula $RSO_2Z$ by the method described in Claim 14 to give, after acidification, the compound I where X=CO and $R_3$=H, of formula

XXIII

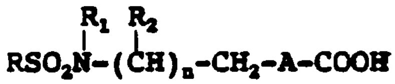

**XXIII**

in which the radicals in formulae IVb, XXXI, XXXII and XXIII have the same meaning as in Claim 1.

**16.** Process for the preparation of salts of chemical compounds according to Claims 1, characterized in that the acid of general formula I where $R_3$=H is reacted with a stoichiometric amount of an inorganic or organic base in a protic solvent such as water, methanol or ethanol or a ketone solvent such as butanone or acetone, at a temperature between 0°C and +60°C.

**17.** Process for the preparation of chemical compounds according to Claims 1 and 5, characterized in that a compound of general formula I, where $R_1$ = COO-tBu and $R_3$ differs from H, is hydrolysed by pouring the crude reaction mixture thus obtained into a concentrated aqueous solution of strong acid and more precisely hydrochloric acid, by heating at a temperature varying from 40 to 100°C and more precisely of between 50 and 70°C, to give the compound I where $R_1$ = $R_3$ = H and where the other radicals have the same meaning as in Claim 1.